(19) Europäisches Patentamt
European Patent Office
Office européen des brevets

(11) **EP 1 419 143 B1**

(12) **EUROPEAN PATENT SPECIFICATION**

(45) Date of publication and mention of the grant of the patent:
**15.04.2009 Bulletin 2009/16**

(21) Application number: **02752924.7**

(22) Date of filing: **22.08.2002**

(51) Int Cl.:
***C07C 401/00*** *(2006.01)* ***A61K 31/59*** *(2006.01)*

(86) International application number:
**PCT/CA2002/001294**

(87) International publication number:
**WO 2003/018545 (06.03.2003 Gazette 2003/10)**

(54) **24-SULFUR-SUBSTITUTED ANALOGS OF 1ALPHA,25-DIHYDROXY VITAMIN D3**

24-SCHWEFEL-SUBSTITUIERTE 1-ALPHA-25-DIHYDROXY-VITAMIN-D3-ANALOGEN

ANALOGUES DE LA 1-ALPHA-25-DIHYDROXY-VITAMINE-D3 SUBSTITUES PAR UN SOUFRE EN POSITION 24

(84) Designated Contracting States:
**AT BE BG CH CY CZ DE DK EE ES FI FR GB GR IE IT LI LU MC NL PT SE SK TR**

(30) Priority: **22.08.2001 US 313769 P**
**12.10.2001 US 328429 P**
**13.06.2002 US 387931 P**

(43) Date of publication of application:
**19.05.2004 Bulletin 2004/21**

(73) Proprietors:
- **JOHNS HOPKINS UNIVERSITY**
  **Baltimore, MD 21201 (US)**
- **Cytochroma Inc.**
  **Markham,**
  **Ontario L3R 8E4 (CA)**

(72) Inventors:
- **POSNER, Gary, H.**
  **Baltimore, MD 21208 (US)**
- **CRAWFORD, Kenneth, R.**
  **Apt 106A, San Mateo, CA 94403 (US)**
- **YANG, Hong, Woon**
  **Superior, CO 80027 (US)**
- **WHITE, Jay, A.**
  **Newmarket, Ontario L3X 1T3 (CA)**
- **JONES, Glenville**
  **Kingston, Ontario K7M 6N7 (CA)**
- **SUH, Byung-Chul**
  **Cockeysville, MD 21030 (US)**
- **JEON, HeungBae**
  **Baltimore, MD 21209 (US)**
- **HATCHER, Mark**
  **Baltimore, MD 21231 (US)**

(74) Representative: **Alcock, David**
**D Young & Co**
**120 Holborn**
**London EC1N 2DY (GB)**

(56) References cited:
**EP-A- 0 386 793**
**WO-A-00/59513**
**WO-A-91/15475**
**WO-A-94/10139**
**WO-A-94/14766**

- **POSNER G H ET AL: "CONCEPTUALLY NEW SULFONE ANALOGUES OF THE HORMONE 1ALPHA,25-DIHYDROXYVITAMIN D3: SYNTHESIS AND PRELIMINARY BIOLOGICALEVALUATION" JOURNAL OF MEDICINAL CHEMISTRY, AMERICAN CHEMICAL SOCIETY. WASHINGTON, US, vol. 42, no. 18, 1999, pages 3425-3435, XP000938943 ISSN: 0022-2623**
- **KENSLER, T.W. ET AL.: "Conceptually new deltanoids (vitamin D analogs) inhibit multistage skin tumorigenesis" CARCINOGENESIS, vol. 21, no. 7, 2000, pages 1341-1345, XP001121614**

EP 1 419 143 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

This invention was made with government support under NIH Grant Number CA 44530. The government has certain rights in the invention.

## FIELD OF THE INVENTION

The present invention relates to novel analogs of the hormone 1α,25-dihydroxy vitamin $D_3$ that show selective inhibition of the enzyme CYP24 and which are low-calcemic, to pharmaceutical and diagnostic compositions containing them and to their medical use, particularly in the treatment and/or prevention of cancer, dermatological disorders, bone disorders, thyroid disorders, wound healing and osteoporosis.

## BACKGROUND OF THE INVENTION

The vitamin D metabolic pathway is part of a vital endocrine system that is highly regulated at certain stages and produces metabolites that control the secretion of the parathyroid gland hormones (Beckman, M., and DeLuca, H. (1997) Methods in Enzymol. 282, 200-223; Jones, G., Strugnell, S., and DeLuca, H. (1998) Physiol. Rev. 78, 1193-1231). 1α,25-Dihydroxy vitamin $D_3$, also known as calcitriol (see below), a hormone produced in the vitamin D pathway, regulates phosphate and calcium levels in the blood which in turn control bone mass, the state of bones, and affects cellular differentiation in the skin and the immune system (Armbrecht, H.J., Okuda, K., Wongsurawat, N., Nemani, R., Chen, M., and Boltz, M. (1992) J. Steroid Biochem. Molec. Biol. 43, 1073-1081). In the vitamin D pathway, cytochrome P450s are enzymes that introduce functional groups by hydroxylation, usually at positions 1, 25, and 24, of vitamin $D_3$ (Beckman, M., and DeLuca, H. (1997) Methods in Enzymol. 282, 200-223).

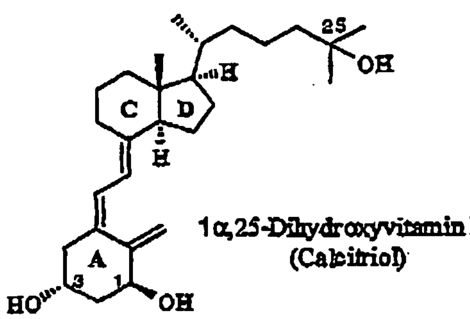

1α,25-Dihydroxyvitamin $D_3$
(Calcitriol)

1α,25-Dihydroxy vitamin $D_3$ is converted to 1α,24,25-trihydroxy-$D_3$ by a mitochondrial P450 known as CYP 24 (Bell, N.H., (1998) J. Bone Miner. Res 13, 350- 35211). CYP 24 is induced by 1α,25-dihydroxy-$D_3$ and is found in the kidney as well as other vitamin D target tissues such as the parathyroid cells, keratinocytes, osteoblasts, and enteroctyes (Jones, G., Strugnell, S., and DeLuca, H. (1998) Physiol. Rev. 78, 1193-1231). 1α,25-Dihydroxy vitamin $D_3$ (1,25-D3) has an important role in the antiproliferative and growth regulatory effects on normal and neoplastic cells (for e.g. prostate cancer cells). Clinical use of 1,25-D3 analogs as effective drugs requires separating desirable antiproliferative and pro-differentiating activities from undesirable calcemic activity. There is a continuing need for synthetic analogs of 1α,25-dihydroxy vitamin $D_3$ that selectively exhibit desirable pharmacological activities but do not exhibit hypercalcemic and other undesirable activity.

A series of vitamin $D_3$-like non-fluorinated and fluorinated 16-ene side chain tert-butyl sulfones have been synthesized (Posner et al (1999) 42, no. 18, 3425-3435). Even though these novel C, D-ring side chain analogues of the hormone 1α,25-dihydroxyvitamin $D_3$ lack a terminal OH group, thought previously to be essential for high biological activity, they are taught as being anti-proliferative and, in several cases, transcriptionally active *in vitro* but desirably non-calcemic *in vivo*.

WO 94/10139 discloses vitamin $D_3$ analogues. Amongst other substitutions, the 24-methylene of 1α,25-dihydroxy-20-epi-vitamin $D_3$, has been replaced by the group, $(CH_2)_n$-Y-$(CH_2)_m$, where n is 0, 1 or 2, m is 1 or 2, and Y is oxygen or sulphur. WO 94/10139 states that the compounds show anti-inflammatory and immuno-modulating effects as well as strong activity in inducing differentiation and inhibiting undesirable proliferation of certain cells.

Additionally, further deltanoids have been synthesized with modifications to the 1α and/or 25-hydroxyl groups, positions traditionally considered essential for stimulating biological responses (Kensler et al (2000) 21, no. 7, 1341-1345). The biological response produced in mice as a result of administration of these deltanoids was also tested.

WO 00/59513 discloses sulfur-containing analogues of 1α,25-dihydroxyvitamin $D_3$. The analogues are synthesized in a convergent manner by joining A-ring and C, D ring fragments. WO 00/59513 states that each analogue with 1α,3β-substituent stereochemistry shows a pharmacologically desirable combination of high anti-proliferative and high transcriptional activities in vitro and also low calcemic activity in vivo.

## SUMMARY OF THE INVENTION

It has been found that 24-aryl sulfone analogs of 1α,25-dihydroxy vitamin $D_3$ show selective inhibition of the enzyme CYP24.

The present invention therefore provides compounds of Formula I, and pharmaceutically acceptable salts, hydrates, solvates and prodrugs thereof, wherein the prodrug is an ester prodrug selected from the group consisting of phenyl esters, aliphatic $C_8$-$C_{24}$ esters, acyloxymethyl esters, carbamates and amino acid esters:

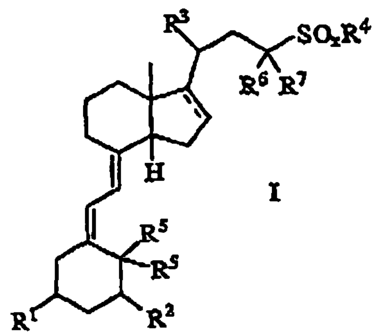

I

wherein

$R^1$ and $R^2$ are independently selected from the group consisting of OH, $OC_{1-4}$alkyl, and halo;

$R^3$ is $C_{1-4}$alkyl;

$R^4$ is selected from the group consisting of aryl and heteroaryl with both aryl and heteroaryl being unsubstituted or substituted with 1-5 groups independently selected from $C_{1-4}$alkyl, hydroxy-substituted $C_{1-6}$alkyl, $OC_{1-4}$alkyl, OH, $CF_3$, $OCF_3$, halo, SH, $SC_{1-4}$alkyl, $NH_2$, $NHC_{1-4}$alkyl, $N(C_{1-4}alkyl)(C_{1-4}alkyl)$, CN, C(O)OH, $C(O)OC_{1-4}$alkyl, $C(O)NHC_{1-}C_{1-4}$alkyl, $CH=N-OC_{1-4}$akyl, $NHC(O)C_{1-4}$akyl, $OC(O)C_{1-4}$alkyl, $SOC_{1-4}$alkyl, $SO_2C_{1-4}$alkyl, $SO_2NHC_{1-4}$alkyl and $SO_2NH_2$;

$R^5$ are either both H or together form $=CH_2$;

$R^6$ and $R^7$ are independently H, $C_{1-4}$alkyl or are taken together to form a $C_{3-6}$cyloalkyl ring;

x is 0-2; and

= represents a single or a double bond.

In an embodiment, the present invention provides compounds of Formula I wherein the stereochemistry is that of natural 1α,25-dihydroxy vitamin $D_3$. Accordingly, the present invention relates to a compound of Formula I, and pharmaceutically acceptable salts, hydrates, solvates and prodrugs thereof, wherein the prodrug is an ester prodrug selected from the group consisting of phenyl esters, aliphatic $C_8$-$C_{24}$ esters, acyloxymethyl esters, carbamates and amino acid esters:

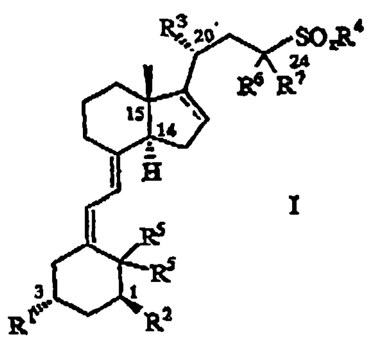

I

wherein
$R^1$ and $R^2$ are independently selected from the group consisting of OH, $OC_{1-4}$alkyl, and halo;
$R^3$ is $C_{1-4}$alkyl;
$R^4$ is selected from the group consisting of aryl and heteroaryl with both aryl and hetetoaryl being unsubstituted or substituted with 1-5 groups independently selected from $C_{1-4}$alkyl, hydroxy-substituted $C_{1-4}$alkyl, $OC_{1-4}$alkyl, OH, $CF_3$, $OCF_3$, halo, SH, $SC_{1-4}$alkyl, $NH_2$, $NHC_{1-4}$alkyl, $N(C_{1-4}alkyl)(C_{1-4}alkyl)$. CN, C(O)OH, $C(O)OC_{1-4}$alkyl, $C(O)NHC_{1-4}$alkyl, $CH=N-OC_{1-4}$alkyl, $NHC(O)C_{1-4}$alkyl, $OC(O)C_{1-4}$alkyL, $SOC_{1-4}$alkyl, $SO_2C_{1-4}$alkyl, $SO_2NHC_{1-4}$alkyl, and $SO_2NH_2$;
$R^5$ are either both H or together form = $CH_2$;
and $R^7$ are independently H. $C_{1-4}$alkyl or are taken together to form a $C_{3-6}$cyloalkyl ring;
x is 0-2; and
---- represents a single or a double bond.

In a further embodiment of the invention, the compounds of Formula I are those wherein $R^6$ and $R^7$ are H.

According to another aspect of the present invention, there is provided a pharmaceutical composition comprising a compound as defined in the claims and a pharmaceutically acceptable carrier or diluent.

In a further embodiment, there is provided a compound as defined in the claims for use as a medicament.

By selectively modulating CYP24, the enzyme that metabolizes 1α,25-dihydroxy vitamin $D_3$, the levels of 1α, 25-dihydroxy, vitamin $D_3$ may also be modulated. Diseases that benefit from a modulation of the levels of 1α,25-dihydroxy vitamin $D_3$ can therefore be treated using a modulator of CYP24. By acting preferentially on CYP24, side effects caused by interaction with other enzymes and receptors may be reduced.

Inhibition of CYP24, should inhibit the catabolism of 1α,25-dihydroxy vitamin $D_3$ which is expected to lengthen the biological lifetime of this hormone and thus allow smaller amounts of it to be used for effective disease treatment. Such smaller dosing is expected to avoid, or at least minimize, the hypercalcemic toxicity associated with medicinal use of 1α,25-dihydroxy vitamin $D_3$ (calcitriol).

Diseases which may benefit for a modulation in the levels of 1α,25-dihydroxy vitamin $D_3$ include, but are not limited to:

(i) in the parathyroid - hyper- and hypo-parathyroidism, Osudohypo-parathyroidism, Secondary hyperparathyroidism;
(ii) in the pancreas-diabetes;
(iii) in the thyroid-medullary carcinoma;
(iv) in the skin - psoriasis, wound healing;
(v) in the lung -sarcoidosis and tuberculosis;
(vi) in the kidney - chronic renal disease hypophosphtatemic VDRR, vitamin D dependent rickets;
(vii) in the bone- anticonvulsant treatment, fibrogenisis imperfecta ossium, osteitits fibrosa cystica, osteomalacia, osteporosis, osteopenia, osteosclerosis, renal osteodytrophy, rickets;
(viii) in the intestine glucocorticoid antagonism, idopathic hypercalcemia, malabsorption syndrome, steatorrhea, tropical sprue.

The invention further includes a use of a compound as defined in the claims to prepare a medicament to inhibit cell proliferation.

The invention further includes a use of a compound as defined in the claims to prepare a medicament to inhibit cancer cell proliferation.

Other features and advantages of the present invention will become apparent from the following detailed description.

## BRIEF DESCRIPTION OF THE DRAWINGS

The invention will now be described in relation to the drawings in which:

Figure 1A is a graph showing the inhibition of CYP24 activity by compound I(a) (indicated as $KRC24SO_2Ph$-1) compared to ketoconazole.
Figure 1B is a graph showing the inhibition of CYP27B1 activity by compound I(a) (indicated as $KRC24SO_2Ph$-1) compared to ketoconazole.
Figure 1C is a graph showing the inhibition of CYP27A1 activity by compound I(a) (indicated as $KRC24SO_2Ph$-1) compared to ketoconazole.
Figure 2 is a graph showing the binding of compound I(a) (indicated as $KRC24SO_2Ph$-1) compared to 1α,25-dihydroxy vitamin $D_3$ at the vitamin D receptor.
Figure 3 is a graph showing the activity of compound I(a) (indicated as $KRC24SO_2Ph$-1) in the vitamin D transcription assay compared to 1α,25-dihydroxy vitamin $D_3$.

Figure 4 is a graph showing the activity of compound I(a) (indicated as $KRC24SO_2Ph$-1) in the DBP binding assay compared to 1α,25-dihydroxy vitamin $D_3$.

## DETAILED DESCRIPTION OF THE INVENTION

### I. Definitions

The term "$C_{1-4}$alkyl" as used herein means straight and/or branched chain alkyl groups containing from one to four carbon atoms and includes methyl, ethyl, propyl, isopropyl, t-butyl and the like.

The term "hydroxy-substituted $C_{1-4}$alkyl" as used herein means straight and/or branched chain alkyl groups containing from one to four carbon atoms and substituted with 1-2 hydroxyl groups and includes hydroxymethyl, 1-hydroxyethyl, 2-hydroxyl-2-propyl and the like.

The term "$C_{1-4}$alkoxyl" as used herein means straight and/or branched chain alkoxy groups containing from one to four carbon atoms and includes methoxy, ethoxy, propyoxyl, isopropyloxy, t-butoxy and the like.

The term "$C_{3-6}$cycloalkyl" as used herein means a 3- to 6-membered saturated carbocyclic ring.

The term "aryl" as used herein means unsubstituted or substituted mono- or bicyclic aromatic groups containing from 6 to 10 carbon atoms and includes phenyl and naphthyl and the like.

The term "heteroaryl" as used herein means unsubstituted or substituted mono- or bicyclic heteroaromatic groups containing from 5 to 10 atoms, of which 1-3 atoms may be a heteroatom selected from the group consisting of S, O and N, and includes furanyl, thienyl, pyrrolo, pyridyl, indolo, benzofuranyl and the like.

The term "halo" as used herein means halogen and includes chloro, flouro, bromo, iodo and the like.

The term "pharmaceutically acceptable salt" means an acid addition salt or a basic addition salt which is suitable for or compatible with the treatment of patients.

The term "pharmaceutically acceptable acid addition salt" as used herein means any non-toxic organic or inorganic salt of any base compound of the invention, or any of its intermediates. Basic compounds of the invention that may form an acid addition salt include those where $R^4$ is substituted with a group having a basic nitrogen, for example $NH_2$ and $NHC_{1-4}$alkyl. Illustrative inorganic acids which form suitable salts include hydrochloric, hydrobromic, sulfuric and phosphoric acids, as well as metal salts such as sodium monohydrogen orthophosphate and potassium hydrogen sulfate. Illustrative organic acids that form suitable salts include mono-, di-, and tricarboxylic acids such as glycolic, lactic, pyruvic, malonic, succinic, glutaric, fumaric, malic, tartaric, citric, ascorbic, maleic, benzoic, phenylacetic, cinnamic and salicylic acids, as well as sulfonic acids such as p-toluene sulfonic and methanesulfonic acids. Either the mono or di-acid salts can be formed, and such salts may exist in either a hydrated, solvated or substantially anhydrous form. In general, the acid addition salts of the compounds of the invention are more soluble in water and various hydrophilic organic solvents, and generally demonstrate higher melting points in comparison to their free base forms. The selection of the appropriate salt will be known to one skilled in the art. Other non-pharmaceutically acceptable salts, e.g. oxalates, may be used, for example, in the isolation of the compounds of the invention, for laboratory use, or for subsequent conversion to a pharmaceutically acceptable acid addition salt.

The term "pharmaceutically acceptable basic addition salt" as used herein means any non-toxic organic or inorganic base addition salt of any acid compound of the invention, or any of its intermediates. Acidic compounds of the invention that may form a basic addition salt include those where $R^4$ is substituted with a group having acidic hydrogen, for example C(O)OH. Illustrative inorganic bases which form suitable salts include lithium, sodium, potassium, calcium, magnesium or barium hydroxide. Illustrative organic bases which form suitable salts include aliphatic, alicyclic or aromatic organic amines such as methylamine, trimethylamine and picoline or ammonia. The selection of the appropriate salt will be known to a person skilled in the art.

The term "solvate" as used herein means a compound of the invention, or a pharmaceutically acceptable salt of a compound of the invention, wherein molecules of a suitable solvent are incorporated in the crystal lattice. A suitable solvent is physiologically tolerable at the dosage administered. Examples of suitable solvents are ethanol, water and the like. When water is the solvent, the molecule is referred to as a "hydrate".

The term "compound(s) of the invention" as used herein means compound(s) of Formula I, and salts, hydrates, solvates and prodrugs thereof.

The term an "effective amount" or a "sufficient amount " of an agent as used herein is that amount sufficient to effect beneficial or desired results, including clinical results, and, as such, an "effective amount" depends upon the context in which it is being applied. For example, in the context of administering an agent that inhibits cancer cell proliferation, an effective amount of an agent is, for example, an amount sufficient to achieve such a reduction in cancer cell proliferation as compared to the response obtained without administration of the agent

As used herein, and as well understood in the art, "treatment" is an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening)

state of disease, preventing spread of disease, delay or slowing of disease progression, amelioration or palliation of the disease state, and remission (whether partial or total), whether detectable or undetectable. "Treatment" can also mean prolonging survival as compared to expected survival if not receiving treatment.

"Palliating" a disease or disorder means that the extent and/or undesirable clinical manifestations of a disorder or a disease state are lessened and/or time course of the progression is slowed or lengthened, as compared to not treating the disorder.

The term "modulate" as used herein includes the inhibition or suppression of a function or activity (such as cell proliferation) as well as the enhancement of a function or activity.

To "inhibit" of "suppress" or "reduce" a function or activity, such as cancer cell proliferation, is to reduce the function or activity when compared to otherwise same conditions except for a condition or parameter of interest, or alternatively, as compared to another conditions.

The term "animal" as used herein includes all members of the animal kingdom including human. The animal is preferably a human.

The term "a cell" as used herein includes a plurality of cells. Administering a compound to a cell includes *in vivo*, *ex vivo* and *in vitro* treatment.

The term "cancer cells" as used herein includes all forms of cancer or neoplastic disease.

## II. Compounds of the Invention

Novel compounds showing selective inhibition of the enzyme GYP24 have been prepared As such, the compounds as defined in the claims are useful for treatment cell proliferative diseases, such as cancer.

Accordingly, the present invention provides compounds of Formula I, and pharmaceutically acceptable salts, hydrates, solvates and prodrugs thereof, wherein the prodrug is an ester prodrug selected from the group consisting of phenyl esters, aliphatic $C_8$-$C_{24}$ esters, acyloxymethyl esters, carbamates and amino acid esters:

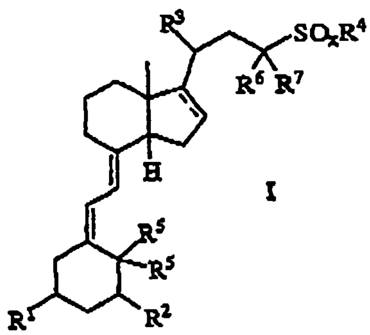

I

wherein

$R^1$ and $R^2$ are independently selected from the group consisting of OH $OC_{1-4}$alkyl, and halo;

$R^3$ is $C_{1-4}$alkyl;

$R^4$ is selected from the group consisting of aryl and Heteroaryl with both aryl and heteroaryl being unsubstituted or substituted with 1-5 groups independently selected from $C_{1-4}$alkyl, hydroxy-substituted $C_{1-4}$alkyl, $OC_{1-4}$alkyl, OH, $CF_3$, $OCF_3$, halo, SH, $SC_{1-4}$alkyl, $NH_2$, $NHC_{1-4}$alkyl, $N(C_{1-4}alkyl)(C_{1-4}alkyl)$, CN, C(O)OH, C(4)$OC_{1-4}$alkyl, C(O)$NHC_{1-4}$alkyl, CH=N -$OC_{1-4}$alkyl, NHC(O)$C_{1-4}$alkyl, OC(O)$C_{1-4}$alkyl $SOC_{1-4}$alkyl, $SO_2C_{1-4}$alkyl, $SO_2NHC_{1-4}$alkyl and $SO_2NH_2$;

$R^5$ are either both H or together form $=CH_2$;

$R^6$ and $R^7$ are independently H, $C_{1-4}$alkyl or are taken together to form a $C_{3-6}$cyloalkyl ring;

x is 0.2; and

---- represents a single or a double bond.

The compounds of Formula I include those in which $R^1$ and $R^2$ are independently selected from the group consisting of OH, $OC_{1-4}$alkyl, and halo. In embodiments of the invention, $R^1$ and $R^2$ are independently selected from the group consisting of OH, $OCH_3$, and fluoro. In a further embodiment, $R^1$ and $R^2$ are both OH.

The present invention includes compounds of Formula I wherein $R^3$ is $C_{1-4}$alkyl. In embodiments of the invention, $R^3$ is $CH_3$.

The present invention includes compounds of Formula wherein $R^4$ is selected from the group consisting of $C_{1-6}$alkyl, aryl and heteroaryl with both aryl and heteroaryl being unsubstituted or substituted with 1-5 groups independently selected from $C_{1-4}$alkyl, hydroxy-substituted $C_{1-6}$alkyl, $OC_{1-4}$alkyl, OH, $CF_3$, $OCF_3$, halo, SH, $SC_{1-4}$alkyl, $NH_2$, $NHC_{1-4}$alkyl, $N(C_{1-4}alkyl)(C_{1-4}alkyl)$, CN, C(O)OH, C(O)$OC_{1-4}$alkyl, C(O)$NHC_{1-4}$alkyl, CH=N-$OC_{1-4}$alkyl, NHC(O)

$C_{1-4}$alkyl, $OC(O)C_{1-4}$alkyl, $SOC_{1-4}$alkyl, $SO_2C_{1-4}$alkyl, $SO_2NHC_{1-4}$alkyl and $SO_2NH_2$. In embodiments of the invention, $R^4$ is selected from unsubstituted or substituted phenyl, pyridyl, thienyl, furanyl and pyrrolo. In further embodiments, $R^4$ is selected from unsubstituted or substituted phenyl, In still further embodiments of the present invention both aryl and heteroaryl may be either unsubstituted or substituted with 1-3 groups independently selected from $C_{1-4}$alkyl, hydroxy-substituted $C_{1-6}$alkyl, $OC_{1-4}$alkyl, OH, $CF_3$, $OCF_3$, halo, SH, $SC_{1-4}$alkyl, $NH_2$, $NHC_{1-4}$alkyl, $N(C_{1-4}alkyl)(C_{1-4}alkyl)$, CN, C(O)OH, $C(O)OC_{1-4}$alkyl, $CH=N-OC_{1-4}$alkyl, $C(O)NHC_{1-4}$alkyl, $NHC(O)C_{1-4}$alkyl, $OC(O)C_{1-4}$alkyl, $SOC_{1-4}$alkyl, $SO_2C_{1-4}$alkyl, $SO_2C_{1-4}$alkyl and $SO_2NH_2$. Preferably the substituent is located at a position other than that *ortho* to the $SO_2$ group. In further embodiments, both aryl and heteroaryl may be either unsubstituted or substituted with 1-2 groups independently selected from methyl, 3-hydroxy-3-pentyl, methoxy, OR, $CF_3$, $OCF_3$, halo, $NH_2$, $NMe_2$ and CH=N-OMe. In further embodiment, both aryl and heteroaryl may be either unsubstituted or substituted with 1-2 groups independently selected from methyl, 3-hydroxy-3-pentyl, Cl, F and CH=N-OMe. In specific embodiments of the invention, $R^4$ is selected from the group consisting of phenyl, 4-chlorophenyl, 3,4-dichloropheny, 4-fluorophenyl, 4-methylphenyl, 3,4-difluorophenyl, 4-(3-hydroxy-3-pentyl)phenyl, 4-(CH=N-OMe)phenyl, 4-methoxyphenyl, 4-trifluormethylpheny and 4-ntirophenyl. In more specific embodiments of the invention, $R^4$ is selected from the group consisting of phenyl, 4-chlorophenyl, 3,4-dichloropheny, 4-(3-hydroxy-3-pentyl)phenyl, 4-fluorophenyl and 4-methylphenyl.

The compounds of Formula I include those where $R^5$ are either both H or, together, $R^5$ form the group $-CH_2$.

The compounds of Formula I include those where $R^6$ and $R^7$ are independently H, $C_{1-4}$alkyl or are taken together to form a $C_{3-6}$cyloalkyl ring. In embodiments of the invention, $R^6$ and $R^7$ are independently H, methyl or are taken together to form a $C_{3-4}$cyloalkyl ring. In further embodiments of the invention, $R^6$ and $R^7$ are both H or are taken together to form a $C_{3-4}$cyloalkyl ring.

The present invention further includes compounds of Formula I wherein x is 0-2. In embodiments of the invention, x is 2.

The present invention also includes compounds of Formula I wherein ---- represents a single or a double bond.

All of the compounds of Formula I have more than one asymmetric centre. Where the compounds according to the invention possess more than one asymmetric centre, they may exist as diastereomers. It is to be understood that all such isomers and mixtures thereof in any proportion are encompassed within the scope of the present invention. The stereochemistry of the compounds of the invention is preferably that of natural 1α,25-dihydroxy vitamin $D_3$. Therefore, in an embodiment, the present invention provides compounds of Formula I, and pharmaceutically acceptable salts, hydrates, solvates and prodrugs thereof wherein the prodrug is an ester prodrug selected from the group consisting of phenyl esters, aliphatic $C_8$-$C_{24}$ esters, acyloxymethyl esters, carbamates and amino acid esters:

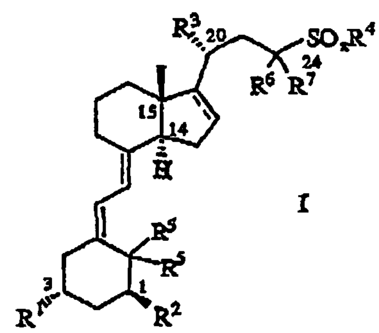

I

wherein

$R^1$ and $R^1$ are independently selected from the group consisting of OH, $OC_{1-4}$alkyl, and halo;

$R^3$ is $C_{1-4}$alkyl;

$R^4$ is selected from the group consisting of aryl and heteroaryl with both aryl and heteroaryl being unsubstituted or substituted with 1-5 groups independently selected from $C_{1-4}$alkyl, hydroxy-substituted $C_{1-6}$alkyl, $OC_{1-4}$alkyl; OH $CF_3$, $OCF_3$, halo, SH, $SC_{1-4}$alkyl, $NH_2$, $NHC_{1-4}$alkyl, $N(C_{1-4}alkyl)(C_{1-4}alkyl)$, CN, C(O)OH, $C(O)OC_{1-4}$alkyl, $C(O)NHC_{1-4}$alkyl, $NHC(O)C_{1-4}$alkyl, $OC(O)C_{1-4}$alkyl, $SOC_{1-4}$alkyl, $SO_2C_{1-4}$alkyl, $SO_2NHC_{1-4}$alkyl and $SO_2NH_2$;

$R^5$ are either both H or together form $=CH_2$;

$R^6$ and $R^7$ are independently H, $C_{1-4}$alkyl or are taken together to form a $C_{3-6}$cyloalkyl ring;

x is 0-2; and

---- represents a single or a double bond.

When ---- is a single bond in the compounds of Formula I, it is an embodiment of the invention that the stereochemistry at carbon 17 is that of natural 1α,25-dihydroxy vitamin $D_3$(i.e. R). It is to be understood that, while the

relative stereochemistry for compounds of Formula I in an embodiment of the invention, is that of natural 1α,25-dihydroxy vitamin $D_3$ such compounds may contain certain amounts of the unnatural isomer, for example, less than about 25%, preferably less than about 20%, more preferably, less than about 10%.

In specific embodiments of the present invention, the compounds of the invention include:

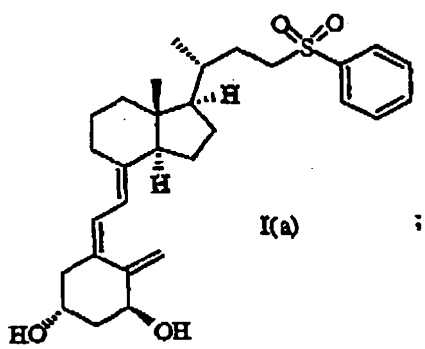
I(a) ;

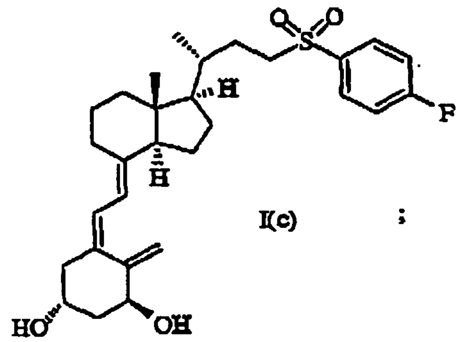
I(c) ;

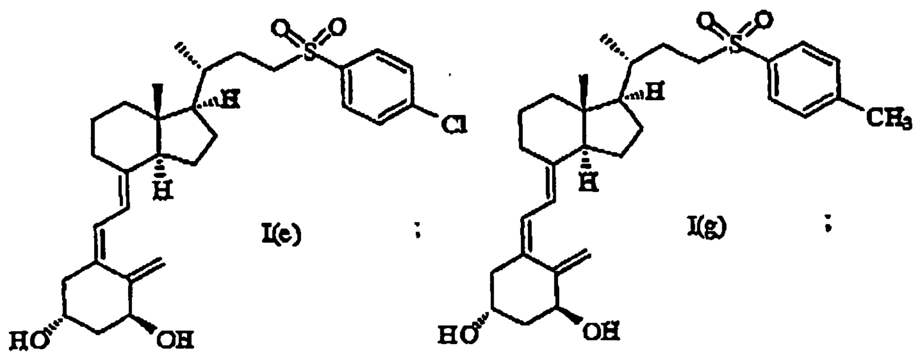
I(e) ;

I(g) ;

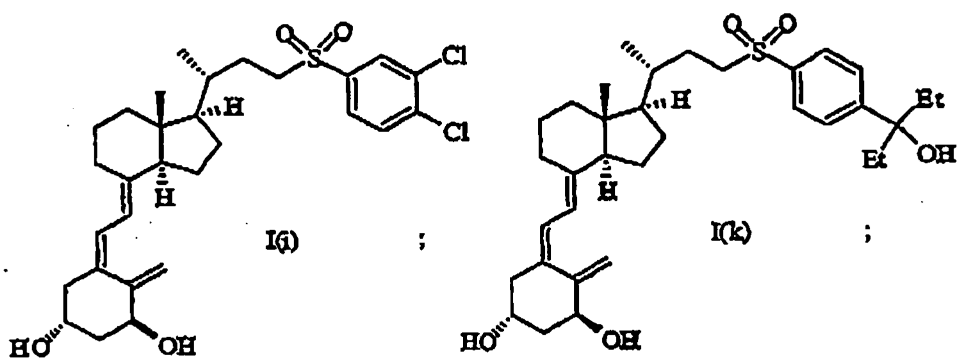
I(i) ;

I(k) ;

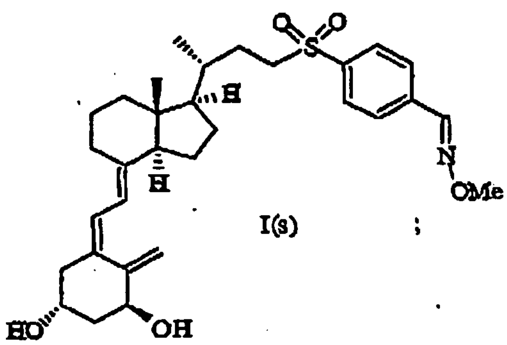

I(s) ;

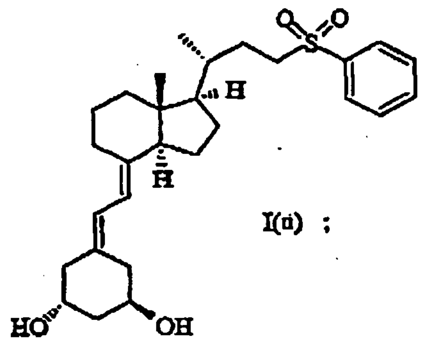

I(u) ;

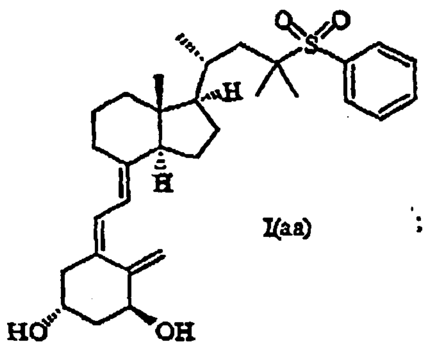

I(aa) ;

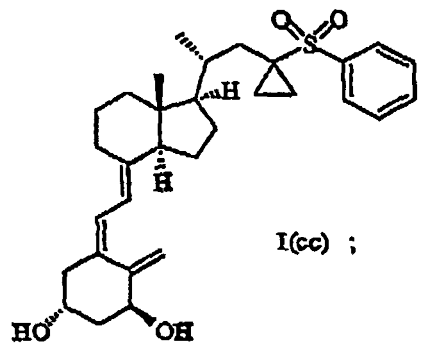

I(cc) ;

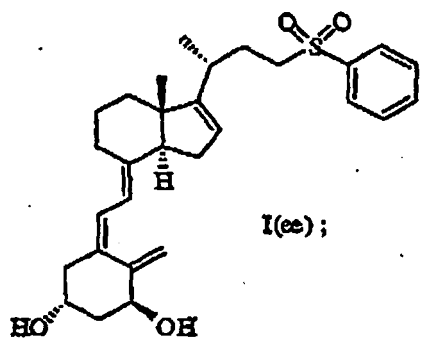

I(ee) ;

and pharmaceutically acceptable salts, hydrates, solvates and prodrugs thereof, wherein the prodrug is an ester prodrug selected from the group consisting of phenyl esters, aliphatic $C_8$-$C_{24}$ esters, acyloxymethyl esters, carbomates and amino acid esters.

### III. Methods of Preparing Compounds of the Invention

In accordance with another aspect of the present invention, the compounds as defined in the claims can be prepared by processes analogous to those established in the art. Therefore, compounds as defined in the claims may be prepared, for example, by the reaction sequence shown in Scheme 1:

## Scheme 1

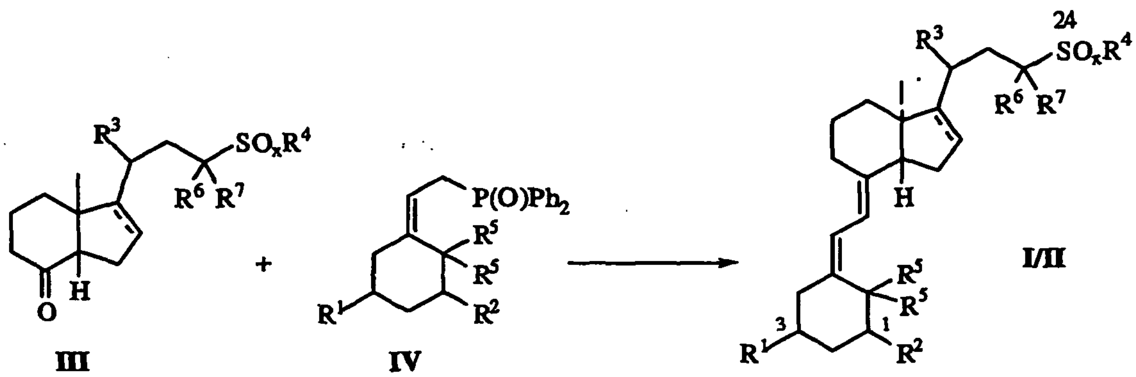

III IV I/II

Ketones of Formula III, wherein $R^3$, $R^4$, $R^5$, $R^6$, x and ---- are as defined in Formulae I ad II, may be reacted with phosphine oxides of Formula IV, wherein $R^1$, $R^2$ and $R^5$ are as defined in Formula I, under standard Homer-Wadsworth-Emmons (HWE) coupling conditions. Therefore phosphine oxides IV, wherein $R^1$, $R^2$ and $R^5$ are as defined in Formula I, are treated with a strong base, for example an alkyl lithium such as n-butyl lithum, under anhydrous conditions in an inert atmosphere and solvent, for example tetrahydrofuran and, at temperatures in the range of about -60 ˚C to about 90 ˚C, suitably at about -78 ˚C. To the resulting intermediate ylide is added a cold, preferably at about -78 ˚C, solution of a ketone III in an inert solvent such as THF while maintaining the anhydrous conditions. After removal of any protecting groups using standard chemistries (if needed), compounds of Formula I may be obtained.

Ketones of Formula III, wherein wherein $R^3$, $R^4$, $R^5$, $R^6$, x and ---- are as defined in Formula I, may be prepared, for example, as shown in Scheme 2:

## Scheme 2

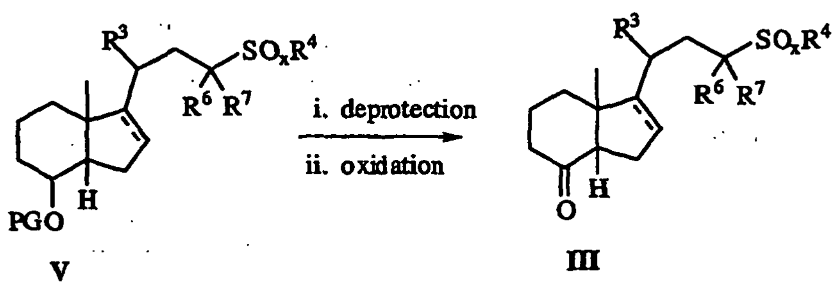


V III

Suitably protected oxysulfones V, wherein $R^3$, $R^4$, $R^5$, $R^6$, x and ---- are as defined in Formula I and PG is a suitable protecting group, are first deprotected and then oxidized to provide ketones III, wherein $R^3$, $R^4$, $R^5$, $R^6$, x and ---- are as defined in Formula I. For example, when PG is trialkyl silyl, such as triethyl silyl, deprotection may be affected by reacting compounds of Formula V with tetrabutylammonium fluoride (TBAF) in an inert solvent, such as THF, and in an inert atmosphere, suitably at about room temperature. Oxidation of the resulting alcohol may be performed, for example, using pyridinium dichromate (PDC), tetrapropylammonium perruthenate (TPAP)/morpholine N-oxide (NMO), or any other suitable oxidizing agent, in an inert solvent such as methylene chloride, under standard conditions.

Compounds of Formula V, wherein $R^3$, $R^4$, $R^6$, $R^7$, x and ---- are as defined in Formula I and PG is a suitable protecting group, may be obtained, for example, as shown in Scheme 3:

## Scheme 3

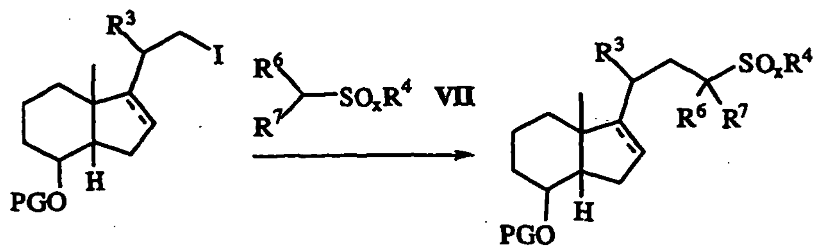


VI V

Compounds of Formula VI, wherein $R^3$ and ---- are as defined in Formula I and PG is a suitable protecting group, may be reacted with the anion of compounds of Formula VII, wherein $R^4$, $R^6$, $R^7$, x and ---- are as defined in Formula I, under anhydrous conditions at temperatures in the range of about -60 °C to about -90 °C, suitably at about -78 °C. The anions of compounds of Formula VII may be prepared by treating compounds of Formula VII with a strong base, for example an alkyl lithium such as n-butyl lithium, under inert conditions and, in the presence of hexamethyl phosphoramide (HMPA), for example, or N,N,$N^1$,$N^1$-tetramethylethylenediamine (TMEDA).

Compounds of Formula VII, wherein $R^4$, $R^6$ and $R^7$ are as defined in Formula I and x is 1 or 2, are either commercially available or may be prepared, for example, by the oxidation of the corresponding compounds of Formula VII, wherein $R^4$, $R^6$ and $R^7$ are as defined in Formula I and x is 0, as shown in Scheme 4. Suitable oxidizing agents include Ozone®, m-chloroperbenzoic acid and $RuCL_3 \cdot H_2O$/periodic acid ($H_5IO_6$). The use of sterically hindered oxidizing reagents assists in the isolation of the sulfoxide (i.e. compounds of Formula VII, where x = 1). An example of such an oxidizing reagent is camphorsulfonyl oxaziridine (available as pure enantiomers which can lead to the formation of enantiomerically enriched sulfoxides).

## Scheme 4

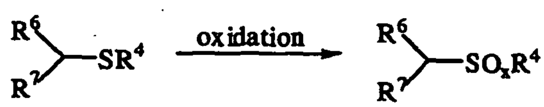


VII, x = 0 VII, x = 1,2

Compounds of Formula VII, wherein $R^4$, $R^6$ and $R^7$ are as defined in Formula I and x is 0, are either commercially available or may be prepared, for example, as shown in Scheme 5. Therefore a reagent of Formula VIII, wherein $R^6$ and $R^7$ are as defined in Formula I and LG is a suitable leaving group, such as halogen, may be reacted with a compound of Formula IX, wherein $R^4$ is as defined in Formula I, in the presence of a base, for example sodium methoxide and an inert solvent, to provide compounds of Formula VII, wherein $R^4$, $R^6$ and $R^7$ are as defined in Formula I and x is 0.

**Scheme 5**

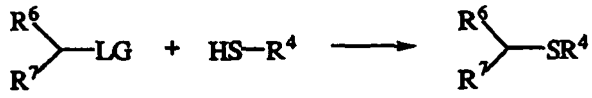

VIII IX VII, x = 0

An alternate route to the compounds of Formula V, wherein $R^3$, $R^4$, x and ---- are as defined in Formula I, $R^6$ and $R^7$ are H and PG is a suitable protecting group, is shown in Scheme 6. Accordingly, a compound of Formula X wherein $R^3$ and ---- are as defined in Formula I and PG is a suitable protecting group, may be reacted with a compound of Formula IX, wherein $R^4$ is as defined in Formula I, in the presence of a base such as 1,8-diazabicyclo[5.4.0]undec-7-ene (DBU), at elevated temperatures, such as about 110-150 °C, suitably at about 130 °C, in an inert, high-boiling solvent, such as benzene, to provide a compound of Formula V, wherein $R^3$, $R^4$, and ---- are as defined in Formula I, $R^6$ and $R^7$ are H, x is 0 and PG is a suitable protecting group. Oxidation of a compound of Formula V, wherein $R^3$, $R^4$, and ---- are as defined in Formula I, $R^6$ and $R^7$ are H, x is 0 and PG is a suitable protecting group, with suitable oxidizing agents, provides compounds of Formula V, wherein $R^3$, $R^4$, and ---- are as defined in Formula I, $R^6$ and $R^7$ are H, x is 1 or 2 and PG is a suitable protecting group. Suitable oxidizing agents include, for example Ozone®, m-chloroperbenzoic acid and $RuCl_3 \cdot H_2O$/periodic acid ($H_5IO_6$). The use of sterically hindered oxidizing reagents assists in the isolation of the sulfoxide (i.e. compounds of Formula V, where x = 1). An example of such an oxidizing reagent is camphorsulfonyl oxaziridine (available as pure enantiomers which can lead to the formation of enantiomerically enriched sulfoxides).

**Scheme 6**

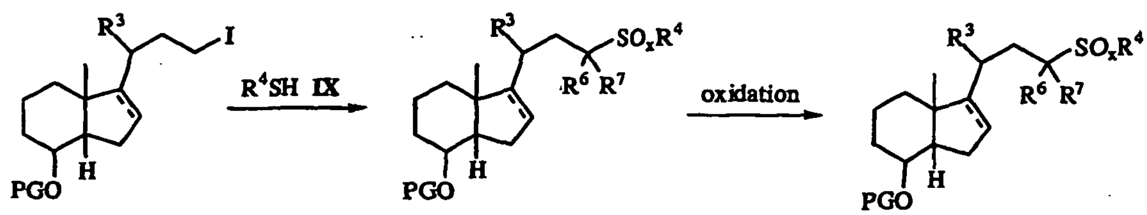


X V, $R^6$ and $R^7$ = H, x = 0 V, $R^6$ and $R^7$ = H, x = 1 or 2

Compounds of Formula V, wherein $R^3$, $R^4$ and ---- are as defined in Formula I, x is 1 or 2, $R^7$ and $R^8$ are both H and PG is a suitable protecting group, may alternatively be prepared from aldehyde XI as shown in Scheme 7. Therefore, a compound of Formula VII, wherein $R^4$ is as defined in Formula I, $R^6$ and $R^7$ are both H, and x is 1 or 2, is first treated with a strong base, such as an alkyl lithium, such as n-butyl lithium, under inert conditions and, in the presence of hexamethyl phosphoramide (HMPA), for example, or N,N,$N^1$,$N^1$-tetramethylethylenediamine (TMEDA), to generate the corresponding anion, which is then reacted with a compound of Formula XI, wherein $R^3$ and ---- are as defined in Formula I and PG is a suitable protecting group, under anhydrous conditions at temperatures in the range of about -60 °C to about 90 °C, suitably at about -78 °C. The resulting $\alpha,\beta$-unsaturated sulfone may then be hydrogenated, for example, in the presence of $H_2$ over palladium on carbon, to provide compounds of Formula V, wherein $R^3$, $R^4$ and ---- are as defined in Formula I, x is 1 or 2, $R^7$ and $R^8$ are both H and PG is a suitable protecting group.

## Scheme 7

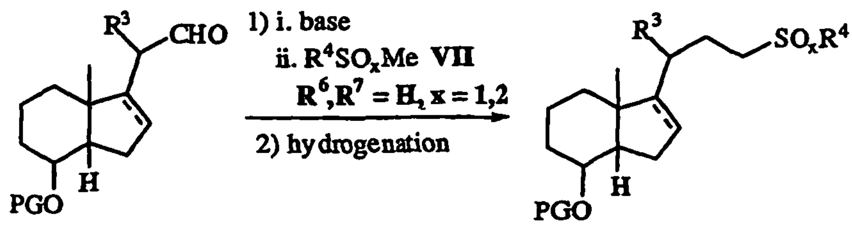


XI

V, $R^6$ and $R^7$ = H, x = 1,2

Aldehydes of Formula XI, wherein $R^3$ and ---- are as defined in Formula I and PG is a suitable protecting group may be prepared using standard chemistries, for example as shown in Scheme 8. The alcohol groups of compounds of Formula XII, wherein $R^3$ and ---- are as defined in Formula I, may be selectively protected to form compounds of Formula XII, wherein PG and PG are the protecting groups for the primary and secondary alcohol groups respectively, using standard chemistries (see "Protective Groups in Organic Chemistry" McOmie, J.F.W. Ed., Plenum Press, 1973 and in Greene, T.W. and Wuts, P.G.M., "Protective Groups in Organic Synthesis", John Wiley & Sons, 1991.). The primary protected alcohol tosylate of compounds of Formula XII may then be selectively oxidized directly to the corresponding aldehyde XI, for example, in the presence of sodium hydrogen carbonate in a polar aprotic solvent such as dimethlysulfoxide (DMSO) at an elevated temperature, for example 150°C, using a procedure described in Komblum, et al. J. Am Chem. Soc. 1959, 81:4113-4116.

## Scheme 8

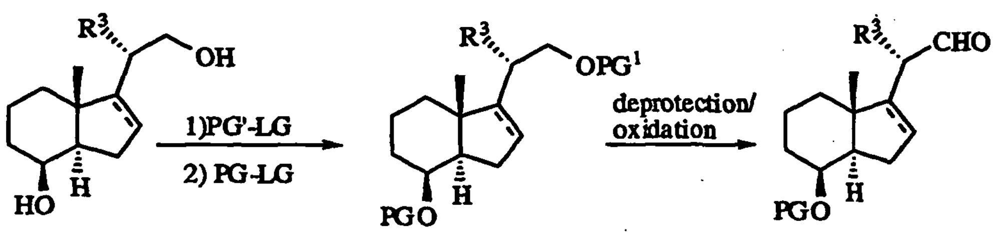


XII XIII XI

The preparation of compounds of Formula VI, wherein $R^3$ and ---- are as defined in Formula I, and PG is a suitable protecting group, is known in the art. Therefore compounds of Formula VI may be prepared as described in Posner, G. H. et al. J. Med Chem .1992, 42, 3425-3435, the contents of which are incorporated herein by reference.

The preparation of compounds of Formula X, wherein $R^3$ and ---- are as defined in Formula I, and PG is a suitable protecting group, is known in the art. Therefore compounds of Formula X may be prepared as described in Posner, G. H. et al. J Med. Chem .1992, 42, 3425-3435; in Jaekyoo Lee, Ph.D. Thesis, 1997, Johns Hopkins University; or in Posner G.H. et al. US Patent No. 6,380,408, the contents of which are incorporated herein by reference.

The preparation of compounds of Formula IV, wherein $R^1$, $R^2$ and $R^5$ are as defined in Formula I is known in the art Therefore compounds of Formula IV, wherein $R^1$ and $R^2$ are as define in Formula I and both $R^5$'s together form $=CH_2$, may be prepared as described in Posner, G. H. et al. J. Med Chem. 1992, 35, 3280-3287, the contents of which are incorporated herein by reference. Compounds of Formula IV, wherein $R^1$ and $R^2$ are as define in Formula I and both $R^5$'s are is H, may be prepared as described in Hilpert, H. and Wirz, B. Tetrahedron 2001, 57, 681-694, the contents of which are incorporated herein by reference.

The preparation of compounds of Formula XII, where $R^3$ and ---- are as defined in Formula I is known. Therefore compounds of Formula XII, where $R^3$ and ---- are as defined in Formula I, may be prepared as described in Posner, G.

H. et al. J. Org. Chem. 1997, 62, 3299-3314, the contents of which are incorporated herein by reference.

The preparation of enantiomerically pure compounds of Formula I and or II, may be accomplished by using enantiomerically pure compounds of Formula III and IV in the reaction shown in Scheme I. In this reaction, a mixture of the 1α, 3β and 1β, 3α diasteromers is typically obtained, with the 1α,3β diastereomer as the major product. These diasteromers may be separated using chromatography, for example using high performance liquid chromatography (HPLC).

In some cases the chemistries outlined above may have to be modified, for instance by use of protective groups, to prevent side reactions due to reactive groups, such as reactive groups attached as substituents. This may be achieved by means of conventional protecting groups, for example as described in "Protective Groups in Organic Chemistry" McOmie, J.F.W. Ed., Plenum Press, 1973 and in Greene, T.W. and Wuts, P.G.M., "Protective Groups in Organic Synthesis", John Wiley & Sons, 1991.

The formation of a desired compound salt is achieved using standard techniques. For example, the neutral compound is treated with an acid or base in a suitable solvent and the Formed salt is isolated by filtration, extraction or any other suitable method.

The formation of solvates of the compounds as defined in the claims will vary depending on the compound and the solvate. In general, solvates are formed by dissolving the compound in the appropriate solvent and isolating the solvate by cooling or using an antisolvent. The solvate is typically dried or azeotroped under ambient conditions.

Prodrugs of the compounds as defined in the claims are phenyl esters, aliphatic ($C_8$-$C_{24}$) esters, acyloxymethyl esters, carbamates and amino acid esters.

A radiolabeled compound as defined in the claims may be prepared using standard methods known in the art. For example, tritium may be incorporated into a compound of the invention using standard techniques, for example by hydrogenation of a suitable precursor to a compound of the invention using tritium gas and a catalyst Alternatively, a compound of the invention containing radioactive iodo may be prepared from the corresponding trialkyltin (suitably trimethyltin) derivative using standard iodination conditions, such as [$^{125}$I] sodium iodide in the presence of chloramine-T in a suitable solvent, such as dimethylformamide. The trialkyltin compound may be prepared from the corresponding non-radioactive halo, suitably iodo, compound using standard palladium-catalyzed stannylation conditions, for example hexamethylditin in the presence of tetrakis(triphenylphosphine) palladium (0) in an inert solvent, such as dioxane, and at elevated temperatures, suitably 50-100°C.

## IV. Uses

As hereinbefore mentioned, novel compounds of the Formula I have been prepared. Accordingly, the present invention includes all uses of the compounds as defined in the claims including their use in therapeutic methods and compositions for modulating cell prolifieration.

Inhibiting catabolism of calcitriol is expected to lengthen the biological lifetime of this hormone and thus to allow smaller amounts of it to be used for effective human chemotherapy; such smaller dosing is expected to avoid, or at least to the hypercalcemic toxicity associated with medicinal use of calcitriol. Selectively inhibiting the cytochrome P450 enzymatic pathway, through which calcitriol is catabolized (mainly via C-24 hydroxylation), is one important way to prolong the lifetime of this hormone. Therefore, the compounds of Formula I were tested *in vitro,* using a standard protocol, for their ability to inhibit specifically CYP24, responsible for 24-hydroxylation of calcitriol. Antimycotic ketoconazole, a drug used clinically for chemotherapy of human prostate cancer (Trachtenberg, J. et al. J. Urol. 1984, J32,61-63), was used as a control standard for inhibition of CYP24. Compounds of the invention have been shown to Selectively inhibit the CYP24 (see Table 1).

In standard hypercalcemia assays, compound Ia did not increase the levels of calcium in the urine at concentration that were 20-fold higher than the concentration of calcitriol (1α,25-dihydroxy vitamin $D_3$) that provided an increase in the calcium levels in the urine, Compound 1(u) was also tested and found to be strongly non-calcemic.

The compounds as defined in the claims are CYP24 modulators and are useful in modulating CYP24 activity, including the inhibition of CYP24 activity, for the treatment of various conditions such as all cell proliferative disorders.

By selectively modulating CYP24, the enzyme that metabolizes 1α,25-dihydroxy vitamin $D_3$, the levels of 1α, 25-dihydroxy vitamin $D_3$ may also be modulated. Diseases that benefit from a modulation of the levels of 1α,25-dihydroxy vitamin $D_3$ can therefore be treated using a modulator of CYP24. By acting preferentially on CYP24, side effects caused by interaction with other enzymes and receptors may be reduced.

Inhibition of CYP24, should inhibit the catabolism 1α,25-dihydroxy vitamin $D_3$.

Other diseases which may benefit for a modulation in the levels of 1α,25-dihydroxy vitamin $D_3$ include, but are not limited to:

i. in the parathyroid - hyper- and hypo-parathyroidism, Osudohypo-parathyroidism, Secondary hyperparathyroidism;
ii. in the pancreas - diabetes;

iii. in the thyroid - medullary carcinoma;
iv. in the skin-psoriasis, wound healing;
v. in the lung - sarcoidosis and tuberculosis;
vi. in the kidney - chronic renal disease, hypophosphtatemic VDRR, vitamin D dependent rickets;
vii. in the bone - anticonvulsant treatment, fibrogenisis imperfecta ossium, osteitits fibrosa cystica, osteomalacia, osteporosis, osteopenia, osteosclerosis, renal osteodytrophy, rickets;
viii. in the intestine - glucocorticoid antagonism, idopathic hypercalcemia, malabsorption syndrome, steatorrhea, tropical sprue.

The present invention further includes a use of a compound as defined in the claims to prepare a medicament to inhibit cell proliferation, preferably cancer cell proliferation. In particular, the method of the invention is useful in inhibiting the proliferation of abnormal but not normal cells. Abnormal cells include any type of cell that is causative of or involved in a disease or condition and wherein it is desirable to modulate, or inhibit the proliferation of the abnormal cell to treat the disease or condition. Examples of abnormal cells include malignant or cancerous cells as well as cell that over-proliferate in inflammatory conditions such as psoriasis. Preferably, the cell proliferative disorder is cancer, in particular cancer of the breast, prostate and lung.

While the compounds as defined in the claims may act by modulating CYP24 activity, one of skill in the art will appreciate that other modes or mechanisms of action for the compounds of the invention are possible.

One skilled in the art can determine which compounds would have therapeutic utility, for example, in inhibiting cell proliferation in any type of cancer or cell proliferate disorder. Compounds may be examine for their efficacy in inhibiting cell growth in cell proliferation assays such as inhibition of growth of murine keratinocyte cells (cell line PE) and for the inhibition of TPA induced ornithine decarboxylase (ODC) activity as described in US. Patent No. 5,830,885, the contents of which are incorporated herein by reference.

In addition to cancer, the compounds as defined in the claims are useful in treating other conditions involving aberrant or abnormal cell proliferation. Other cell proliferative disorders that may be treated by the present invention include inflammatory diseases, allergies, autoimmune disease, graft rejection, psoriasis, restenosis, artherosclerosis, and any other disorder wherein it is desirable to inhibit, prevent or suppress cell growth. Compounds of the invention may be tested for their efficacy in a particular cell proliferation disorder using assays and techniques known to those of skiN in the art. For example, the following references provide assays for various conditions: Rheumatoid Arthritis: "Regulation of IL-15 - Simulated TNF-alpha Production by Rolipram", Journal of Immunology (1999) volume 163 page 8236 by C. S. Kasyapa et al.; Allergy: "A novel Lyn-Binding Peptide Inhibitor Blocks Eosinophil Differentiation, Survival, and Airway eosinophilic inflammation". Journal of Immunology (1999) volume 163 page 939 by T. Adachi et al; Psoriasis: Journal of Immunology (2000) volume 165 page 224 "Inhibition of Keratinocyte apoptosis by IL-15: a new parameter in the pathegenosis of psoriasis" by R. Üchert; and Psoriasis: International Archives of allergy and Immunology (2000) Volume 123 page 275. "T-cell receptor mimic peptides and their potential application in T-cell mediated disease "by A. H. Enk.

The compounds as defined in the claims are preferably formulated into pharmaceutical compositions for administration to human subjects in a biologically compatible form suitable for administration *in vivo.* Accordingly, in another aspect, the present invention provides a pharmaceutical composition comprising a compound as defined in the claims in admixture with a suitable diluent or carrier.

The compositions containing the compounds as defined in the claims can be prepared by known methods for the preparation of pharmaceutically acceptable compositions which can be administered to subjects, such that an effective quantity of the active substance is combined in a mixture with a pharmaceutically acceptable vehicle. Suitable vehicles are described, for example, in Remington's Pharmaceutical Sciences (Remington's Pharmaceutical Sciences, Mack Publishing Company, Easton, Pa., USA 1985). On this basis, the compositions include, albeit not exclusively, solutions of the substances in association with one or more pharmaceutically acceptable vehicles or diluents, and contained in buffered solutions with a suitable pH and iso-osmotic with the physiological fluids.

The compounds as defined in the claims may be used in the form of the free base, in the form of salts, solvates and as hydrates. All forms are within the scope of the invention. Acid addition salts may be formed and provide a more convenient form for use; in practice, use of the salt form inherently amounts to use of the base form. The acids which can be used to prepare the acid addition salts include preferably those which produce, when combined with the free base, form pharmaceutically acceptable salts, that is, salts whose anions are non-toxic to the animal organism in pharmaceutical doses of the salts, so that the beneficial properties inherent in the free base are not vitiated by side effects ascribable to the anions. Although pharmaceutically acceptable salts of the basic compounds are preferred, all acid addition salts are useful as sources of the free base form even if the particular salt per se is desired only as an intermediate product as, for example, when the salt is formed only for the purposes of purification and identification, or when it is used as an intermediate in preparing a pharmaceutically acceptable salt by ion exchange procedures.

The compositions as defined in the claims may be administered, for example, by oral, parenteral, buccal,

sublingual, nasal, rectal, patch, pump or transdermal administration and the pharmaceutical compositions formulated accordingly. Parenteral administration includes intravenous, intraperitoneal, subcutaneous, intramuscular, transepithelial, nasal, intrapulmonary, intrathecal, rectal and topical modes of administration. Parenteral administration may be by continuous infusion over a selected period of time.

A compound as defined in the claims may be orally administered, for example, with an inert diluent or with an assimilable edible carder, or it may be enclosed in hard or soft shell gelatin capsules, or it may be compressed into tablets, or it may be incorporated directly, with the food of the diet. For oral therapeutic administration, the compound as defined in the claims may be incorporated with excipient and used in the form of ingestible tablets, buccal tablets, troches, capsules, elixirs, suspensions, syrups, wafers, and the like.

A compound of the invention may also be administered parenterally. Solutions of a compound of the invention can be prepared in water suitably mixed with a surfactant such as hydroxypropylcellulose. Dispersions can also be prepared in glycerol, liquid polyethylene glycols, DMSO and mixtures thereof with or without alcohol, and in oils. Under ordinary conditions of storage and use, these preparations contain a preservative to prevent the growth of microorganisms. A person skilled in the art would know how to prepare suitable formulations. Conventional procedures and ingredients for the selection and preparation of suitable formulations are described, for example, in Remington's Pharmaceutical Sciences (1990 - 18th edition) and in The United States Pharmacopeia: The National Formulary (USP 24 NF19) published in 1999.

The pharmaceutical forms suitable for injectable use include sterile aqueous solutions or dispersion and sterile powders for the extemporaneous preparation of sterile injectable solutions or dispersions. In all cases the form must be sterile and must be fluid to the extent that easy syringability exists.

Compositions for nasal administration may conveniently be formulated as aerosols, drops, gels and powders. Aerosol formulations typically comprise a solution or fine suspension of the active substance in a physiologically acceptable aqueous or non-aqueous solvent and are usually presented in single or multidose quantities in sterile form in a sealed container, which can take the form of a cartridge or refill for use with an atomizing device. Alternatively, the sealed container may be a unitary dispensing device such as a single dose nasal inhaler or an aerosol dispenser fitted with a metering valve which is intended for disposal after use. Where the dosage form comprises an aerosol dispenser, it will contain a propellant which can be a compressed gas such as compressed air or an organic propellant such as fluorochlorohydrocarbon. The aerosol dosage forms can also take the form of a pump-atomizer.

Compositions suitable for buccal or sublingual administration include tablets, lozenges, and pastilles, wherein the active ingredient is formulated with a carrier such as sugar, acacia, tragacanth, or gelatin and glycerine. Compositions for rectal administration are conveniently in the form of suppositories containing a conventional suppository base such as cocoa butter.

The compounds as defined in the claims may be administered to an animal alone or in combination with pharmaceutically acceptable carriers, as noted above, the proportion of which is determined by the solubility and chemical nature of the compound, chosen route of administration and standard pharmaceutical practice.

The dosage of the compounds and/or compositions as defined in the claims can vary depending on many factors such as the pharmacodynamic properties of the compound, the mode of administration, the age, health and weight of the recipient, the nature and extent of the symptoms, the frequency of the treatment and the type of concurrent treatment, if any, and the clearance rate of the compound in the animal to be treated. One of skill in the art can determine the dosage based on the above factors. The compounds as defined in the claims may be administered initially in a suitable dosage that may be adjusted as required, depending on the clinical response. For ex vivo treatment of cells over a short period, for example for 30 minutes to 1 hour or longer, higher doses of compound may be used than for long term in vivo therapy.

The compounds as defined in the claims can be used alone or in combination with other agents that modulate CYP24 activity or in combination with other types of treatment. (which may or may not modulate CYP24) for cell proliferative disorders or other disorders that benefit from a modulation in the levels of 1$\alpha$,25-dihydroxy vitamin $D_3$ and/or an inhibition of the catabolism of 1$\alpha$,25-dihydroxy vitamin $D_3$. Preferably the compounds of the invention may be administered in combination with 1$\alpha$,25-dihydroxy vitamin $D_3$ (calcitriol) or other vitamin D receptor agonists. Inhibiting catabolism of vitamin D receptor agonists is expected, to lengthen the biological lifetime or efficacy of these therapies and thus to allow smaller amounts of the drug to be used for effective human chemotherapy; such smaller dosing is expected to avoid, or at least to minimize, the hypercalcemic toxicity associated with medicinal use of calcitriol or other vitamin D receptor agonists.

The general treatments are based on the cancer type and do not specifically target CYP24 activity. In a particular aspect of the present invention, the compounds as defined in the claims may be used in combination with other therapies and therapeutics to treat dermatological disorders, bone disorders, cancer and osteoporosis.

The following non-limiting examples are illustrative of the present invention:

## EXAMPLES

### Materials and Methods

Unless otherwise noted, all reactions were performed in oven-dried glassware stirred under an atmosphere of ultra-high-purity argon. THF was distilled from Na/benzophenone ketyl and $CH_2Cl_2$, distilled from $CaH_2$ immediately prior to use. Organolithiums were titrated prior to use following known methods (Suffert, J. J. Org. Chem. 1989, 54, 509-510). All other reagents were used as received from commercial suppliers. Analytical TLC analysis was conducted on precoated glass-backed silica gel plates (Merck Kieselgel 60 $F_{254}$, 250 mm thickness) and visualized with *p*-anisaldehyde or $KMnO_4$ stains. Column chromatography was performed using short path silica gel (particle size < 230 mesh) or flash silica gel (particle size 230-400 mesh). Preparative-plate chromatography was performed using silica-gel-coated glass preparative plates (500-1000 μm) from Analtech and analyzed by UV. HPLC was carried out using a Rainin HPLX™ system equipped with two 25-mL/min preparative pump heads using (1) a Chiral Technologies CHIRALCEL® OJ 10-mm x 250-mm (semipreparative) column packed with cellulose tris(4-methylbenzoate) on a 10 μm silica-gel substrate or (2) a Phenomenex LUNA™ 10-mm x 250-mm (semipreparative) column packed with 110 Å silica gel (5 μm pore size) as C-18-bonded silica and a Rainin Dynamax™ UV-C dual-beam variable-wavelength detector set at 254 nm. Yields are reported for pure products (>95% based on their chromatographic and spectroscopic homogeneity) and are unoptimized. Melting points were determined in open capillaries using a Mel-Temp metal-block apparatus and are uncorrected. Optical rotations were measured at the Na line using a Perkin-Elmer 141 Polarimeter. NMR spectra were obtained on a Varian XL-400 spectrometer operating at 400 MHz for $^1H$, 376 MHz for $^{19}F$, and 100 MHz for $^{13}C$ and a Bruker 300 AMX spectrometer operating at 300 MHz for $^1H$. Chemical shifts are reported in ppm (δ) and are referenced to $CDCl_3$ (7.26 ppm for $^1H$ and 77.0 ppm for $^{13}C$), tetramethylsilane (TMS, 0.00 ppm for $^1H$), and $CFCl_3$ (0.00 ppm for $^{19}F$). IR spectra were obtained using a Perkin Elmer 1600 Series FT-IR instrument HRMS were obtained at the mass spectrometry facility at the Ohio State University on a Micromass QTOF Electrospray mass spectrometer. Elemental analyses were performed by Atlantic Microlab Inc., Norcross, GA.

**Example 1: General Procedure for the Preparation of Aryl Methyl Sulfones VII**

To an ice-cold solution containing the appropriate aryl methyl sulfide (6.00 mmol) in MeOH (24.0 mL) was added oxone® (9.00 mmol) as a solution in $H_2O$ (20.0 mL) dropwise via addition funnel. The resulting cloudy slurry was stirred at room temperature overnight, diluted with water, and extracted with $CHCl_3$ (3X). The combined organics were washed with water and brine, dried over $Na_2SO_4$, and concentrated under reduced pressure to give essentially quantitative recovery of the aryl methyl sulfones VII (a-c) as crystalline solids.

a) **Methyl-(4-methozyphenyl) sulfone.** According to the general procedure for the preparation of aryl methyl sulfones described above, 1-methanesulfanyl-4-methoxybenzene (1.00 g, 6.48 mmol) gave 1.20 g (99%) of the title compound as a white solid: mp 114-115 ˚C (lit. mp 115 ˚C, Helv. Chim. Acta. 1999, 82, 372-388); $^1H$ NMR (400 MHz, $CDCl_3$) δ 7.89-7.83 (dt, *J* = 9.5, 2.8, 2.2 Hz, 2H), 7.04-6.99 (dt, *J* = 9.5, 2.8, 2.2 Hz, 2H), 3.88 (s, 3H), 3.02 (s, 3H); $^{13}C$ NMR (100 MHz, $CDCl_3$) δ 163.6, 132.3, 129.5, 114.5, 55.7, 44.8; IR (neat) 3020, 3010, 2982, 1575, 1412, 1323, 1293, 1142, 1092, 1023, 835, 766, 544, 528 $cm^{-1}$; Anal. Calcd for $C_8H_{10}O_3S$: C, 51.60; H, 5.41. Found: C, 51.64; H, 5.43.

b) **Methyl-(4-nitrophenyn sulfone.** According to the general procedure for the preparation of aryl methyl sulfones described above, 1-methanesulfanyl-4-nitrobenzene (1.00 g, 5.41 mmol) gave 1.19 g (100%) of the title compound as a yellow solid: mp 137-139 ˚C; $^1H$ NMR (400 MHz; $CDCl_3$) δ 8.45-8.40 (dt, *J* = 9.0, 2.5 Hz, 2H), 8.19-8.14 (dt, *J* = 9.0, 2.5 Hz, 2H), 3.12 (s, 3H).

c) **Methyl-(4-trifluoromethylphenyl) sulfone.** According to the general procedure for the preparation of aryl methyl sulfones described above, 1-methanesulfanyl-4-trifluoromethylbenzene synthesized from 4-chloro-l-trifluoromethyl benzene and sodium methanethiolate as described in Cabiddu, M.G. et al., J. Organometallic Chem. 1997, 531, 125-140. (1.07 g, 5.57 mmol) gave 1.21 g (97%) of the title compound as a white solid: mp 100-101 ˚C; $^1H$ NMR (300 MHz, $CDCl_3$) δ 8.13-8.07 (d, *J* = 11.1 Hz, 2H), 7.89-7.83 (d, *J* = 11.1 Hz, 2H), 3.09 (s, 3H); $^{13}C$ NMR (100 MHz, $CDCl_3$) δ 143.9, 135.4 (d, *J* = 32.6 Hz), 128.1, 126.5 (d, *J* = 0.8 Hz), 123.0 (d, *J* = 123.0 Hz), 44.3; $^{19}F$ NMR (375 MHz, $CDCl_3$, $CFCl_3$) δ -69.4 (m).

**Example 2: Preparation of Methyl (p-acetalphenyl) sulfone:**

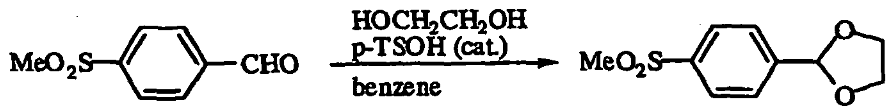


A mixture of 4-methylsulphonyl benzaldehyde(750 mg, 3.87 mmol, 95% purity) and ethylene glycol (0.9 mL, 16.0 mmol) in benzene (10 mL) in the presence of catalytic amount of *p*-TsOH was refluxed for 6.5 h. After benzene was distilled off, the residue was dissolved into EtOAc. The organic layer was washed with brine, saturated aq. $NaHCO_3$, and brine again, dried over $MgSO_4$, filtered, concentrated to afford 781.9 mg (88%) of a crude product which was directly used for the next reaction without further purification. $R_f$ 0.37 (1:1-EtOAc:Hex); $^1H$ NMR (400 MHz, $CDCl_3$) δ 7.95-7.98 (m, 2H), 7.68-7.71 (m, 2H), 5.89 (s, 1H), 4.05-4.15 (m, 4H), 3.05 (s, 3H).

**Example 3: __(Triethylsilyl)-oxy-aryl Sulfones V__**

**(a) __(Triethylsilyl)-oxy-phenyl Sulfone__**

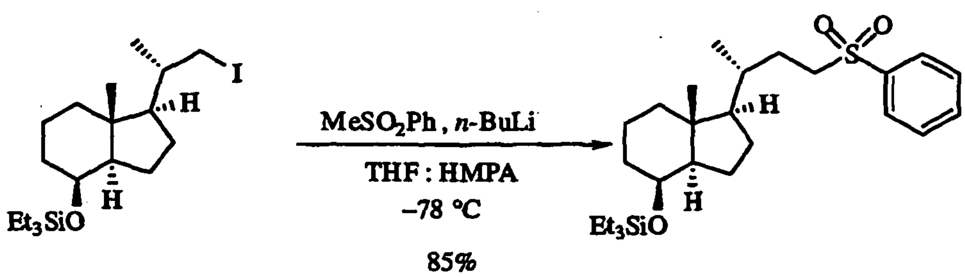


To a cold (-78 ˚C) solution of methyl phenyl sulfone (125 mg, 0.802 mmol) in THF (2.25 mL) was added a solution of *n*-BuLi (556 μL, 0.802 mmol, 1.44 M in hexanes) dropwise via syringe. After 15 min, HMPA (0.1-0.2 mL) was added and the solution was stirred for an additional 15 min at -78 ˚C. A precooled (-78 ˚C) solution of the (+)-triethylsilyl iodide (Posner, G. H.; Crawford, K. R. unpublished results, 100 mg, 0.229 mmol) in THF (0.75 mL) was added slowly via cannula. The reaction mixture was then warmed to room temperature. The reaction was quenched with $H_2O$, extracted with $Et_2O$ (3X), washed with brine, dried over $MgSO_4$, and concentrated to a crude solid that was purified by chromatography (5→20% EtOAc/hexanes) to give 91 mg (85%) of the title compound as a colorless oil: $[\alpha]_D^{25}$ +36.7 (*c* 4.3, $CHCl_3$); $^1H$ NMR (400 MHz, $CDCl_3$) δ 7.92-7.85 (m, 2H), 7.67-7.59 (tt, *J*= 7.4,1.5 Hz,1H), 7.59-7.50 (m, 2H), 4.04-3.96 (m, 1H), 3.16-3.04 (m, 1H), 3.02 2.91 (m, 1H), 0.92 (t, *J* = 8.0 Hz, 9H), 0.84 (s, 3H), 0.83 (d, *J* = 6.8 Hz, 3H), 0.52 (q, *J* = 8.0 Hz, 6H); $^{13}C$ NMR (100 MHz, $CDCl_3$) δ 139.2, 133.5, 129.1, 128.0, 69.2, 55.9, 53.6, 52.9, 42.1, 40.6, 34.4, 34.2, 28.2, 26.9, 22.8, 18.2, 17.5, 13.4, 6.9, 4.9; IR (neat) 2949, 2912, 2873, 1446, 1317, 1306, 1234, 1148, 1087, 1021, 803, 740, 724, 689 $cm^{-1}$; HRMS: calcd for $C_{26}H_{44}O_5SSi$ + Na, 487.2678, found 487.2672.

In a like manner, the following additional compounds were prepared:

**(b) (+)-(Triethylsilyl)-oxy-(4-fluorophenyl) Sulfone:** By replacing methyl phenyl sulfone with methyl (4-fluorophenyl) sulfone. The crude mixture was purified by flash chromatography (EtOAc:Hex = 1:15 to 1:13) to afford 78 mg (85%) of C24-p-fluorophenyl sulfone. $R_f$0.37 (1:9-EtOAc:Hex); $[\alpha]_D^{26}$ +36.0 (c 1.11, $CHCl_3$); $^1H$ NMR (400 MHz, $CDCl_3$) δ 7.90-7.94 (m, 2H), 7.23-7.27 (m, 2H), 4.01 (d, *J*= 2.4 Hz, 1H), 3.12 (ddd, *J* = 4.0, 12.0, 13.6 Hz, 1H), 2.98 (ddd, *J* = 4.8, 11.6, 14.0 Hz, 1H), 1.70-1.88 (m, 2H), 1.61-1.68 (m, 2H), 1.40-1.57 (m, 3H), 1.02-1.38 (m, 8H), 0.93 (t, J = 7.6 Hz, 9H), 0.86 (s, 3H), 0.85 (d, *J*= 7.2 Hz, 3H), 0.54 (q, J= 8.0 Hz, 6H); $^{13}C$ NMR (100 MHz, $CDCl_3$) δ 165.7 (d, *J*= 255.1), 135.2 (d, *J*= 3.2), 130.9 (d, *J*= 9.6), 116.5 (d, *J* = 22.3), 69.2, 55.9, 53.8, 52.9, 42.1, 40.6, 34.5, 34.2, 28.3, 27.0, 22.8, 18.3, 17.6, 13.5, 6.9, 4.9; IR (thin film) 2950, 2876, 1592, 1494, 1321, 1289, 1236, 1148, 1087 $cm^{-1}$; HRMS calc'd for [M+Na]: 505.2578 for $C_{26}H_{43}FO_3SSiNa$. found: 505.2561.

**(c) (+)-Triethylsilyl)-oxy-(4-chlorophenyl) Sulfone: By replacing methyl phenyl** sulfone with methyl (4-chlorophenyl) sulfone. The crude mixture was purified by flash chromatography (EtOAc:Hex = 1:12 to 1:7) to afford 87.5

mg (96%) of C24-p-chlorophenyl sulfone. $R_f$0.35 (1:9-EtOAc:Hex); $[\alpha]_D^{26}$ +37.8 (c 0.91, $CHCl_3$); $^1$H NMR (400 MHz, $CDCl_3$) δ 7.82-7.86 (m, 2H), 7.53-7.56 (m, 2H), 4.01 (m, 1H), 3.11 (ddd, *J*= 4.8,12.0,13.6 Hz, 1H), 2.97 (ddd, *J*= 4.8, 11.6, 14.0 Hz, 1H), 1.76-1.90 (m, 2H), 1.60-1.69 (m, 2H), 1.40-1.58 (m, 3H), 1.02-1.36 (m, 8H), 0.93 (t, *J* = 8.0 Hz, 9H), 0.86 (s, 3H), 0.85 (d, *J* = 6.4 Hz, 3H), 0.54 (q, *J* = 8.0 Hz, 6H); $^{13}$C NMR (100 MHz, $CDCl_3$) δ 140.3, 137.6, 129.5, 69.1, 55.9, 53.7, 52.9, 42.1, 40.6, 34.4, 34.2, 28.2, 27.0, 22.8, 18.2, 17.6, 13.4, 6.9, 4.9; IR (thin film) 2950, 2875, 1312, 1150, 1088 $cm^{-1}$; HRMS calc'd for [M+Na]: 521.2288 for $C_{26}H_{43}ClO_3SSiNa$. found: 521.2275.

**(d) (+)-(Triethylsilyl)-oxy-(4-methylphenyl) Sulfone:** By replacing methyl phenyl sulfone with methyl (4-methyphenyl) sulfone. The crude mixture was purified by flash chromatography (EtOAc:Hex=1:12 to 1:10) to afford 84.6 mg (93%) of C24-p-tolyl sulfone. $R_f$0.23 (1:9-EtOAc:Hex); $[\alpha]_D^{26}$ +36.9 (c 0.96, $CHCl_3$); $^1$H NMR (400 MHz, $CDCl_3$) δ 7.76-7.78 (m, 2H), 7.33-7.36 (m, 2H), 3.99 (d, *J*= 2.4 Hz, 1H), 3.09 (ddd, *J*= 4.0, 12.0, 13.6 Hz, 1H), 2.95 (ddd, *J*= 4.8, 11.6, 14.0 Hz,1H), 2.44 (s, 3H), 1.72-1.87 (m, 3H), 1.62-1.68 (m, 2H), 1.38-1.60 (m, 3H), 1.00-1.35 (m, 7H), 0.92 (t, *J* = 7.6 Hz, 9H), 0.84 (s, 3H), 0.82 (d, *J* = 6.4 Hz, 3H), 0.52 (q, *J* = 7.6 Hz, 6H); $^{13}$C NMR (100 MHz, $CDCl_3$) δ 144.4, 136.2, 129.8, 128.0, 69.2, 55.9, 53.7, 52.9, 42.1, 40.6, 34.5, 34.2, 28.3, 27.0, 22.8, 21.6, 18.2, 17.6, 13.4, 6.9, 4.9; IR (thin film) 2950, 2875, 1598, 1456, 1316, 1148, 1088 $cm^{-1}$; HRMS [M+Na] calc'd 501.2829 for $C_{27}H_{46}O_3SSiNa$ found: 501.2810.

**(e) (+)-(Triethylsilyl)-oxy-(3,4-dichlorophenyl) Sulfone:** By replacing methyl phenyl sulfone with methyl (3,4-dichlorophenyl) sulfone, The crude mixture was purified by flash chromatography (EtOAc:Hex = 1:12) to afford 65.6 mg (66%) of C24-3,4-dichlorophenyl sulfone. $R_f$0.38 (1:9-EtOAc:Hex); $[\alpha]_D^{26}$ +32.2 (c 1.03, $CHCl_3$); $^1$H NMR (400 MHz, $CDCl_3$) δ 8.00 (d, *J* = 2.0 Hz, 1H), 7.73 (dd, J= 2.0, 8.4 Hz, 1H), 7.66 (d, *J* = 8.4 Hz, 1H), 4.02 (d, *J* = 2.4 Hz, 1H), 3.13 (ddd, *J* = 4.8, 12.0, 14.0 Hz, 1H), 2.99 (ddd, J = 4.8, 11.6, 14.0 Hz, 1H), 1.76-1.90 (m, 3H), 1.62-1.73 (m, 2H), 1.42-1.59 (m, 4H), 1.28-1.38 (m, 3H), 1.14-1.26 (m, 2H), 1.06 (dt, *J*= 3.2, 13.2 Hz, 1H), 0.94 (t, *J*= 8.0 Hz, 9H), 0.87 (s, 3H), 0.86 (d, *J*= 6.0 Hz, 3H), 0.54 (q, *J* = 8.0 Hz, 6H); $^{13}$C NMR (100 MHz, $CDCl_3$) δ 139.0, 138.6, 134.0, 131.3, 130.0, 127.1, 69.1, 55.8, 53.7, 52.9, 42.1, 40.6, 34.4, 34.2, 28.1, 27.0, 22.8, 18.2, 17.5, 13.4, 6.9, 4.8; IR (thin film) 2950, 2875, 1455, 1370, 1322, 1156, 1091 $cm^{-1}$; HRMS [M+Na] calc'd 555.1893 for $C_{26}H_{42}Cl_2O_3SSiNa$. found: 555.1886.

**(f) (+)-(Triethylsilyl)-oxy-p-[3-(*tert*-Butyldimethylsiloxy)isopentylphenyl sulfone:** By replacing methyl phenyl sulfone with p-(3-(*tert*-Butyldimethylsiloxy)isopentyl)phenyl methyl sulfone. The crude mixture was purified by flash chromatography (EtOAc:Hex = 1:19 to 1:15) to afford 107.7 mg (94%) of C24-p-[3-(*tert*-Butyldimethylsiloxy)isopentyl] phenyl sulfone. $R_f$0.39 (1:9-EtOAc:Hex); $[\alpha]_D^{26}$ +21.3 (c 0.97, $CHCl_3$); $^1$H NMR (400 MHz, $CDCl_3$) δ 7.82-7.85 (m, 2H), 7.54-7.57 (m, 2H), 4.00 (m, 1H), 3.11 (ddd, *J*= 4.0, 12.0, 13.6 Hz, 1H), 2.98 (ddd, *J* = 4.8, 11.2, 13.6 Hz, 1H), 1.75-1.96 (m, 7H), 1.43-1.66 (m, 5H), 1.24-1.35 (m, 5H), 1.00- 1.20 (m, 2H), 1.00 (s, 9H), 0.93 (s, 9H), 0.84 (d, *J* = 6.0 Hz, 3H), 0.84 (s, 3H), 0.63 (t, *J*= 7.2 Hz, 6H), 0.53 (q, *J*= 8.0 Hz, 6H), 0.16 (s, 6H); $^{13}$C NMR (100 MHz, $CDCl_3$) δ 152.6, 136.5, 127.5, 126.8, 81.5, 69.2, 55.8, 53.6, 52.9, 42.1, 40.6, 35.65, 35.62,34.5, 34.2, 28.3,26.9,26.2,22.8,18.9,18.3,17.6,13.5, 8.2,6.9,4.9, -2.1; IR (thin film) 2952, 2877, 1462, 1318, 1256, 1151, 1025, 836, 800, 771 $cm^{-1}$; HRMS [M+Na] calc'd 687.4269 for $C_{37}H_{68}O_4SSi_2Na$. found: 687.4293.

**(g) (+)-(Triethylsilyl)-oxy-p-Acetalphenyl sulfone:** By replacing methyl phenyl sulfone with *p*-acetalphenyl methyl sulfone (Example 2). The crude mixture was purified by flash chromatography (EtOAc:Hex = 1:4) to afford 83.5 mg (74%) of C24-p-acetalphenyl sulfone. $R_f$0.26 (1:4-EtOAc:Hex); $[\alpha]_D^{26}$ +33.2 (c 1.10, $CHCl_3$); $^1$H NMR (400 MHz, $CDCl_3$) δ 7.91-7.93 (m, 2H), 7.67-7.69 (m, 2H), 5.89 (s, 1H), 4.05-4.15 (m, 4H), 4.01 (m, 1H), 3.14 (ddd, *J* = 4.0, 12.0,13.6 Hz, 1H), 2.97 (ddd, *J* = 4.8, 11.6, 13.6 Hz, 1H), 1.75-1.89 (m, 3H), 1.60-1.70 (m, 2H), 1.40-1.58 (m, 3H), 1.28-1.36 (m, 3H), 1.02- 1.21 (m, 4H), 0.93 (t, *J* = 8.4.Hz, 9H), 0.86 (s, 3H), 0.83 (d, *J* = 6.0 Hz, 3H), 0.54 (q, *J* = 8.4 Hz, 6H); $^{13}$C NMR (100 MHz, $CDCl_3$) δ 143.8, 139.7, 128.1, 127.2, 102.4, 69.1, 65.4, 55.9, 53.6, 52.9, 42.1, 40.6, 34.4, 34.2, 28.1, 26.9, 22.8, 18.2, 17.5, 13.4, 6.8, 4.8; IR (thin film) 2950, 2876, 1316, 1149, 1085, 1018, 973, 948, 744, 725, 547 $cm^{-1}$; HRMS [M+Na] calc'd 559.2884 for $C_{29}H_{48}O_5SSiNa$. found: 559.2930.

In a like manner, the following additional compounds can be prepared:

**(h) (Triethylsilyl)-oxy-(4-methoxyphenyl) sulfone:** By replacing methyl phenyl sulfone with methyl (4-methoxyphenyl) sulfone (Example 1a);

(i) (Triethylsilyl)-oxy-(4-nitrophenyl) sulfone: By replacing methyl phenyl sulfone with methyl (4-nitrophenyl) sulfone (Example 1b); and

**(j) (Triethysilyl)-oxy-(4-trifluoromethyl phenyl) sulfone:** By replacing methylpheny sulfone with methyl (4-trifluoromethyl phenyl) sulfone (Example 1c).

**Example 4: <u>C,D-Ring Ketones III</u>**

**(a) (+)-Ketophenyl sulfone**

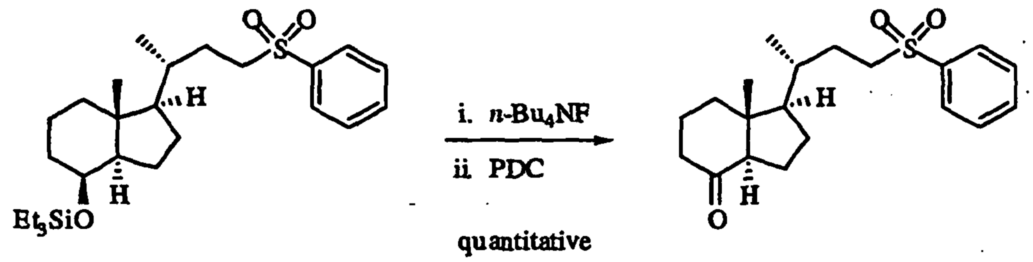


To a solution of (triethylsilyl)-oxyphenyl sulfone (Example 3a, 86 mg, 0.185 mmol) in THF (~0.7 M) was added a solution of tetrabutylammonium fluoride (TBAF, 740 μL, 0.740 mmol, 1.0 M in THF). The reaction mixture was stirred for 18 h and concentrated under reduced pressure to a brown syrup. This brown syrup was then dissolved in $CH_2Cl_2$ and treated with pyridinium dichromate (PDC, 290 mg, 0.555 mmol) and celite® (109 mg) for 12 h. The contents of the flask were then passed through a 1" plug of silica gel, rinsed with EtOAc (3X), concentrated, and purified by flash chromatography (35→40% EtOAc/hexanes) or preparative-plate chromatography (50% EtOAc/hexanes) to afford pure C,D-ring ketone (67 mg) in quantitative yield as a colorless oil: $[\alpha]_D^{25}$ +17.7 (c 4.3, $CH_2Cl_3$); $^1H$ NMR (400 MHz, $CDCl_3$) δ 7.92-7.84 (m, 2H), 7.67-7.60 (tt, *J*= 7.6, 1.7 Hz, 1H), 7.59-7.51 (m, 2H), 3.16-3.04 (m, 1H), 3.03-2.91 (m, 1H), 2.45-2.33 (dd, *J* = 11.4, 7.4 Hz, 1H), 2.29-2.11 (m, 2H), 2.07-1.91 (m, 2H), 0.89 (d, *J* = 6.4 Hz, 3H), 0.52 (s, 3H); $^{13}C$ NMR (100 MHz, $CDCl_3$) δ 139.0, 133.6, 129.2, 127.9, 61.6, 55.7, 53.4, 49.6, 40.7, 38.7, 34.3, 28.1, 27.1, 23.8, 18.9, 18.3, 12.4; IR (neat) 2956,2875, 1709, 1446, 1306, 1145, 1086, 747, 690 $cm^{-1}$; HRMS: calcd for $C_{20}H_{28}O_5S$ + Na, 371.1657, found 371.1664.

In a like manner, the following additional compounds were prepared:

**(b) (+)-Keto-(4-fluorophenyl) Sulfone:** By replacing (triethylsilyl)-oxyphenyl sulfone with (tricthylsilyl)-oxy-(4-fluorophenyl) sulfone (Example 3b). The reaction mixture was directly purified by short path flash chromatography ($CH_2Cl_2$ then EtOAc:Hex = 1:2) to give 50.5 mg (85% for 2 steps) of keto-p-fluorophenyl sulfone. $R_f$0.30 (1:2-EtoAc: Hex); $[\alpha]_D^{26}$ +11.5 (c 0.96, $CHCl_3$); $^1H$ NMR (400 MHz, $CDCl_3$) δ 7.90-7.95 (m, 2H), 7.24-7.28 (m, 2H), 3.13 (ddd, *J* = 4.4, 12.0, 13.6 Hz, 1H), 3.00 (ddd, *J* = 4.8, 11.2, 14.0 Hz, 1H), 2.43 (dd, *J*= 7.6, 1.6 Hz, 1H), 2.17-2.32 (m, 2H), 1.97-2.08 (m, 2H), 1.67-1.94 (m, 4H), 1.45-1.60 (m, 4H), 1.39 (q, *J* = 9.2 Hz, 1H), 1.27 (m, 1H), 0.93 (d, *J*= 6.0 Hz, 3H), 0.61 (s, 3H); $^{13}C$ NMR (100 MHz, $CDCl_3$) δ 211.4,165.7 (d, *J*= 255.1), 135.1 (d,*J*= 3.0), 130.8 (d, *J*= 9.9), 116.6 (d, *J* = 22.8), 61.6, 55.8, 53.6, 49.7, 40.8, 38.7, 34.4, 28.2, 27.2, 23.8, 18.9, 18.3, 12.4; IR (thin film) 2957, 2876, 1710, 1591,1494,1316, 1289,1232, 1144, 1086 $cm^{-1}$; HRMS [M+Na] calc'd 389.1557 for $C_{20}H_{27}FO_3SNa$. found: 389.1547.

**(c) (+)-Keto-(4-chlorophenyl) Sulfone:** By replacing (triethylsilyl)-oxyphenyl sulfone with (triethylsilyl)-oxy-(4-chlorophenyl) sulfone (Example 3c). The reaction mixture was directly purified by short path flash chromatography ($CH_2Cl_2$ then EtOAc:Hex = 1:2) to give 56.2 mg (82% for 2 steps) of keto-p-chlorophenyl sulfone. $R_f$0.34 (1:2-EtOAc: Hex); $[\alpha]_D^{26}$ +12.5 (c 0.97, $CHCl_3$); $^1H$ NMR (400 MHz, $CDCl_3$) δ 7.83-7.86 (m, 2H), 7.54-7.58 (m, 2H), 3.13 (ddd, *J*= 4.8, 12.0, 14.0 Hz, 1H), 3.00 (ddd, *J*= 4.8, 11.2, 14.0 Hz, 1H), 2.43 (m, 1H), 2.17-2.31 (m, 2H), 1.97-2.08 (m, 2H), 1.68-1.94 (m, 4H), 1.22-1.59 (m, 4M, 0.93 (d, *J*= 6.4 Hz, 3H), 0.61 (s, 3H); $^{13}C$ NMR (100 MHz, $CDCl_3$) δ 211.4, 140.4, 137.5, 129.6, 129.5, 61.7, 55.8, 53.6, 49.7, 40.8, 38.8, 34.4, 28.2, 27.2, 23.8, 18.9, 18.3, 12.4; IR (thin film) 2957, 2876, 1710, 1315, 1149, 1088 $cm^{-1}$; HRMS [M+Na] calc'd 405.1267 for $C_{20}H_{27}ClO_3SNa$ found: 405.1263.

**(d) (+)-Keto-(4-methylphenyl) Sulfone:** By replacing (triethylsilyl)-oxyphenyl sulfone with (triethylsilyl)-oxy-(4-methylphenyl) sulfone (Example 3d). The reaction mixture was directly purified by short path flash chromatography ($CH_2Cl_2$ then EtOAc:Hex = 1:2) to give 57.4 mg (89% for 2 steps) of keto-p-fluorophenyl. $R_f$ 0.26 (1:2-EtOAc:Hex); $[\alpha]_D^{26}$ +9.4 (c 1.15, $CHCl_3$); $^1H$ NMR (400 MHz, $CDCl_3$) δ 7.77-7.79 (m, 2H), 7.36-7.38 (m, 2H), 3.11 (ddd, *J* = 4.4, 12.0, 13.6 Hz, 1H), 2.98 (ddd, *J* = 4.8, 11.2, 13.6 Hz, 1H), 2.46 (s, 3H), 2.42 (dd, *J* = 8.0, 12.0 Hz, 1H), 2.16-2.31 (m, 2H), 1.96-2.08 (m, 2H), 1.66-1.94 (m, 4M, 1.44-1.58 (m, 4H), 1.38 (q, *J*= 9.2 Hz, 1H), 1.25 (m, 1H), 0.92 (d, *J* = 6.4 Hz, 3H), 0.60 (s, 3H); $^{13}C$ NMR (100 MHz, $CDCl_3$) δ 211.5, 144.6, 136.0, 129.8, 127.9, 61.6, 55.8, 53.5,49.6, 40.7, 38.7, 34.3, 28.2, 27.1, 23.8, 21.6, 18.9, 18.3, 12.4; IR (thin film) 2956, 2875, 1710, 1314, 1143, 1087 $cm^{-1}$; HRMS [M+Na] calc'd 385.1808 for $C_{21}H_{30}O_3SNa$. found: 385.1825.

**(e) (+)-Keto-(3,4-dichlorophenyl) Sulfone:** By replacing (triethylsilyl)-oxyphenyl sulfone with (triethylsilyl)-oxy-(3,4-dichlorophenyl) sulfone (Example 3e). The reaction mixture was directly purified by short path flash chromatography ($CH_2Cl_2$ then EtOAc:Hex = 1:2) to give 47.3 mg (94% for 2 steps) of keto-3,4-dichlororophenyl sulfone. $R_f$0.39 (1:2-EtOAc-Hex); $[\alpha]_D^{26}$ +10.9 (c 0.99, $CHCl_3$); $^1H$ NMR (400 MHz, $CDCl_3$ ) δ 8.00 (dd, *J* = 0.4, 2.0 Hz, 1H), 7.73 (dd, *J*= 2.0, 8.4 Hz, 1H), 7.67 (dd, *J*= 0.4, 8.4 Hz, 1H), 3.14 (ddd, *J*= 4.8, 11.6, 14.0 Hz, 1H), 3.01 (ddd, *J*= 4.8, 11.2, 14.0 Hz, 1H), 2.44 (dd, *J*= 7.6, 12.0 Hz, 1H), 2.17-2.32 (m, 2H), 1.98-2.09 (m, 2H), 1.69-1.95 (m, 41-1), 1.46-1.60 (m, 4H), 1.40 (q, *J* = 9.2 Hz, 1H), 1.29 (m, 1H), 0.94 (d, *J*= 6.4 Hz, 3H), 0.62 (s, 3H); $^{13}C$ NMR

(100 MHz, $CDCl_3$) δ 211.4, 138.85, 138.81, 134.0, 131.4, 130.0, 127.0, 61.7, 55.8, 53.6, 49.7, 40.8, 38.8, 34.5, 28.0, 27.3, 23.8, 18.9, 18.3, 12.4; IR (thin film) 3086, 2957, 2876, 1710, 1455, 1370, 1317, 1150, 1094, 1034, 824, 753, 676, 634 $cm^{-1}$; HRMS [M+Na] calc'd 439.0872 for $C_{20}H_{26}Cl_2O_3SNa$. found: 439.0832.

**(f) (+)-Keto-p-[3-(*tert*-Butyldimethylsiloxy)isopentyl]phenyl Sulfone:** By replacing (triethylsilyl)-oxyphenyl sulfone with p-[3-(*tert*-butyldimethylsiloxy)isopentyl]phenyl sulfone (Example 3f). The reaction mixture was directly purified by short path flash chromatography ($CH_2Cl_2$ then EtOAc:Hex = 1:2) to give 75.8 mg (97% for 2 steps) of keto-p-(3-(*tert* Butyldimethylsiloxy)isopentyl)phenyl sulfone. $R_f$ 0.53 (1:2-EtOAc:Hex); $[\alpha]_D^{26}$ +3.6 (c 1.64, $CHCl_3$); $^1H$ NMR (400 MHz, $CDCl_3$) δ 7.83-7.85 (m, 2H), 7.55-7.58 (m, 2H), 3.12 (ddd, *J*= 4.0, 12.0, 14.0 Hz, 1H), 3.00 (ddd, *J*= 4.8,10.8,13.6 Hz, 1H), 2.41 (dd, *J*= 7.6, 11.2, 1H), 2.16-2.30 (m, 2H), 1.79-2.07 (m, 8H), 1.63-1.74 (m, 2H), 1.45-1.57 (m, 3H), 1.34-1.42 (m, 1H), 1.14-1.28 (m, 2H), 1.00 (s, 9H), 0.92 (d, *J*= 6.8 Hz, 3H), 0.62 (t, *J*= 7.2 Hz, 6H), 0.58 (s, 3H), 0.17 (s, 6H); $^{13}C$ NMR (100 MHz, $CDCl_3$) δ 211.4, 152.8, 136.3, 127.5, 126.8, 81.4, 61.6, 55.7, 53.4, 49.6, 40.8, 38.7, 35.59, 35.56, 27.1, 26.2, 23.8, 18.9, 18.8, 18.3, 12.4, 8.2, -2.2; IR (thin film) 2956, 1713, 1458, 1315, 1256, 1147, 1062, 836, 798, 771 $cm^{-1}$; HRMS [M+Na] calc'd 571.3248 for $C_{31}H_{52}O_4SSiNa$. found: 571.3284

**(g) (+)-Keto-p-Acetalphenyl Sulfone:** By replacing (triethylsilyl)-oxyphenyl sulfone with (+)-(triethylsilyl)-oxy-p-Acetalphenyl sulfone (Example 3g). The reaction mixture was directly purified by short path flash chromatography ($CH_2Cl_2$ then EtOAc:Hex = 1:1) to give 66 mg (100% for 2 steps) of keto-p-acetalphenyl sulfone. $R_f$ 0.38 (1:1-EtOAc: Hex); $[\alpha]_D^{26}$ +11.1 (c 0.97, $CHCl_3$); $^1H$ NMR (400 MHz, $CDCl_3$) δ 7.91-7.93 (m, 2H), 7.68-7.70 (m, 2H), 5.88 (s, 1H), 4.06-4.15 (m, 4H), 3.12 (ddd, *J*= 4.4, 11.6, 14.0 Hz, 1H), 2.99 (ddd, *J*= 4.8, 11.2, 14.0 Hz, 1H), 2.42 (dd, *J*= 7.6,12.0 Hz, 1H), 2.17-2.31 (m, 2H), 1.97-2.06 (m, 2H), 1.66-1.94 (m, 4H), 1.44-1.58 (m, 3H), 1.37 (q, *J* = 9.6 Hz, 1H), 0.92 (d, *J* = 6.4 Hz, 3H), 0.60 (s, 3H); $^{13}C$ NMR (100 MHz, $CDCl_3$) δ 211.4, 143.9, 139.6, 128.0, 127.3, 102.3, 65.4, 61.6, 55.8, 53.5, 49.6, 40.7, 38.7, 34.4, 28.1, 27.1, 23.8, 18.9,18.2, 12.4; IR (thin film) 2957, 2879, 1709, 1381, 1145, 1086, 943, 754, 549 $cm^{-1}$; HRMS [M+Na] calc'd 443.1863 for $C_{23}H_{32}O_5SNa$. found: 443.1843.

In a like manner, the following additional compounds can be prepared:

**(h) Keto-(4-methoxyphenyl) sulfone:** By replacing (triethylsilyl)-oxyphenyl sulfone with (triethylsilyl)-oxy-(4-methoxyphenyl) sulfone (Example 3h);

**(i) Keto-(4-nitrophenyl) sulfone:** By replacing (triethylsilyl)-oxyphenyl sulfone with (triethylsilyl)-oxy-(4-nitrophenyl) sulfone (Example 3i); and

**(j) Keto-(4-trifluoromethylphenyl) sulfone:** By replacing (triethylsilyl)-oxyphenyl sulfone with (triethylsilyl)-oxy-(4-trifluoromethylphenyl) sulfone (Example 3j).

**Example 5: 24-Phenyl Sulfone Vitamin-$D_3$ Analogs (I)**

(a)

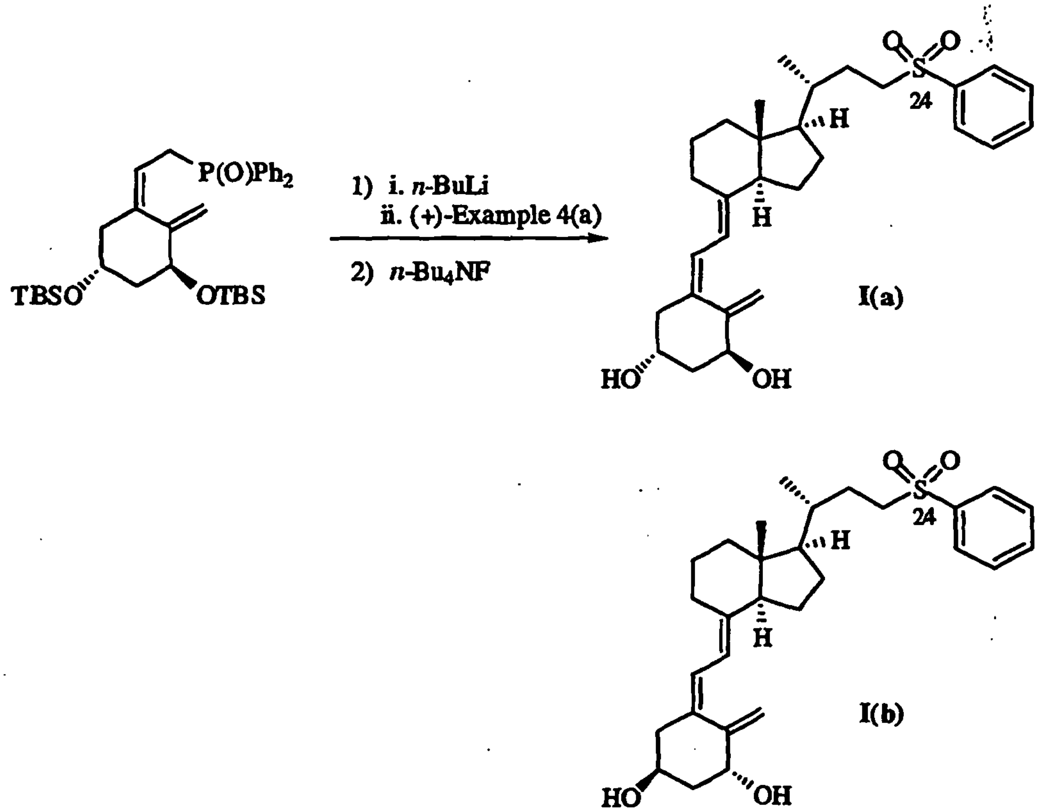


I(a)

I(b)

Prior to reaction, the phosphine oxide (Posner, G. H. et al. J. Med. Chem. 1992, 35, 3280-3287) and C,D-ring ketone of Example 3a were azeotrophically dried with benzene and left under vacuum for 48 h. A solution of *n*-BuLi in hexanes (58 μL, 0.086 mmol, 1.48 M in hexanes) was added dropwise to a cold (-78 °C) solution of phosphine oxide (50 mg, 0.086 mmol) in THF (1.30 mL) under dry argon. The resulting deep red solution was stirred for 1 h, at which time a cold (-78 °C) solution of C,D-ring ketone (Example 3a, 15 mg, 0.043 mmol) in THF (1.2 mL) was added dropwise *via* cannula. The resulting solution was stirred at -78 °C in the dark for approximately 3 h, then slowly warmed to -40 °C over 2 h. The reaction mixture was quenched with $H_2O$ (1 mL), warmed to rt, extracted with $Et_2O$ (3 x 10 mL), washed with brine, dried over $MgSO_4$, filtered, concentrated, and purified by silica gel column chromatography (20→50% EtOAc/hexanes) to afford the coupled products as a clear oil. This oil was immediately dissolved in THF (5.0 mL) and treated with TBAF (215 μL, 0.215 mmol, 1.0 M in THF) in the dark for 16 h. Concentration of the reaction mixture and column chromatography (EtOAc) yielded a mixture of diastereomers. This diastereomeric mixture was separated by HPLC (CHIRALCEL® OJ semipreparative column, 15% EtOH/hexanes, 3 mL/min) giving enantiomerically pure, hybrid vitamin-$D_3$ analogs **I(a)** (9 mg, 43%, 1α,3β, $R_f$ 37.2 min) and **I(b)** (4 mg, 19%, 1β,3α, $R_f$ 31.7 min). **I(a)** (1α,3β): $[\alpha]^{25}_{D}$ +31.8 (c 8.3, $CHCl_3$); $^1H$ NMR (400 MHz, $CDCl_3$) δ 7.95-7.88 (m, 2H), 7.70-7.62 (tt, *J*= 7.6, 1.7 Hz, 1H), 7.62-7.53 (m, 2H), 6.35 (d, *J* = 11.2 Hz, 1H), 5.99 (d, *J* = 11.2 Hz, 1H), 5.32 (m, 1H), 4.98 (m, 1H), 4.47-4.38 (m, 1H), 4.27-4.17 (m, 1H), 3.18-3.06 (m, 1H), 3.06-2.92 (m, 1H), 2.86-2.75 (dd, *J* = 12.6,4.2 Hz, 1H), 2.64-2.53 (dd, *J* = 13.6,3.2 Hz, 1H), 2.36-2.25 (dd, ***J*** = 13.4, 6.6 Hz, 1H), 0.88 (d, *J* = 6.0 Hz, 3H), 0.49 (s, 3H); $^{13}C$ NMR (100 MHz, $CDCl_3$) δ 147.6, 142.5, 139.2, 133.6, 133.2, 129.2, 128.0, 124.8, 117.2, 111.8, 70.8, 66.8, 56.1, 55.7, 53.6, 45.8, 45.2, 42.8, 40.3, 35.0, 28.9, 28.2, 27.3, 23.4, 22.1, 18.5, 12.0; IR (neat) 3647 3119, 3020, 2943, 2871, 1446, 1304, 1216, 1143, 1086, 1055, 753, 688, 534 $cm^{-1}$, HRMS: calcd for $C_{29}H_{40}O_4$ + Na, 507.2545, found 507.2507; UV pending. **I(b)** (1β,3α): $[\alpha]^{25}_{D}$ +11.4 (c 2.7, $CHCl_3$); $^1H$ NMR (400 MHz, $CDCl_3$) δ 7.95-7.87 (m, 2H), 7.70-7.62 (tt, *J*= 7.6, 1.7 Hz, 1H), 7.61-7.53 (m, 2H), 6.37 (d, *J* = 11.2 Hz, 1H), 5.99 (d, *J* =11.2 Hz, 1H), 5.31 (m, 1H), 4.98 (m, 1H), 4.47-4.38 (m, 1H), 4.27-4.16 (m, 1H), 3.18-3.06 (m, 1H), 3.06-2.92 (m, 1H), 2.86-2.75 (dd, *J* = 12.6,4.2 Hz, 1H), 2.65-2.54 (dd, *J* = 13.6,3.2 Hz, 1H), 2.35-2.24 (dd, *J* = 13.4, 6.6 Hz, 1H), 0.88 (d, *J* = 6.4 Hz, 3H), 0.50 (s, 3H); $^{13}C$ NMR (100 MHz, $CDCl_3$) δ 147.2, 142.6, 139.2, 133.6, 133.0, 129.2, 128.0, 124.8, 117.2, 112.5, 71.3, 66.8, 56.1, 55.7, 53.6, 45.8, 45.4, 42.8, 40.3, 35.0, 28.9, 28.2, 27.3, 23.4, 22.1,

18.5, 12.0; IR (neat) 3636-3125, 3066, 3019, 2936, 2866, 1447, 1379, 1306, 1215, 1144, 1085, 1053, 956, 917, 800, 753, 689, 667, 601, 534 $cm^{-1}$ ; HRMS: calcd for $C_{29}H_{40}O_4$ + Na, 507.2545, found 507.2533.

In a like manner, the following additional compounds were prepared:

(b)

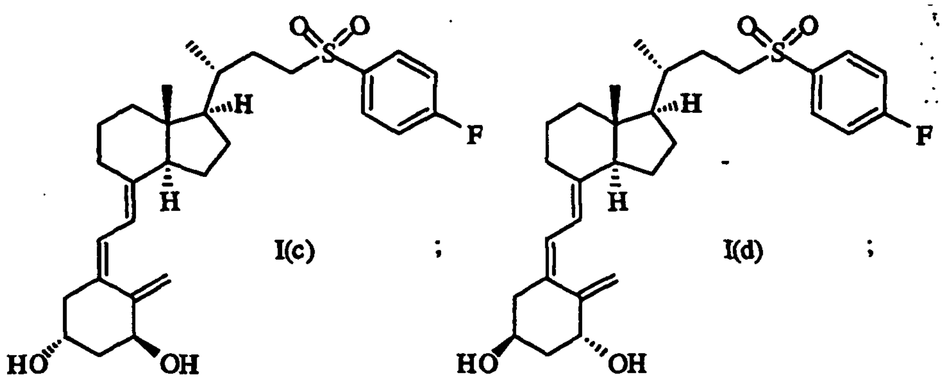

I(c) ; I(d) ;

by replacing the compound from Example 4a with the compound of Example 4b. The diastereomers were purified by HPLC (Chiralcel OJ column, 25% EtOH in Hexanes, 2.5 mL/min, 254 nm) to afford 14 mg (55%) of (+)-**I(c)** (1α,3β, $t_R$ 34.2 min) as a viscous oil and 5.3 mg (21%) of (+)-**I(d)** (1β,3α, $t_R$ 27.0 min) as a viscous oil. (+)-**I(c)**: $R_f$0.61 (EtOAc); $[\alpha]_D^{26}$ +32.3 (c 1.68, $CHCl_3$); $^1$HNMR (400 MHz, $CDCl_3$) δ 7.91-7.94 (m, 2H), 7.23-7.27 (m, 2H), 6.36 (d, *J* = 11.2 Hz, 1H), 6.00 (d, *J*= 11.2 Hz, 1H), 5.32 (m, 1H), 4.98 (m, 1H), 4.43 (m, 1H), 4.23 (m, 1H), 3.13 (ddd, J= 4.8,11.6, 14.0 Hz, 1H), 2.99 (ddd, *J* = 4.8, 11.2, 14.0 Hz, 1H), 2.81 (dd, *J* = 4.0 12.4 Hz, 1H), 2.59 (dd, *J* = 2.8, 13.2 Hz, 1H), 2.31 (dd, *J* = 6.4, 13.2 Hz, 1H), 1.89-2.04 (m, 4H), 1.74-1.87 (m, 2H), 1.62-1.73 (m, 4H), 1.44-1.57 (m, 5H), 1.15-1.30 (m, 3H), 0.89 (d, *J*= 6.4 Hz, 3H), 0.51 (s, 3H); $^{13}$C NMR (100 MHz, $CDCl_3$) δ 165.8 (d, 255.1 Hz), 147.6, 142.4, 135.2 (d, 3.0 Hz), 133.2, 130.9 (d, 9.1 Hz), 124.8, 117.3, 116.6 (d, 22.0 Hz), 111.8, 70.8, 66.8, 56.2, 55.7, 53.8, 45.8, 45.2, 42.8, 40.3, 35.0, 28.9, 28.3, 27.3, 23.4, 22.1, 18.5,12.0; IR (thin film) 3380, 2946, 2874, 1591, 1494, 1315, 1288, 1231, 1143, 1086, 1054, 840, 754, 668 $cm^{-1}$; HRMS [M+Na] calc'd 525.2445 for $C_{29}H_{39}FO_4SNa$. found: 525.2462; UV (MeOH) $\lambda_{max}$ 264 nm (ε 14000). (+)-**I(d)**: $R_f$ 0.61 (EtOAc); $[\alpha]_D^{26}$ +21.5 (*c* 0.57, $CHCl_3$); $^1$H NMR (400 MHz, $CDCl_3$) δ 7.90-7.95 (m, 2H), 7.25-7.28 (m, 2H), 6.37 (d, *J*= 11.6 Hz, 1H), 6.00 (d, *J*= 11.2 Hz, 1H), 5.31 (dd, *J*= 1.2,2.0 Hz, 1H), 4.99 (d, *J*= 1.2 Hz, 1H), 4.44 (m, 1H), 4.22 (m, 1H), 3.13 (ddd, *J* = 4.4, 12.0, 14.0 Hz, 1H), 2.99 (ddd, *J* = 4.8, 11.6, 14.0 Hz, 1H), 2.82 (dd, *J* = 4.4 12.8 Hz, 1H), 2.61 (dd, *J*= 4.0, 13.6 Hz, 1H), 2.29 (dd, *J* = 7.6, 13.2 Hz, 1H), 1.90-2.03 (m, 4H), 1.74-1.87 (m, 2H), 1.44-1.73 (m, 9H), 1.15-1.30 (m, 3H), 0.89 (d, *J* = 6.4 Hz, 3H), 0.51 (s, 3H); $^{13}$C NMR (100 MHz, $CDCl_3$) δ 165.8 (d, 255.9 Hz), 147.3, 142.5, 135.3 (d, 3.0 Hz), 133.1, 130.9 (d, 9.9 Hz), 124.8, 117.3, 116.6 (d, 22.8 Hz), 112.5, 71.3, 66.8, 56.1, 55.7, 53.8, 45.8, 45.4, 42.8, 40.3, 35.0, 28.9, 28.3, 27.3; 23.4, 22.2, 18.5, 12.0; IR (thin film) 3382, 2929, 2873, 1591, 1494, 1315, 1288, 1232, 1143, 1086, 1053, 840, 754, 569 $cm^{-1}$; HRMS [M+Na] calc'd 525.2445 for $C_{29}H_{39}FO_4SNa$. found: 525.2474; UV (MeOH) $1_{max}$ 258 nm (e 12000).

(c)

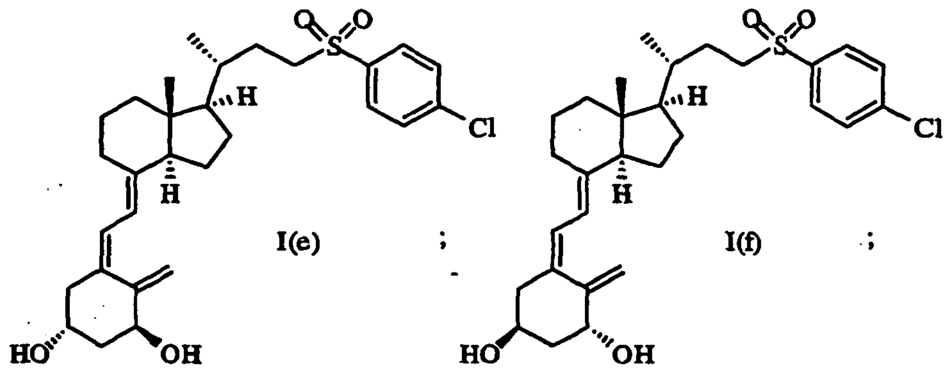

I(e) ; I(f) ;

by replacing the compound of Example 4a with the compound of Example 4c; The diastereomers were purified by HPLC (Chiralcel OJ column, 20% EtOH in Hexanes, 2.5 mL/min, 254 nm) to afford 12.3 mg (44%) of (+)-**I(e)** (1α,3β, $t_R$ 29.6 min) as a viscous oil and 3.9 mg (14%) of (+)-**I(f)** (1β,3α, $t_R$ 25.8 min) as a viscous oil. (+)-**I(e)**: $R_f$ 0.58 (EtOAc); $[\alpha]_D^{26}$ +33.5 (*c* 0.88, $CHCl_3$); $^1H$ NMR (400 MHz, $CDCl_3$) δ 7.83-7.86 (m, 2H), 7.54-7.57 (m, 2H), 6.36 (d, *J* = 11.2, 1H), 6.00 (d, *J* = 11.2 Hz, 1H), 5.33 (s, 1H), 4.99 (s, 1H), 4.43 (m, 1H), 4.23 (m, 1H), 3.13 (ddd, *J* = 4.4, 12.0, 14.0 Hz, 1H), 2.99 (ddd, *J*= 4.8, 11.6, 14.0 Hz, 1H), 2.82 (dd, *J*= 4.0 12.4, 1H), 2.59 (dd, *J*= 3.6, 13.6 Hz, 1H), 2.31 (dd, *J*= 6.8, 13.6 Hz, 1H), 1.44-2.05 (m, 15H), 1.16-1.30 (m, 3H), 0.89 (d, *J*= 6.4 Hz, 3H), 0.51 (s, 3H); $^{13}C$ NMR (100 MHz, $CDCl_3$) δ 147.6, 142.5, 140.4, 137.7, 133.2, 129.6, 129.5, 124.8, 117.3, 111.8, 70.8, 66.8, 56.2, 55.7, 53.7, 45.8, 45.2, 42.8, 40.3, 35.0, 28.9, 28.3, 27.4, 23.4, 22.2, 18.5, 12.0; IR (thin film) 3382, 2926, 1583, 1313, 1148, 1088, 756 $cm^{-1}$; HRMS [M+Na] calc'd 541.2150 for $C_{29}H_{39}ClO_4SNa$. found: 541.2139; UV (MeOH) $\lambda_{max}$ 264 nm (ε 14000). (+)-**I(f):** $R_f$ 0.58 (EtOAc); $[\alpha]_D^{26}$ +18.4 (*c* 0.42, $CHCl_3$); $^1H$ NMR (400 MHz, $CDCl_3$) δ 7.83-7.86 (m, 2H), 7.54-7.57 (m, 2H), 6.37 (d, *J*=11.2, 1H), 6.00 (d, *J*= 11.2 Hz, 1H), 5.32 (m, 1H), 4.99 (m, 1H), 4.44 (m, 1H), 4.22 (m, 1H), 3.13 (ddd, *J*= 4.4,11.6,14.0 Hz, 1H), 2.99 (ddd, *J*= 4.8, 11.2, 14.0 Hz, 1H), 2.82 (dd, *J* = 4.0, 12.4, 1H), 2.61 (dd, *J*= 4.0, 12.8 Hz, 1H), 2.30 (dd, *J*=,7.2,13.2 Hz, 1H), 1.90-2.04 (m, 3H), 1.72-1.88 (m, 3H), 1.44-1.67 (m, 9H), 1.20-1.30 (m, 3H), 0.89 (d, *J* = 6.4 Hz, 3H), 0.51 (s, 3H); $^{13}C$ NMR (100 MHz, $CDCl_3$) δ 147.3, 142.5, 140.4, 137.7, 133.1, 129.6, 129.5, 124.8, 117.3, 112.5, 71.3, 66.8, 56.1, 55.7, 53.7, 45.8, 45.4, 42.8, 40.3, 35.0, 28.9, 28.3, 27.3, 23.4, 22.2, 18.5, 12.0; IR (thin film) 3366, 2926, 1583, 1475, 1314, 1148, 1089, 758, 668, 630 $cm^{-1}$; HRMS [M+Na] calc'd 541.2150 for $C_{29}H_{39}ClO_4SNa$. found: 541.2112; UV (MeOH) $\lambda_{max}$ 253 nm (ε 8700).

(d)

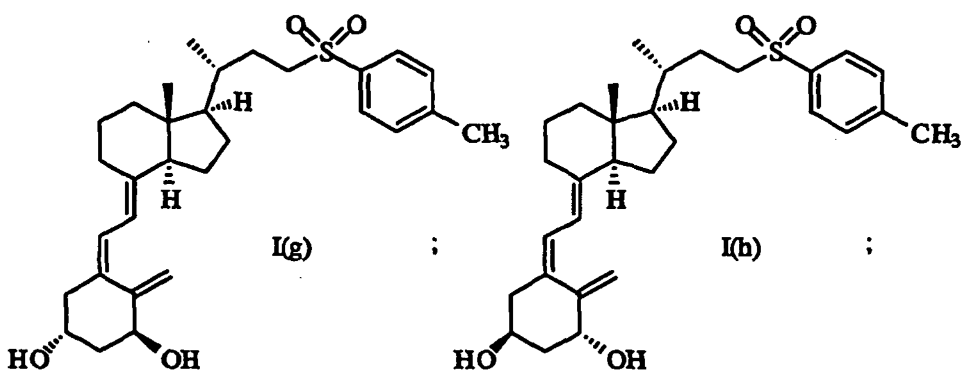

I(g) ; I(h) ;

by replacing the compound of Example 4a with the compound of Example 4d.
The diastereomers were purified by HPLC (Chiralcel OJ column, 17% EtOH in Hexanes, 2.5 mL/min, 254 nm) to afford 6.2 mg (53%) of (+)-**I(g)** (1α,3β, $t_R$ 37.7 min) as a viscous oil and 2.0 mg (17%) of (+)-**I(h)** (1β,3α, $t_R$ 31.4 min) as a viscous oil. (+)-**I (g):** $R_f$0.61 (EtOAc); [α]$D^{26}$ +38.6 (*c* 0.70, $CHCl_3$); $^1H$ NMR (400 MHz, $CDCl_3$) δ 7.77-7.80 (m, 2H), 7.35-7.37 (m, 2H), 6.36 (d, *J*=11.2 Hz, 1H), 6.00 (d, *J* = 11.6 Hz, 1H), 5.32 (dd, *J*= 1.6,1.6 Hz, 1H), 4.99 (dd, *J*=1.2, 1.2

Hz, 1H), 4.43 (m, 1H), 4.23 (m, 1H), 3.11 (ddd, *J* = 4.8, 12.0, 14.0 Hz, 1H), 2.97 (ddd, *J* = 4.8, 11.2, 13.6 Hz, 1H), 2.81 (dd, *J*= 4.4, 12.4 Hz, 1H), 2.59 (dd, *J*= 4.0, 13.2 Hz, 1H), 2.46 (s, 3H), 2.31 (dd, *J*= 6.4, 13.2 Hz, 1H), 1.89-2.03 (m, 4H), 1.74-1.86 (m, 2H), 1.43-1.72 (m, 8H), 1.17-1.30 (m, 4H), 0.88 (d, *J*= 6.0 Hz, 3H), 0.50 (s, 3H); $^{13}C$ NMR (100 MHz, $CDCl_3$) δ 147.6, 144.5, 142.6, 136.3, 133.2, 129.8, 128.0, 124.8, 117.2, 111.8, 70.8, 66.8, 56.2, 55.7, 53.7, 45.8, 45.2, 42.8, 40.3, 35.0, 28.9, 28.3, 27.3, 23.4, 22.1, 21.6, 18.5, 12.0; IR (thin film) 3392, 2926, 2873, 1597, 1448, 1313, 1302, 1285, 1142, 1087, 1054, 754, 668 $cm^{-1}$; HRMS [M+Na] calc'd 521.2696 for $C_{30}H_{42}O_4SNa$. found: 521.2662; UV (MeOH) $\lambda_{max}$ 262 nm (ε 18000). (+)**-I(h):** $R_f$ 0.61 (EtOAc); $[\alpha]_D^{26}$ +22.8 (*c* 0.20, $CHCl_3$); $^1H$ NMR (400 MHz, $CDCl_3$) δ 7.77-7.79 (m, 2H), 7.35-7.37 (m, 2H), 6.37 (d, *J*= 11.6 Hz, 1H), 5.99 (d, *J*=10.8 Hz, 1H), 5.31 (dd, *J*= 1.2, 1.2 Hz, 1H), 4.99 (m, 1H), 4.44 (m, 1H), 4.22 (m, 1H), 3.11 (ddd, *J* = 4.0, 12.0, 14.0 Hz, 1H), 2.97 (ddd, *J* = 4.8, 11.2, 14.0 Hz, 1H), 2.81 (dd, *J* = 4.8, 12.8 Hz, 1H), 2.61 (dd, *J*= 4.0, 13.6 Hz, 1H), 2.46 (s, 3H), 2.29 (dd, *J*= 7.6,13.2 Hz, 1H), 1.90-2.04 (m, 3H), 1.74-1.87 (m, 2H), 1.43-1.70 (m, 9H), 1.17-1.29 (m, 4H), 0.88 (d, *J*= 6.4 Hz, 3H), 0.50 (s, 3H); $^{13}C$ NMR (100 MHz, $CDCl_3$) δ 147.3, 144.5, 142.6, 136.3, 133.0, 129.8, 128.0, 124.8, 117.2, 112.5, 71.3, 66.8, 56.2, 55.7, 53.7, 45.8, 45.4, 42.8, 40.3, 35.0, 28.9, 28.4, 27.3, 23.4, 22.2, 21.6, 18.5, 12.0; IR (thin film) 3400, 2926, 2872, 1597, 1449, 1313, 1302, 1286, 1142, 1087, 1053, 754, 668 $cm^{-1}$; HRMS [M+Na] calc'd 521.2696 for $C_{30}H_{42}O_4SNa$. found: 521.2707; UV (MeOH) $\lambda_{max}$ 264 nm (ε 8700).

(e)

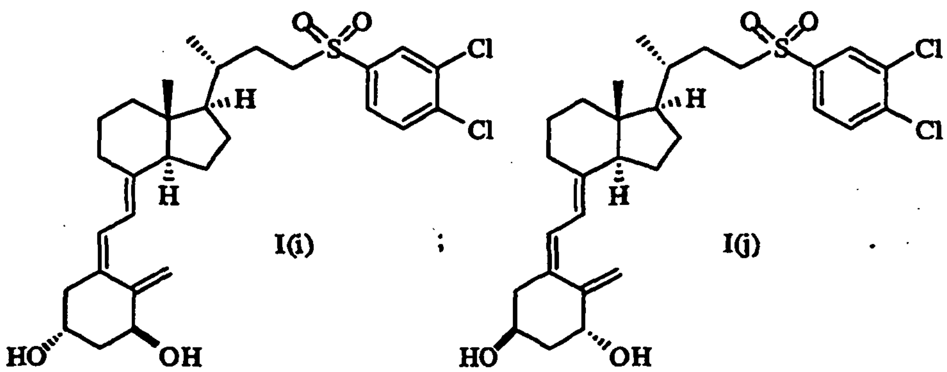

I(i) ; I(j) .

by replacing the compound of Example 4a with the compound of example 4e;

The diastereomers were purified by HPLC (Chiralcel OJ column, 22% EtOH in Hexanes, 2.5 mL/min, 254 nm) to afford 17.7 mg (52%) of (+)-I(i) (1α,3β $t_R$ 36.4 min) as a viscous oil and 5.3 mg (16%) of (+)-**I(j)** (1β,3α, $t_R$ 29.0 min) as a viscous oil. (+)**-I (i):** $R_f$ 0.73 (EtOAc); $[\alpha]_D^{26}$ +28.3 (*c* 2.10, $CHCl_3$); $^1H$ NMR (400 MHz, $CDCl_3$) δ 8.00 (d, *J*= 2.0 Hz, 1H), 7.73 (dd, *J*= 2.4, 8.4 Hz, 2H), 7.66 (d, *J*= 8.4 Hz, 1H), 6.36 (d, *J*= 11.6 Hz, 1H), 6.01 (d, *J*= 11.2 Hz, 1H), 5.33 (dd, *J*= 1.2, 1.6 Hz, 1H), 4.99 (m, 1H), 4.43 (dd, *J*= 4.4, 7.6 Hz, 1H), 4.23 (m, 1H), 3.14 (ddd, *J*= 4.4, 12.0, 14.0 Hz, 1H), 3.00 (ddd, *J*= 4.8,11.2, 14.0 Hz, 1H), 2.82 (dd, *J*= 4.4, 12.0 Hz, 1H), 2.59 (dd, *J* = 3.6, 13.6 Hz, 1H), 2.31 (dd, *J*= 6.4, 13.6 Hz, 1H), 1.92-2.04 (m, 4H), 1.76-1.90 (m, 2H), 1.64-1.71 (m, 4H), 1.45-1.58 (m, 5H), 1.20-1.31 (m, 3H), 0.90 (d, *J*= 6.0 Hz, 3H), 0.52 (s, 3H); $^{13}C$ NMR (100 MHz, $CDCl_3$) δ 147.6, 142.4, 139.0, 138.8, 134.1, 133.3, 131.4, 130.0, 127.l, 124.8, 117.3, 111.8, 70.8,66.8,56.1, 55.7, 53.7, 45.8, 45.2, 42.8, 40.3, 35.0, 28.9, 28.2, 27.4, 23.4, 22.1, 18.4, 12.0; IR (thin film) 3375, 2945, 1454, 1370, 1316, 1149, 1093,1053, 1034, 824, 754, 676, 633 $cm^{-1}$; HRMS [M+Na] calc'd 575.1760 for $C_{29}H_{38}Cl_2O_4SNa$. found: 575.1764; UV (MeOH) $\lambda_{max}$ 262 nm (e 12000). (+)**-I (j):** $R_f$ 0.73 (EtOAc); [α]D[26] +22.6 (*c* 0.54, $CHCl_3$); $^1H$ NMR (400 MHz, $CDCl_3$) δ 8.00 (d, *J* = 2.0 Hz, 1H), 7.73 (dd, *J* = 2.0, 8.4Hz, 2H), 7.66 (d, *J*= 8.4 Hz, 1H), 6.37 (d, *J*= 11.2 Hz, 1H), 6.00 (d, *J*= 11.2 Hz, 1H), 5.32 (m, 1H), 4.99 (m, 1H), 4.44 (m, 1H), 4.22 (m, 1H), 3.14 (ddd, *J*= 4.4, 12.0, 14.0 Hz, 1H), 3.00 (ddd, *J*= 4.4, 11.6, 14.0 Hz, 1H), 2.82 (dd, *J*= 4.4, 12.4 Hz, 1H), 2.61 (dd, *J*= 4.0, 13.2 Hz, 1H), 2.30 (dd, *J*= 7.6, 13.2 Hz, 1H), 1.90-2.04 (m, 4H), 1.76-1.89 (m, 2H), 1.46-1.72 (m, 9H), 1.20-1.31 (m, 3H), 0.91 (d, *J*= 6.0 Hz, 3H), 0.52 (s, 3H); $^{13}C$ NMR (100 MHz, $CDCl_3$) δ 147.2, 142.4, 139.0, 138.8, 134.1, 133.1, 131.4, 130.1, 127.1, 124.8, 117.3, 112.6, 71.3, 66.8, 56.1, 55.7, 53.8, 45.8, 45.4, 42.8, 40.3, 35.0, 28.9, 28.2, 27.4, 23.4, 22.2, 18.5, 12.0; IR (thin film) 3371, 2945, 2872, 1454, 1370, 1316, 1149, 1093, 1053, 1034, 824, 754, 676 $cm^{-1}$; HRMS [M+Na] calc'd 575.1760 for $C_{29}H_{38}Cl_2O_4SNa$. found: 575.1764; UV (MeOH) $\lambda_{max}$ 264 nm (ε 11000).

(f)

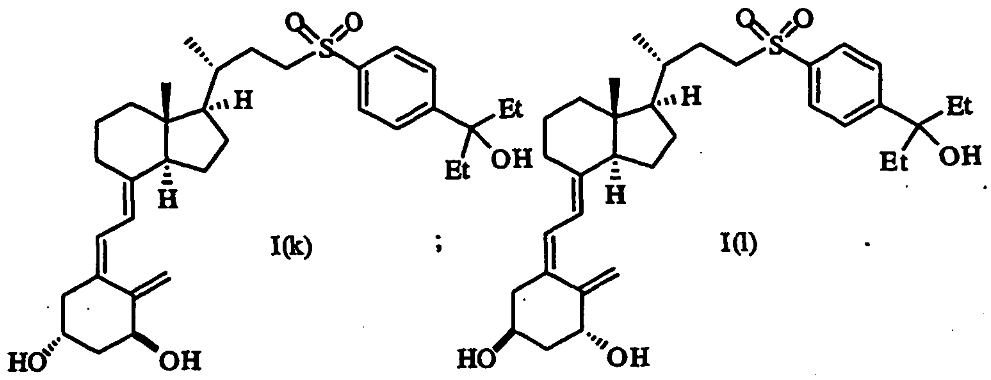

I(k) ; I(l) .

by replacing the compound of Example 4a with the compound of example 4f.

The diastereomers were then purified by HPLC (Chiralcel OJ column, 15% EtOH in Hexanes, 2.5 mL/min, 254 nm) to afford 8.2 mg (47%) of (+)-**I(k)** (1α,3β, $t_R$ 36.3 min) as a viscous oil and 5.1 mg (29%) of **I(l)** (1β,3α, $t_R$ 30.5 min) as a viscous oil. (+)-**I(k)**: $R_f$ 0.56 (EtOAc); $[\alpha]_D^{26}$ +25.1 (*c* 2.10, $CHCl_3$); $^1H$ NMR (400 MHz, $CDCl_3$) δ 7.85-7.88 (m, 2H), 7.58-7.60 (m, 2H), 6.36 (d, *J*=10.8 Hz, 1H), 5.99 (d, *J* =11.2 Hz, 1H), 5.32 (dd, *J*=1.2, 2.0 Hz, 1H), 4.98 (m, 1H), 4.43 (dd, *J*= 4.4, 7.6 Hz, 1H), 4.22 (tt, *J*= 3.6, 6.4 Hz, 1H), 3.13 (ddd, *J*= 4.4, 12.0, 14.0 Hz, 1H), 3.00 (ddd, *J* = 4.8,11.2,13.6 Hz, 1H), 2.81 (dd, *J*= 4.4, 12.4 Hz, 1H), 2.59 (dd, *J*= 3.2, 13.6 Hz, 1H), 2.31 (dd, *J*= 6.4, 13.6 Hz, 1H), 1.79-2.05 (m, 10H), 1.40-1.75 (m, 9H), 1.11-1.29 (m, 4H), 0.89 (d, *J* = 6.4 Hz, 3H), 0.75 (t, *J* = 7.2 Hz, 6H), 0.49 (s, 3H); $^{13}C$ NMR (100 MHz, $CDCl_3$) δ 152.2, 147.6, 142.5, 137.0, 133.2, 127.8, 126.5, 124.8, 117.2, 111.8, 77.4, 70.8, 66.8, 56.2, 55.6, 53.6, 45.8, 45.2, 42.8, 40.3, 35.29, 35.26, 35.0, 29.0, 28.3, 27.2, 23.4, 22.1, 18.5, 12.0, 7.6; IR (thin film) 3456, 2937, 1458, 1311, 1144, 1086, 1054, 967, 755 $cm^{-1}$; HRMS [M+Na] calc'd 593.3271 for $C_{34}H_{50}O_5SNa$. found: 593.3237; UV (MeOH) $\lambda_{max}$ 261 nm (ε 8600). **I(l):** $R_f$ 0.73 (EtOAc); $^1H$ NMR (400 MHz, $CDCl_3$) δ 7.86-7.88 (m, 2H), 7.58-7.61 (m, 2H), 6.37 (d, *J*= 11.2 Hz, 1H), 5.98 (d, *J*= 11.2 Hz, 1H), 5.31 (m, 1H), 4.98 (m, 1H), 4.43 (dd, *J*= 4.4, 6.4 Hz, 1H), 4.21 (tt, *J*= 3.6, 7.2 Hz, 1H), 3.13 (ddd, *J*= 4.4, 12.0, 14.0 Hz, 1H), 3.00 (ddd, *J*= 4.8, 11.2, 14.0 Hz, 1H), 2.81 (dd, *J*= 4.4, 12.8 Hz, 1H), 2.61 (dd, *J*= 4.0, 13.2 Hz, 1H), 2.29 (dd, *J*= 7.2, 13.2 Hz, 1H), 1.80-2.03 (m, 10H), 1.40-1.75 (m, 9H), 1.13-1.29 (m, 4H), 0.89 (d, *J* = 6.4 Hz, 3H), 0.75 (t, *J*= 7.2 Hz, 6H), 0.49 (s, 3H); The title compound **I(l)** was decomposed during overnight $^{13}C$ NMR

(g)

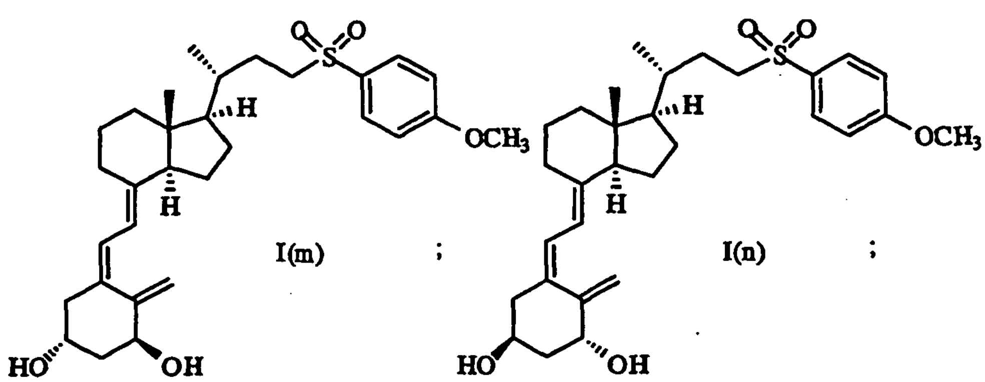

I(m) ; I(n) ;

by replacing the compound of Example 4a with the compound of example 4h;

(h)

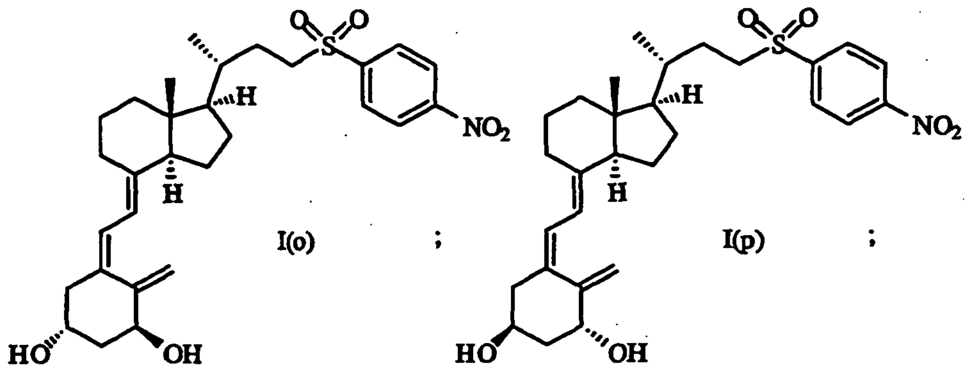

I(o) ; I(p) ;

by replacing the compound of Example 4a with the compound of example 4i; and

(i)

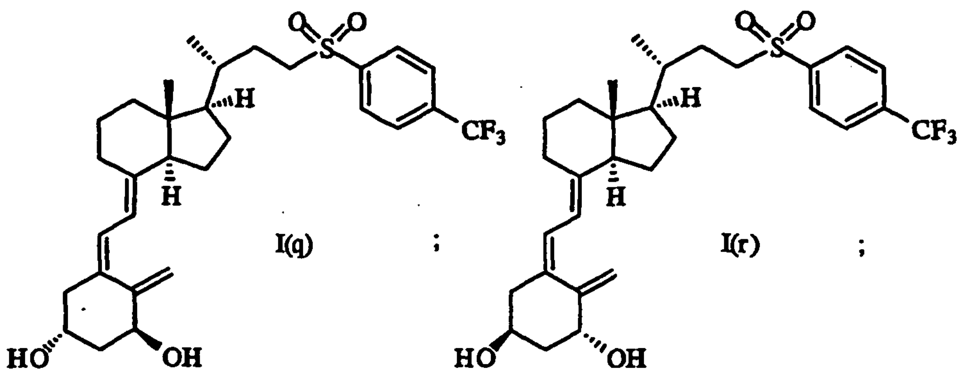

I(q) ; I(r) ;

by replacing the compound of Example 3a with the compound of example 4j.

**Example 6: Preparation of 24-SO2-PhCH(NOMe) I(s) and I(t):**

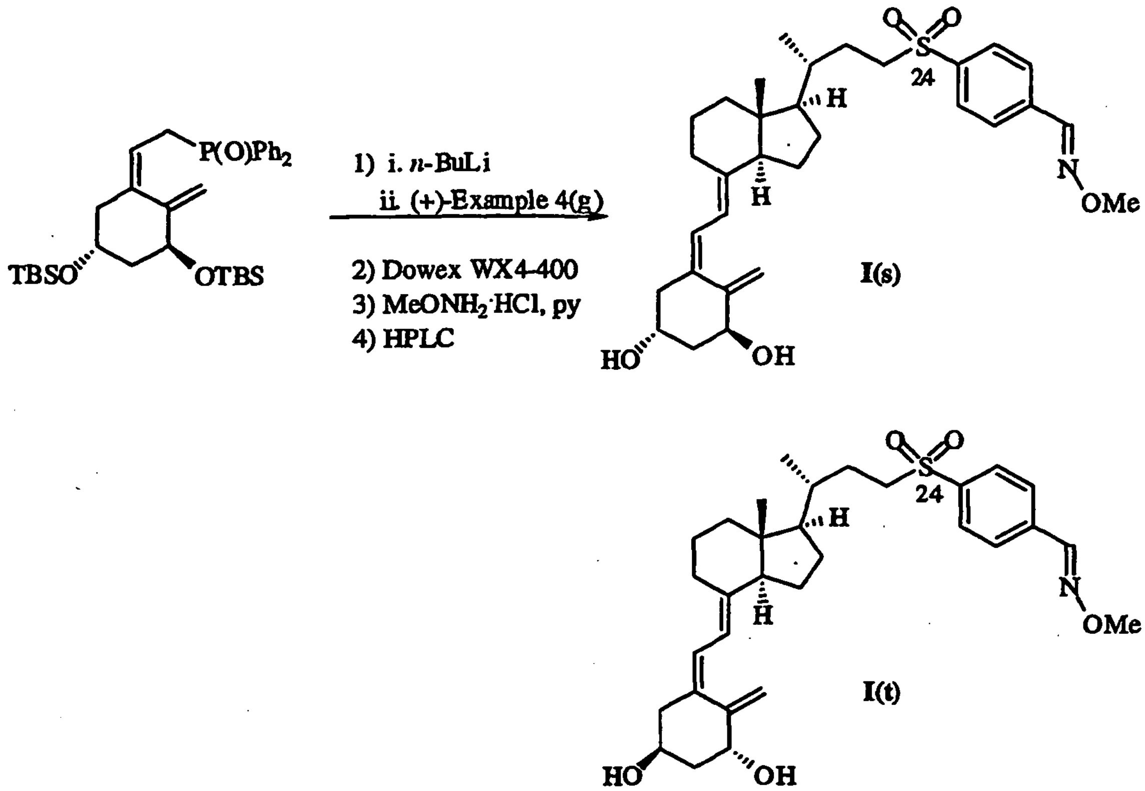


I(s)

I(t)

To a solution of (±)-A-ring phosphine oxide (Posner, G. H. et al. J. Med Chem. 1992, 35, 3280-3287) (72.8 mg, 0.125 mmol) in THF (2 mL) was added 0.047 mL of *n*-BuLi (2.67 M in Hexane, 0.125 mmol) at -78 °C, then the reddish solution was stirred for 10 min at the same temperature. A precooled (-78 °C) solution of C24-p-acetalphenyl sulfone C/D ring ketone from Example 4g (32.4 mg, 0.0770 mmol) in THF (2 mL) was added to the above solution at -78 °C via cannula. The resulting reddish orange solution was stirred for 6 hrs at -78 °C. The reaction was quenched with 2 mL of pH 7 buffer, then warmed to room temperature, extracted with EtOAc, washed with brine, dried over $MgSO_4$, filtered, concentrated in vacuo, and purified by flash chromatography (EtOAc:Hex = 1:4) to give 31.5 mg (52%) of a diastereomeric mixture of bis TBS protected p-acetalphenyl sulfone coupled products. A solution of this latter product (20 mg, 0.0255 mmol) and Dowex 50WX4-400 (794 mg) in $CH_2Cl_2$-aceton (2 mL-2 mL) was stirred for 24h. The reaction mixture was filtered and purified by flash chromatography (EtOAc:Hex =1:1 to 2:1) to afford 5.4 mg of a diastereomeric mixture of the corresponding bishydroxy benzaldehydes along with some unreacted starting material. The latter mixture was treated with $MeONH_2$•HCl (8.9 mg, 0.104 mmol), several beads of molecular sieves 4A, and pyridine (1.2 mL). The reaction mixture was diluted with EtOAc, washed with 1N aq. HCl and brine, dried over $MgSO_4$, filtered, and concentrated in vacuo to give a crude mixture which was then purified by flash chromatography (EtOAc:Hex = 3:1) to afford 3.7 mg (65%) of a diastereomeric mixture of **I(s)** and **I(t)**. The diastereomers were then purified by HPLC (Chiralcel OJ column, 30% EtOH in Hexanes, 2.5 mL/min, 254 nm) to afford **I(s)** (1α,3β, $t_R$ 32.7 min) as a viscous oil and **I(t)** (1β,3α, $t_R$ 25.1 min) as a viscous oil.

**Example 7: Preparation of Compound I(u)**

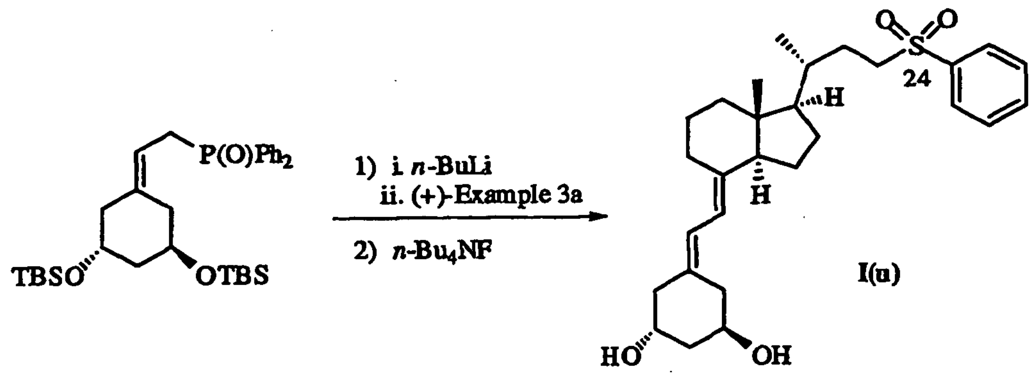


I(u)

A solution of 58 mg (0.10 mmol) of 19-nor-phosphine oxide (Hilpert, H. and Wirz, B. Tetrahedron 2001, 57, 681-694) in 2.0 mL of anhydrous THF was cooled to -78 °C and treated with 64 µL (0.10 mmol, 1.6 M in hexanes) of n-BuLi under argon atmosphere. The mixture turned deep reddish and was stirred for 15 min at -78 °C. To the solution was added dropwise a precooled (-78 °C) solution of 12 mg (0.034 mmol) of the C,D-ring ketone from Example 3a in 1.5 mL of anhydrous THF via cannula. The reaction kept going until the reddish orange color faded to yellow (about 4 hr). The reaction was quenched by adding 1.0 mL of pH 7 buffer at -78 °C, then warmed to room temperature, extracted with EtOAc (20 mL x 2), washed with brine, dried over $MgSO_4$, concentrated. The residue was subjected to column chromatography with EtOAc/hexanes (1/3) as eluent to afford 19 mg (80 %) of the coupled product as a colorless oil. The coupled product (19 mg, 0.027 mmol) was dissolved in 3 mL of anhydrous THF, and to the solution was added 0.40 mL (0.40 mmol) of a 1.0 M solution of TBAF in THF. The resulting mixture was stirred overnight at room temperature, then quenched with 2 mL of water. The solution was extracted with EtOAc (20 mL x 3), washed with brine, dried over $MgSO_4$, concentrated. The residue was subjected to column chromatography with EtOAc as eluent to give 12 mg (94 %) of the crude product of (+)-**I (u)** as a colorless oil. The crude product was purified by HPLC (Chiralcel OJ column, 20 % EtOH in Hexanes, 2.5 mL/min, 254 nm) to afford 10.5 mg of (+)-**I (u)** (1a, 3b, $t_R$ = 29.1 min). : $[\alpha]^{24}{}_D$ = +91.2 (*c* = 0.19, MeOH). $^1H$ NMR (400 MHz, $CDCl_3$) δ 7.90-793 (m, 2H), 7.67 (m, 1H), 7.56-7.60 (m, 2H), 6.29 (d, *J* = 11.2 Hz, 1H), 5.83 (d, *J*=11.2 Hz, 1H), 4.11 (m, 1H), 4.05 (m, 1H), 3.14 (ddd, *J*= 13.6, 12.0, 4.0 Hz, 1H), 3.00 (ddd, *J*= 13.6, 11.2, 4.8 Hz, 1H), 2.78 (dd, *J*= 12.4, 4.0 Hz, 1H), 2.72 (dd, *J* = 13.2, 4.0 Hz, 1H), 2.47 (dd, *J*= 13.2, 3.6 Hz, 1H), 2.17-2.43 (m, 2H), 1.74-1.99 (m, 6H), 1.44-1.68 (m, 9H), 1.17-1.30 (m, 3H), 0.89 (d, *J*= 6.0 Hz, 3H), 0.50 (s, 3H). $^{13}C$ NMR (100 MHz, $CDCl_3$,) δ 142.4, 139.2,133.6, 131.4,129.2, 128.0, 123.7, 115.5, 67.4, 67.2, 56.1, 55.7, 53.6, 45.7, 44.6, 42.1, 40.3, 37.1, 35.0, 28.8, 28.3, 27.3, 23.3, 22.1, 18.5, 12.0. IR (neat, $cm^{-1}$) 3362, 2943, 1447, 1306, 1145, 1086, 1048, 753, 689, 537. HRMS ($[M+Na]^+$) calcd. 495.2539, found 495.2526.

**Example 12: <u>Preparation of Isopropyl Phenyl Sulfone (VII, $R^4$ = Ph, $R^6$,$R^7$ =</u> Me)**

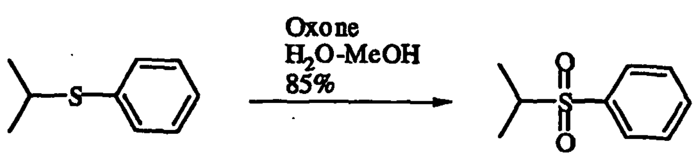


**VII $R^4$ = Ph, $R^6$, $R^7$ = Me**

To a solution of isopropyl phenyl sulphide (500 mg, 3.28 mmol) in MeOH (20 mL) was added a solution of potassium peroxymonosulfate ($2KHSO_5$•$KHSO_4$•$K_2SO_4$, Oxone®) (3.03 g, 9.85 mmol) in water (20 mL) at 0 °C. The resulting white suspension was warmed to room temperature and then stirred for 5h. The mixture was diluted with water (10 mL), extracted with EtOAc (80 mL x 2), washed with brine, dried over $MgSO_4$, concentrated in vacuo, and then purified by column chromatography (25% EtOAc/hexanes) to give 512 mg (85%) of isopropyl phenyl sulfone **VII** ($R^4$ = Ph, $R^6$,$R^7$ = Me) as a colorless oil: $^1H$ NMR (400 MHz, $CDCl_3$) δ 7.74-7.64 (m, 2H). 7.55-7.51 (m, 1H), 7.46-7.42 (m, 2H), 3.08 (septet, *J* = 6.8 Hz, 1H), 1.50 (d, *J*= 6.8 Hz, 6H); $^{13}$NMR (100 MHz, $CDCl_3$) δ 136.54, 133.31, 128.73, 128.55,55.05,15.26.

**Example 13: Preparation of Cyclopropyl Phenyl Sulfone VII ($R^4$ = Ph, $R^6$,$R^7$ = cyclopropyl)**

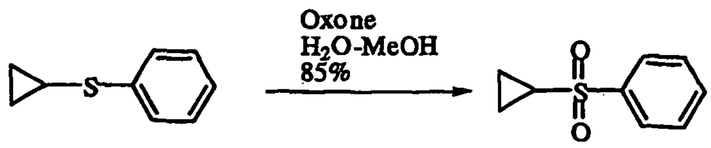


**VII, $R^4$ = Ph, $R^6$, $R^7$ = cyclopropyl**

To a solution of cyclopropyl phenyl sulphide (450 mg, 3.00 mmol) in MeOH (15 mL) was added a solution of potassium peroxymonosulfate ($2KHSO_5 \cdot KHSO_4 \cdot K_2SO_4$, Oxone®) (5.52 g, 8.99 mmol) in water (15 mL) at 0 ˚C. The resulting white suspension was warmed to room temperature and then stirred for 5h. The mixture was diluted with water (10 mL), extracted with EtOAc (60 mL x 2), washed with brine, dried over $MgSO_4$, concentrated in vacuo, and then purified by column chromatography (25% EtOAc/hexanes) to give 494 mg (91%) of cyclopropyl phenyl sulfone **VII** ($R^4$ = Ph, $R^6$,$R^7$ = cyclopropyl) as a colorless oil: $^1$H NMR (400 MHz, $CDCl_3$) δ 7.92-7.90 (m, 2H). 7.66-7.62 (m, 1H), 7.58-7.54 (m, 2H), 3.08 (m, 1H), 1.38-1.33 (m, 2H), 1.06-1,00 (m, 2H); $^{13}$NMR (100 MHz, $CDCl_3$) δ 140.67, 133.33, 129.19,127.53, 32.89, 5.94.

**Example 14: Preparation of 22-Iodo Silyl Ether VI**

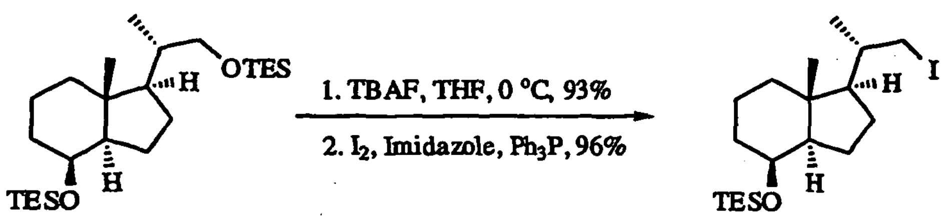


**VI**

To a solution of bis-silylated diol (508 mg, 1.15 mmol) in 10 mL of anhydrous THF was added 1.15 mL of TBAF (1M in THF) dropwise at 0 ˚C. After being stirred for 1 h at 0 ˚C, the reaction mixture was extracted with EtOAc (30 mL x 2), washed with brine, dried over $MgSO_4$, concentrated in vacuo, and then purified by column chromatography (20% EtOAc/hexanes) to give 351 mg (93%) of mono-silylated alcohol as a colorless oil.
To a solution of triphenylphosphine (986 mg, 3.76 mmol), imidazole (578 mg, 8.49 mmol) in 20 mL of $CH_2Cl_2$ was slowly added a solution of iodine (954 mg, 3.76 mmol) in 30 mL of $CH_2Cl_2$ at 0 ˚C. After 15 min, a solution of mono-silylated alcohol (351 mg, 1.07 mmol) in 10 mL of $CH_2Cl_2$ was added into the mixture. After being stirred for 6 h at room temperature, the reaction mixture was extracted with EtOAc (100 mL x 2), washed with brine, dried over $MgSO_4$, concentrated in vacuo, and then purified by column chromatography (100% Hexanes) to give 448 mg (96%) of 22-iodo silyl ether **VI** ( = single bond) as a colorless oil: $^1$H NMR (400 MHz, $CDCl_3$) δ 4.03 (m, 1H), 3.33 (dd, *J* = 9.6, 2.8 Hz, 1H), 3.18 (dd, *J* = 9.6, 5.2 Hz, 1H), 1.92-1.75 (m, 3H), 1.70-1.55 (m, 2H), 1.43-1.06 (m, 8H), 0.99 (d, *J*= 6.0 Hz, 3H), 0.94 (t, *J*= 8.0 Hz, 9H), 0.94 (s, 3H), 0.55 (q, *J*= 8.0 Hz, 6H).

**Example 15: Preparation of Compounds I(aa) and I(bb)**

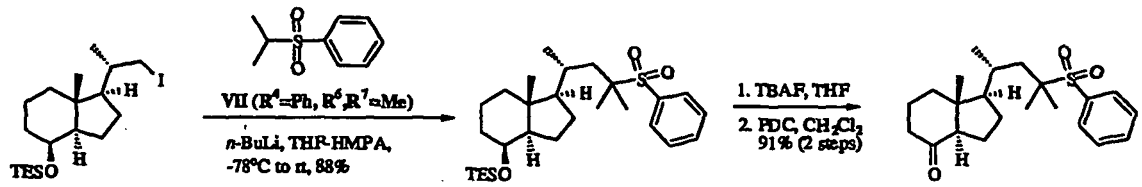


VI

V ($R^4$=Ph, $R^6$,$R^7$=Me)

III ($R^4$=Ph, $R^6$,$R^7$=Me)

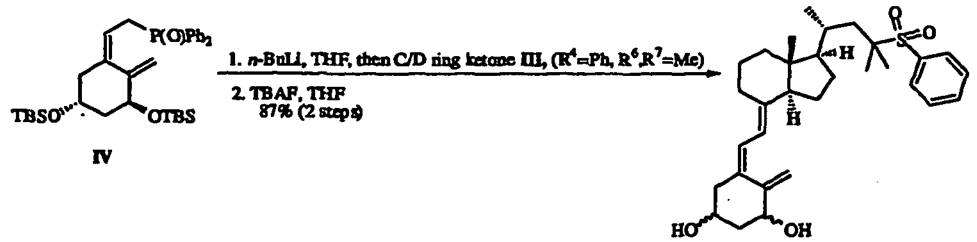


IV

(+)-I(aa) 23-Dimethyl-24-SO2Ph (1α, 3β)
(-)-I(bb) 23-Dimethyl-24-SO2Ph(1β, 3α)

**(a) 23-Dimethyl Silyl Ether V ($R^4$ = Ph, $R^6$, $R^7$ = Me)**: To a solution of isopropyl phenyl sulfone **VII** ($R^4$ = Ph, $R^6$, $R^7$ = Me, Example 12) (38mg, 0.21 mmol) in THF (3mL) at -78 °C was added 0.13 mL (0.21 mmol) of n-BuLi (1.6 M in hexanes). After 15 min stirring, 0.3 mL of HMPA was added at 78 °C. After another 15 min stirring, a precooled (-78 °C) solution of iodide **VI** (---- = single bond, Example 14) (30 mg, 0.069 mmol) in THF (1 mL) was added at -78 °C. The reaction mixture was slowly warmed to room temperature and stirred for 2 h, and then quenched with water, extracted with ether (50 mL x 2), washed with brine, dried over $MgSO_4$, concentrated in vacuo, and then purified by column chromatography (20% EtOAc/hexanes) to give 30 mg (88%) of 23-dimethyl silyl ether **V** ($R^4$ = Ph, $R^6$, $R^7$ = Me) as a colorless oil: $[\alpha]^{24.4}_D$+27.7 (*c* 0.57, $CHCl_3$); $^1$H NMR (400 MHz, $CDCl_3$) δ 7.88-7.85 (m, 2H), 17.66-7.62 (m, 1H), 7.57-7.53 (m, 2H), 4.00 (m, 1H), 1.94-1.84 (m, 2H), 1.81-1.60 (m, 6H), 1.57-1.40 (m, 4H), 0.91(t, *J*= 8.0 Hz, 9H), 0.54 (q, *J*= 8.0 Hz, 6H), 1.34 (s, 3H), 1.30 (s, 3H), 1.26 (s, 3H), 1.14-1.04 (m, 4H), 0.95 (d, *J*= 6.0 Hz, 3H); $^{13}$NMR (100 MHz, $CDCl_3$) δ 135.45, 133.39, 130.67, 128.58, 69.32, 64.10, 57.77, 53.08, 42.23, 40.71, 39.40, 34.50, 32.12, 29.70, 27.90, 22.82, 21.10, 17.59, 14.13. 13.28, 6.94, 4.92; IR (neat, $cm^{-1}$) 2949, 2925, 2872, 1463, 1448, 1378, 1366, 1294, 1282, 1164, 1121, 1075, 1002, 730; HRMS *m/z* ($M^+$ + $Na^+$) calcd 515.2986 for $C_{28}H_{48}O_3SSiNa^+$, found 515.2966.

**(b) 23-Dimethyl C,D-ring Ketone III ($R^4$ = Ph, $R^6$, $R^7$ = Me).** To a solution of silyl ether **V** ($R^4$ = Ph, $R^6$, $R^7$ =Me) (40 mg, 0.080 mmol) in THF (3 mL) was added 0.24 mL (0.24 mmol) of a 1.0 M solution of TBAF in THF, and then it was stirred at 0 °C for 1 h and stirred overnight at room temperature. The reaction mixture was quenched with water (5 mL), extracted with EtOAc (10 mL x 2), washed with brine, dried over MgSO4, concentrated in vacuo, and then purified by column chromatography (25% EtOAc/hexanes) to give 30 mg (99%) of alcohol as a white solid. To a solution of the C,D-ring alcohol (30 mg, 0.080 mmol) in $CH_2Cl_2$ (6 mL) was added 80 mg of oven-dried Celite and PDC (84 mg, 0.22 mmol) at room temperature. The reaction mixture was stirred overnight and then passed through a 2 cm pad of flash silica gel and washed with EtOAc. The filtrate was concentrated and purified by column chromatography (33% EtOAc/hexanes) to give 28 mg (91%) of the desired C,D-ring ketone **III** ($R^4$ = Ph, $R^6$, $R^7$ = Me) as a white solid: mp 149-151 °C; $[\alpha]^{24.7}_D$ +22.3 (*c* 0.96, $CHCl_3$); $^1$H NMR (400 MHz, $CDCl_3$) δ 7.85-7.82 (m, 2H), 7.65-7.61 (m, 1H), 7.55-7.51 (m, 2H), 2.41 (dd, *J*=12.4, 11.2 Hz, 1H), 2.28-2.15 (m, 2H), 2.11-2.06 (m, 1H), 1.96 (m, 1H), 1.91-1.79 (m, 3H), 1.73-1.62 (m, 2H), 1.60-1.43 (m, 6H), 1.32 (s, 3H), 1.27 (s, 3H), 1.00 (d, *J*= 5.6 Hz, 2H), 0.61 (s, 3H); $^{13}$NMR (100 MHz, $CDCl_3$) δ 211.67, 135.29, 133.52, 130.63, 128.66, 63.81, 61.92, 57.62, 49.74, 40.87, 39.48, 38.89, 32.31, 28.03, 23.91, 22.34, 21.26, 21.24, 18.90, 12.31; IR (neat, $cm^{-1}$) 2959, 1715, 1442, 1378, 1305, 1140, 1084, 730, 695; HRMS m/z ($M^+$ + $Na^+$) calcd 399.1964 for $C_{22}H_{32}O_3SNa^+$, found 399.1968.

**(c) 23-Dimethyl-24-$SO_2$Ph analogues (+)-I(aa) and (-)-I(bb)**. A solution of 63 mg (0.11 mmol) of racemic phosphene oxide (±)-**IV** in 2.0 mL of anhydrous THF was cooled to -78 °C and treated with 67.6 μL (0.11 mmol, 1.6 M in hexanes) of n-BuLi under argon atmosphere. The mixture turned reddish orange and was stirred for 10 min at -78 °C. To the solution was added dropwise a solution of 33 mg (0.088 mmol) of the C,D-ring ketone **III** ($R^4$ = Ph, $R^6$, $R^7$ = Me) in 1.0 mL of anhydrous THF. The reaction kept going until the reddish orange color faded to yellow (about 4 h). The reaction was quenched by adding 3.0 mL of pH 7 buffer, then warmed to room temperature, extracted with EtOAc (30 mL x 2), washed with brine, dried over $MgSO_4$, concentrated in vacuo, and then purified by column chromatography (10% EtOAc/hexanes) to afford 30 mg (54%) of the coupled product as a colorless oil.

The coupled product (30 mg, 0.040 mmol) was dissolved in 3 mL of anhydrous THF, and to this solution was added 0.16 mL (0.16 mmol) of a 1.0 M solution of TBAF in THF. The reaction was run in darkness overnight, then extracted with EtOAc (30 mL x 2), washed with brine, dried over $MgSO_4$, concentrated in vacuo, and then purified by column chromatography (80% EtOAc/hexanes) to give 14 mg (67%) of a mixture of two diastereomers as a white solid. The diastereomers were separated by reverse-phase HPLC (C-18 semipreparative column, 49% MeCN/$H_2O$, 3.0 mL/min) to afford 2.5 mg (12%) of **(+)-I(aa)** (1α, 3β, $t_R$ 116 min) and trace amount of **(-)-I(bb)** (1β, 3α, $t_R$ 111 min) as foaming solids. **(+)-I(aa):** $[\alpha]^{24.2}D$ +25.1 (c 0.12, $CHCl_3$); $^1H$ NMR (400 MHz, $CDCl_3$) δ 7.88-7.85 (m, 2H), 7.67-7.63 (m, 1H), 7.57-7.54(m, 2H), 6.36 (d, *J* = 11.2 Hz, 1H), 6.00 (d, *J* = 11.2 Hz, 1H), 5.32 (s, 1H), 4.98 (s, 1H), 4.43 (m, 1H), 4.23 (m, 1H), 2.82 (m, 1H), 2.60 (m, 1H), 2.31 (m, 1H), 2.03-1.82 (m, 8H), 1.70-1.44 (m, 10H), 1.34 (s, 3H), 1.30 (s, 3H), 1.00 (d, *J* = 5.6 Hz, 3H), 0.54 (s, 3H); $^{13}$NMR (100 MHz, $CDCl_3$) δ 147.58, 142.66, 135.38, 133.45, 133.09, 130.66, 128.62, 124.84, 117.21, 111.75, 70.75, 66.81, 63.99, 57.52, 56.31, 45.84, 45.11, 42.79, 40.42, 39.53, 32.85, 28.96, 28.11, 23.46, 22.43, 22.10, 21.30, 21.05, 11.86; IR (neat, $cm^{-1}$) 3436, 2931, 2861, 1719, 1649, 1443, 1296, 11SS, 1126, 1073, 756, 568; UV (MeOH) $\lambda_{max}$ 264 nm (ε 5774); HRMS *m/z* ($M^+$ + $Na^+$) calcd 535.2853 for $C_{31}H_{44}O_4SNa^+$, found 535.2898.

**Example 16: <u>Preparation of Compounds I(cc) and I(dd)</u>**

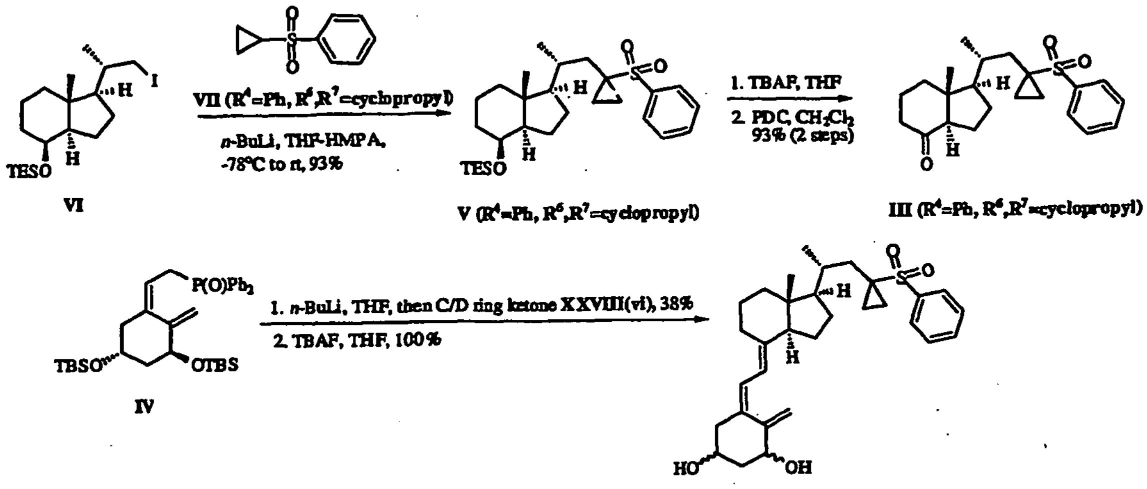


VI

V (R4=Ph, R6,R7=cyclopropyl)

III (R4=Ph, R6,R7=cyclopropyl)

IV

(+)-I(cc) 23-Dimethyl-24-SO2Ph (1α, 3β)
(-)-I(dd) 23-Dimethyl-24-SO2Ph(1β, 3α)

**(a) 25-Cyclopropyl Silyl Ether V ($R^4$ = Ph, $R^6$, $R^7$ = cyclopropyl):** To a solution of cyclopropyl phenyl sulfone **VII** ($R^4$ = Ph, $R^6$, $R^7$ = cyclopropyl) (Example 13, 50mg, 0.27 mmol) in THF (3mL) at -78°C was added 0.17 mL (0.27 mmol) of nBuLi (1.6 M in hexanes). After 15 min stirring, 0.3 mL of HMPA was added at -78 °C. After another 15 min stirring, a precooled (-78 °C) solution of iodide **VI** (Example 14, 40 mg, 0.091 mmol) in THF (1 mL) was added at -78 °C. The reaction mixture was slowly warmed to room temperature and stirred for 3 h, and then quenched with water, extracted with ether (50 mL x 2), washed with brine, dried over $MgSO_4$, concentrated in vacuo, and then purified by column chromatography (15% EtOAc/hexanes) to give 41 mg (93%) of 23-cyclopropyl silyl ether **V** ($R^4$ = Ph, $R^6$, $R^7$ = cyclopropyl) as a colorless oil: $[\alpha]^{23.8}D$+22.2 (*c* 1.85, $CHCl_3$); $^1H$ NMR (400 MHz, $CDCl_3$) δ 7.90-7.87 (m, 2M, 7.66-7.61 (m, 1H), 7.58-7.53 (m, 2H), 3.98 (m, 1H), 2.10-2.06 (m, 1H), 1.90-1.85 (m, 1H), 1.83-1.71 (m, 2H), 1.66-1.43 (m, 5H), 1.34-1.22 (m, 3H), 1.15-1.01 (m, 2H), 0.94(t, *J*= 8.0 Hz, 9H), 0.96-0.80 (m, 2H), 0.85 (s, 3H), 0.78 (d, *J* = 6.4 Hz, 3H), 0.74-0.69 (m, 3H), 0.54 (q, *J* = 8.0 Hz, 6H); $^{13}$NMR (100 MHz, $CDCl_3$) 8 139.18, 133.26, 128.88, 128.73, 69.22, 57.38, 52.93, 42.29, 40.65, 39.29, 37.43, 34.50, 33.56, 27.28, 22.88, 18.81, 17.57, 13.52. 12.57, 12.08, 6.93, 4.90; IR (neat, $cm^{-1}$) 2949, 2875, 1446, 1304, 1142, 1084, 1021, 974, 807, 727, 690; HRMS *m/z* ($M^+$ + $Na^+$) calcd 513.2829 for $C_{29}4_{46}O_3SSiNa^+$, found 513.2863.

**(b) 23-Cycolpropyl C,D-ring Ketone will ($R^4$ = Ph, $R^6$, $R^7$ = cyclopropyl)** To a solution of silyl ether V ($R^4$ = Ph, $R^6$, $R^7$ = cyclopropyl) (36 mg, 0.073 mmol) in THF (3.0 mL) was added 0.22 mL (0.22 mmol) of a 1.0 M solution of TBAF in THF, and then it was stirred at 0 °C for 1 h and stirred overnight at room temperature. The reaction mixture was quenched with water (4 mL), extracted with EtOAc (10 mL x 2), washed with brine, dried over $MgSO_4$, concentrated in vacuo, and then purified by column chromatography (30% EtOAc/hexanes) to give 27 mg (99%) of alcohol

as a colorless oil.

To a solution of the C,D-ring alcohol (27 mg, 0.073 mmol) in $CH_2Cl_2$ (5 mL) was added 70 mg of oven-dried Celite and PDC (77 mg, 0.21 mmol) at room temperature. The reaction mixture was stirred overnight and then passed through a 2 cm pad of flash silica gel and washed with EtOAc. The filtrate was concentrated and purified by column chromatography (33% EtOAc/hexanes) to give 26 mg (93%) of the desired C,D-ring ketone **III** ($R^4$ = Ph, $R^6$, $R^7$ = cyclopropyl) as a white solid: mp 125-127 °C; $[\alpha]^{24.5}_D$ +3.62 (*c* 1.20, $CHCl_3$); $^1H$ NMR (400 MHz, $CDCl_3$) δ 7.88-7.86 (m, 2H); 7.66-7.62 (m, 1H), 7.57-7.53 (m, 2H), 2.35 (dd, *J*=11.6, 11.2 Hz, 1H), 2.29-2.14 (m, 2H), 2.08-2.04 (m, 2H), 2.00-1.95 (m, 1H), 1.89-1.78 (m, 2H), 1.72-1.41 (m, 6H), 1.23 (m, 1H), 1.00-0.95 (m, 2H), 0.90 (d, *J*= 6.4 Hz, 3H), 0.85 (m, 1H), 0.73-0.69 (m, 1H), 0.59 (s, 3H); $^{13}$NMR (100 MHz, $CDCl_3$) δ 211.62, 139.02, 133.43, 128.98, 128.60, 61.74, 57.19, 49.87, 40.85, 39.16, 38.85, 37.85, 33.96, 27.34, 23.90, 18.98, 18.82,12.76, 12.53,12.31; IR (neat, $cm^{-1}$) 2958, 1710, 1446,1379, 1302, 1141, 1083, 728, 692, 643; HRMS *m*/*z* ($M^+$ + $Na^+$) calcd 397.1808 for $C_{22}H_{30}O_3SN^{a+}$, found 397.1807.

**(c) 23-Cyclopropyl-24-$SO_2$Ph analogues (+)-I(cc) and (-)-I(dd).** A solution of 57 mg (0.098 mmol) of racemic phosphine oxide **(±)-IV,** in 2.0 mL of anhydrous THF was cooled to -78°C and treated with 61.1 μL (0.098 mmol, 1.6 M in hexanes) of n-BuLi under argon atmosphere. The mixture turned reddish orange and was stirred for 10 min at -78 °C**.** To the solution was added dropwise a solution of 17 mg (0.046 mmol) of the C,D-ring ketone **III** ($R^4$ = Ph, $R^6$, $R^7$ = cyclopropyl) in 1.0 mL of anhydrous THF. The reaction kept going until the reddish orange color faded to yellow (about 2.5 h). The reaction was quenched by adding 2.0 mL of pH 7 buffer, then warmed to room temperature, extracted with EtOAc (20 mL x 2), washed with brine, dried over $MgSO_4$, concentrated in vacuo, and then purified by column chromatography (30% EtOAc/hexanes) to afford 13 mg (38%) of the coupled product as a colorless oil.

The coupled product (13 mg, 0.018 mmol) was dissolved in 3 mL of anhydrous THF, and to this solution was added 0.07 mL (0.07 mmol) of a 1.0 M solution of TBAF in THF. The reaction was run in darkness overnight, then extracted with EtOAc (20 mL x 2), washed with brine, dried over $MgSO_4$, concentrated in vacuo, and then purified by column chromatography (80% EtOAc/hexanes) to give 10 mg (100%) of a mixture of two diastereomers as a white solid. The diastereomers were separated by reverse-phase HPLC (C-18 semipreparative column, 50% MeCN/$H_2O$, 3.0 mL/min) to afford 2.6 mg (26%) of (+)-I(cc) (1α, 3β, $t_R$ 74 min) and trace amount of (-)-I(dd) (1β), 3α, $t_R$ 71 min) as foaming solids. (+)-**I(cc)**: $[\alpha]^{24.1}_D$ +18.6 (c 0.22, $CHCl_3$); $^1H$ NMR (400 MHz, $CDCl_3$) δ 7.90-7.87 (m, 2H), 7.67-7.62 (m, 1H), 7.58-7.54(m, 2H), 6.36 (d, *J*= 11.2 Hz, 1H), 5.99 (d, *J*=11.2 Hz, 1H), 5.33 (s, 1H), 4.99 (s, 1H), 4.43 (m, 1H), 4.23 (m, 1H), 2.79 (m, 1H), 2.59 (m, 1H), 2.30 (m, 1H), 2.10-1.87 (m, 4H), 1.82-1.74 (m, 2H), 1.28-1.19 (m, 2H), 1.11-1.07 (m, 2H), 1.67-1.53 (m, 8H), 1.00-0.93 (m, 2H), 0.86 (d, *J*= 6.4 Hz, 3H), 0.74-0.68 (m, 2H), 0.50 (s, 3H); $^{13}$NMR (100 MHz, $CDCl_3$) δ 147.61, 142.71, 139.14, 133.34, 133.06, 128.94, 128.68, 124.88, 117.18, 111.78, 70.83, 57.13, 56.17, 45.96, 45.23, 42.86, 40.36, 39.26, 37.78, 34.45, 28.97, 27.54, 23.45, 22.21, 18.99, 12.64, 12.23, 12.04; IR (neat, cm-1) 3401, 2944, 2861, 1647, 1445, 1303, 1142, 1077,1053, 721, 691, 573.

**Example 17: Preparation of Compounds I(ee) and I(ff)**

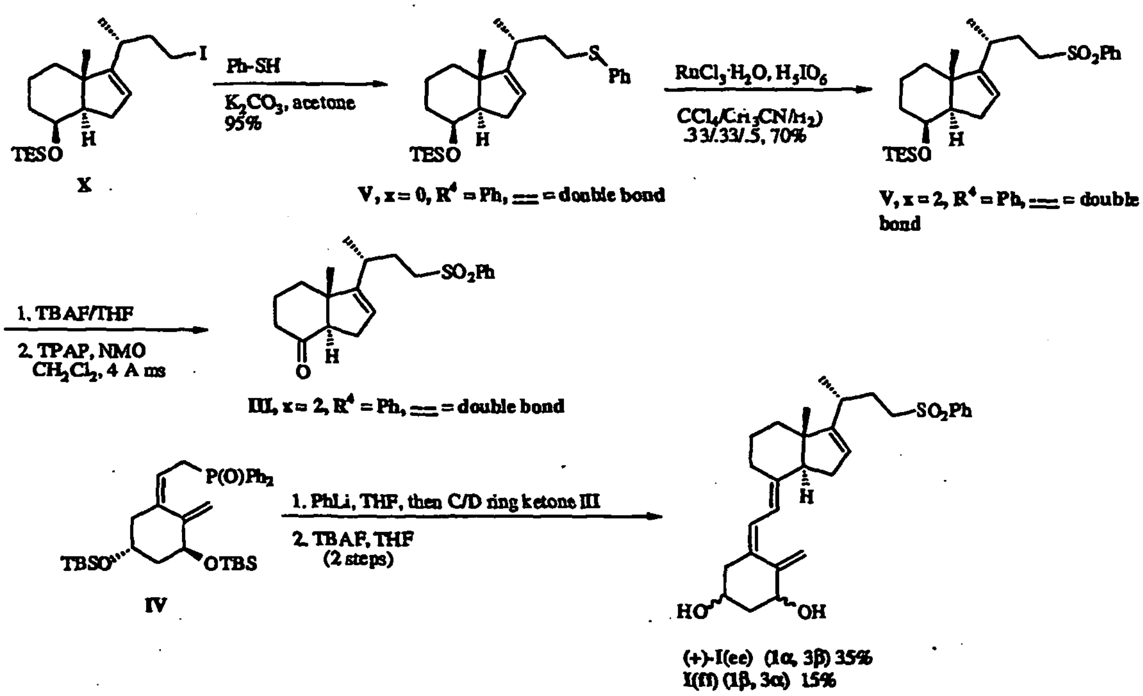


**(a) Compound V (x = 0, ---- double bond, $R^4$ = Ph).** To a flask, 25 mL, containing iodide **X**(---- double bond) (45 mg, 0.100 mmol) was added acetone (2 mL), $K_2CO_3$ (70 mg, 0.502 mmol) and finally thiophenol (52 μL, 0.502 mmol) via a syringe. This mixture was stirred at rt. for 1.5 h and quenched with pH 7.0 phosphate buffer (2 mL). The reaction was extracted with $Et_2O$ (3x, 20 mL), dried over $MgSO_4$, reduced under pressure and purified by silica gel chromatography (100% petroleum ether) to give 45 mg of product as an oil (95%): $[a]^{2.5}_D$ + 18.02 (c 0.3925, $CHCl_3$); $^1H$ NMR (400 MHz, $CDCl_3$) δ 7.27 (m, 4H), 7.14 (m, 1H), 5.25 (m, 1H), 4.10 (d, *J*= 2.4 Hz, 1H), 2.91 (ddd, *J*= 12.8, 9.6, 5.6 Hz, 1H), 2.80 (ddd, *J* = 12.8, 9.2, 6.0 Hz, 1H), 2.21 (m, 2H), 1.93-1.77 (m, 3H), 1.72-1.58 (m, 4H), 1.50-1.39 (m, 2H), 1.33 (dt, *J*= 12.8,3.6 Hz 1H), 0.99 (s, 3H), 0.96 (d, *J*= 6.8 Hz, 3H), 0.94 (t, *J*= 8.0 Hz, 9H), 0.55 (q, *J*= 8.0 Hz, 6H); $^{13}C$ NMR (100 MHz, $CDCl_3$) δ 159.30, 136.93, 128.84, 128.76, 125.58, 120.22, 68.91, 55.07, 48.77, 46.65, 35.68, 34.91, 31.69, 31.05, 30.74, 22.36, 18.72, 18.04, 6.94, 4.91; IR ($CHCl_3$, $cm^{-1}$) 3025, 2954, 1586, 1456, 1028; HRMS *m/z* ($M^+$) calcd 453.261780 for $C_{26}H_{42}OSSiNa^+$ found 453.26329.

**(b) Compound (+)-V (x = 2, ---- = double bond, $R^4$ = Ph.** To a flask, 10 mL, was sequentially added sulfide **V** (x = 0, ---- = double bond, $R^4$ = Ph) (40 mg, 0.093 mmol), $CCl_4$ (0.5 mL), $CH_3CN$ (0.5 mL), $H_2O$ (1 mL) and $H_5IO_6$ (45 mg, 0.195 mmol). This mixture was stirred vigorously for 5 min at rt., after which was added $RuCl_3 \cdot H_2O$ (0.4 mg, 0.0018 mmol) turning the reaction a dark green color. The reaction was stirred until all starting material and intermediate sulfoxide had disappeared by TLC (~2 h) and then passed over a plug of silica gel. The organics were reduced under pressure and purified by silica gel chromatography (85% petroleum ether, 15% ethyl acetate) to give 30 mg of product as an oil (70%): $[a]^{25}_D$ + 21.5 (c 0.893, $CHCl_3$); $^1H$ NMR (400 MHz, $CDCl_3$) δ 7.90 (m, 2H), 7.65 (m, 1H), 7.57 (m, 2H), 5.11 (m, 1H), 4.09 (d, *J* = 2.4 Hz, 1H), 3.12 (ddd, *J* = 14.0, 10.8, 4.8 Hz, 1H), 2.97 (ddd, *J* = 14.0, 11.2, 5.6 Hz, 1H), 2.20 (tt, *J* = 12.8, 1.2 $H_4$ 1H, 2.07 (m, 1H), 1.89-1.72 (m, 4H), 1.69-1.54 (m, 2H), 1.48-1.37 (m, 2H), 1.25 (m, 2H), 0.96 (d, *J*= 6.8 Hz, 3H), 0.94 (t, *J* = 8.0 Hz, 9H), 0.92 (s, 3H), 0.55 (q, *J* = 8.0 Hz, 6H); $^{13}C$ NMR (100 MHz, $CDCl_3$) δ 157.76, 139.07, 133.54, 129.18, 128.05, 121.13, 68.74, 54.98, 54.69, 46.47, 35.52, 34.75, 30.92, 30.68, 28.66, 22.35, 18.70, 17.93, 6.91, 4.87; IR ($CHCl_3$, $cm^{-1}$) 3015, 2933, 1448, 1317, 1149, 1083; HRMS *m/z ($M^+$)* calcd 485.251610 for $C_{26}H_{42}O_3SSiNa^+$ found 485.25125.

**(c) Compound (+)-III (x = 2, ---- = double bond, $R^4$ = Ph).** In a flask, 25 mL, was dissolved the sulfone **V** (x = 2, ---- double bond, $R^4$ = Ph) (28 mg, 0.060 mmol) in THF (1.5 mL). To this was added TBAF (195 μL, 0.195 mmol, 1.0 M in THF) via syringe and the reaction was stirred at rt. for 6 h. The reaction was quenched with water, extracted with $Et_2O$ (3x, 25 mL) and reduced under pressure to give 24 mg of crude product, which was used in the next reaction without further purification.

The crude alcohol was dissolved in $CH_2Cl_2$ (1.5 mL), to which 4 Å ms (~20 mg), NMO (15 mg, 0.130 mmol) and finally TPAP (1.1 mg, 0.0033 mmol) were added. The reaction was vigorously stirred at rt. for 5 h. The crude reaction mixture was passed over a plug of silica and reduced under pressure. The product was then purified by silica gel chromatography (60% hexanes, 40% ethyl acetate) to give 19.1 mg of product (91%): $[\alpha]^{25}_D$ + 22.8 (*c* 0.955, $CHCl_3$); $^1H$ NMR (400 MHz, $CDCl_3$) δ 7.89 (m, 2H), 7.64 (m, 1H), 7.57 (m, 2H), 5.16 (m, 1H), 4.09 (d, *J*= 2.4 Hz,

1H), 3.06 (ddd, *J*=14.0,.10.4, 5.2 Hz, 1H), 2.98 (ddd, *J*= 14.0,10.4, 5.6 Hz, 1H), 2.80 (m, 1H), 2.40 (ddt, *J* = 15.6, 10.8, 1.6 Hz, 1H), 2.27-2.19 (m, 3H), 2.10-2.01 (m, 2H), 1.99-1.90 (m, 1H), 1.89-1.78 (m, 3H), 1.69 (m, 1H), 1.04 (d, *J*= 7.2 Hz, 3H), 0.73 (s, 3H); $^{13}C$ NMR (100 MHz, $CDCl_3$) δ 210.41, 155.62, 139.04, .133.68, 129.28, 127.99, 121.82, 62.94, 54.39, 53.51, 40.37, 34.16, 31.81, 28.41, 2707, 23.87, 21.57, 17.18; IR ($CHCl_3$, $cm^{-1}$) 3018, 2935, 1716, 1450, 1337, 1149, 1096; HRMS *mlz* ($M^+$) calcd 369.149483 for $C_{20}H_{26}O_3SNa^+$ found 369.14909.

**(d) Preparation of Compounds I(ee) and I(ff):** Prior to reaction, phosphine oxide (±)-**IV** and C,D-ring ketone **III** (x = 2, ---- = double bond, $R^4$ = Ph) were azeotrophically dried with benzene and left under vacuum for 24 h. A solution of n-BuLi in hexanes (67 μL, 0.110 mmol) was added dropwise to a cold (-78 °C) solution of phosphine oxide (±)-**IV** (64 mg, 0.110 mmol) in THE (1.20 mL) under dry argon. The resulting deep red solution was stirred for 40 min, at which time a cold (-78 °C) solution of C,D-ring ketone **III** (x = 2, ---- = double bond, $R^4$ = Ph) (19.1 mg, 0.0551 mmol) in THF (1.0 mL) was added dropwise *via* cannula. The resulting solution was stirred at -78°C in the dark for approximately 4 h, after which the dark red color had faded to a light orange color. The reaction mixture was quenched with pH 7.0 phosphate buffer (1 mL), warmed to rt, extracted with $Et_2O$ (3 x 20 mL), washed with brine, dried over $MgSO_4$, filtered, concentrated, and purified by silica gel column chromatography (80% hexanes, 20% ethyl acetate) to afford the coupled products as a clear oil (31.5 mg). This oil was immediately dissolved in THF (1.5 mL) and treated with triethylamine (31 μL, 0.221 mmol) and TBAF (221 μL, 0.221 mmol, 1.0 M in THF) and stirred in the dark for 16 h. The reaction mixture was quenched with $H_2O$ (1 mL), extracted with EtOAc (3 x 15 mL), dried over $MgSO_4$, filtered, concentrated, and purified by silica gel column chromatography (85% ethyl acetate, 15% hexanes) to afford the diol (21 mg) as a mixture of diastereomers. This diastereomeric mixture was separated by HPLC (CHIRALCEL OJ) giving enantiomerically pure, vitamin-$D_3$ analogs **I(ee)** and **I(ff)** in 35% and 15% yield respectively. **I(ee):** $[\alpha]^{25}{}_D$ + 14.7 (c 0.230, $CHCl_3$); $^1H$ NMR (400 MHz, $CDCl_3$) δ 7.9 (m, 2H), 7.66 (m, 1H), 7.58 (m, 2H), 6.35 (d, *J*=11.2 Hz, 1H), 6.08 (d, *J*=11.2 Hz, 1H), 5.34 (m,1H), 5.18 (m, 1H) 5.00 (m, 1H), 4.44 (m, 1H), 4.24 (m, 1H), 3.08 (ddd, *J*= 14, 10.8, 4.8 Hz, 1H), 2.97 (ddd, *J*= 14.0, 10.8, 4.8 Hz, 1H), 2.79 (m, 1H), 2.59 (dd, *J*= 13.6, 3.2 Hz, 1H), 2.32 (m, 2H), 2.17 (m, 2H), 2.07-2.01 (m, 1H), 1.97 (m, 1H), 1.92-1.86 (m, 1H), 1.74 (m, 2H), 1.67-1.51 (m, 2H), 1.40 (m, 1H), 1.02 (d, *J* = 6.8 Hz, 3H), 0.60 (s, 3H); $^{13}C$ NMR (100 MHz, $CDCl_3$) δ 157.28, 147.62, 141.86, 139.07, 133.60, 133.31, 129.24,

**Example 8** **CYP24 Enzyme Assay (Induced KPK1A-ras Cells)**

**(i) Material and reagents:**

$1,25(OH)_2D_3$ $10^{-5}$ M
[$^3$H]- $1,25(OH)_2D_3$ 25,000 CPM/μl
HPK1A-ras cells
48-well plate
Methanol
Dichlorimethane Saturated KCl: KCl 30g, $H_2O$400 ml

**(ii) Procedure:**

1. Induction of HPK1A-ras cells (The day before assay)
When the HPK1A-ras cells are 80-90% confluent, add 1 μL $10^{-5}$ M $1,25(OH)_2D_3$ to 1 mL medium in the plate (final concentration is $10^{-8}$ M).
2. Preparation of cell suspension
After 18 to 20 hours induction, remove the medium and wash the cell twice with PBS. Then tripsinize the cells from plate, centrifuge (2,000 rpm, 5 min) and suspend cells pellet in DMEM medium+1%BSA.
Count the cells and adjust cells density to 250,000/150 μL, add 150 μL cell suspension to each well in 48-well plate. (including 3 well as no cell control, and 3 well cells without drug or inhibitor as control).
3. Add 25 μL ketoconazole (final concentration $10^{-5}$ M, $10^{-6}$ M, $10^{-7}$ M, $10^{-8}$ M) or drugs into each designated well. Keep the plate in 37°C for 10 min.
4. Preparation of substrate
Take certain amount of DMEM+1%BSA medium (25*Well number+200)μL to a tube, add certain amount of $^3$H-1,25 $(OH)_2D_3$ (well number+2) μL and certain amount of 100mM DPPD (well number/5)μL and mix them by vortex.
5. Incubation

Add 25 μL substrate to each well, incubate the plate at 37°C for 3 hour.
Add 25 μL substrate to counting plate (2 well) as a total count.
6. Lipid extraction and counting
Add 500 μL methanol to each well to stop the reaction, transfer them to tube
Add 250 μL dichloromethane and vortex.
Add 250 μL dichloromethane and 250 μL saturated KCl, and vortex.
Centrifuge at 4000 rpm for 5 min.
Transfer 100 μL of aqueous phase (upper phase) to counting plastic counting plate. Add 600 μL of scintillation fluid to each well. Count the plate in scintillation counter.
7. Calculation enzyme activity
CPM of cell control after subtraction of CPM of NCC is as 100% enzyme activity.

**Enzyme activity = (CPM in test compounds well – CPM in NCC well)/(CPM in Cell control - CPM in NCC well) * 100%**

Dilution of Ketoconazole

| Stock $10^{-2}$ M | | | |
|---|---|---|---|
| Concentration (final) | From previous step | DMEM + 1%BSA | Concentration (actual) |
| $10^{-5}$ M | 4 | 496 | $8*10^{-5}$ M |
| $10^{-6}$ M | 12.5 | 112.5 | $8*10^{-6}$ M |
| $10^{-7}$ M | 12.5 | 112.5 | $8* 10^{-7}$ M |
| $10^{-8}$ M | 12.5 | 112.5 | $8*10^{-8}$ M |

Dilution of test compounds

| Stock $10^{-3}$ M | | | |
|---|---|---|---|
| Concentration (final) | From previous step (μL) | DMEM + 1%BSA (μL) | Concentration (actual) |
| $10^{-5}$ M | 10 | 115 | $8*10^{-5}$ M |
| $10^{-6}$ M | 12.5 | 112.5 | $8*10^{-6}$ M |
| $10^{-7}$ M | 12.5 | 112.5 | $8*10^{-7}$ M |
| $10^{-8}$ M | 12.5 | 112.5 | $8*10^{-8}$ M |

**(iii) Results:**

See Figure 1A for Compound I(a) and see Table 1.

**(iv) References:**

Ray S, Ray R, Holick M. Metabolism of 3H-1alpha, 25-dihydroxyvitamin D3 in the cultured human keratinocytes (1995) 59:117-122
Dilworth F J, Scott I, Green A, Strugnell S, Guo Y D, Roberts E A, Kremer R, Calverley, M J, Makin H L J, Jones G. Different mechanisms of hydroxylation site selection by liver and kidney cytochrome P450 species (CYP27 and CYP24) involved in Vitamin D metabolism. (1995) J Biochem 270(28):16766-16774

**Example 9** **Assay of CYP1-alpha hydroxylase (Using Transfected COS-1 Cells)**

**(A) Transit transfection**

(i) Reagent and material

1. COS-1 cells (50-80% confluent)
2. FuGene 6 Transfection Reagent
3. PcDNA vector containing CYP-1alpha hydroxylase cDNA(1μg/μl)
4. DMEM Medium + 10% FCS
5. DMEM Medium (serum-free)
6. 6-well plate

(ii) Transfection cocktail preparation (The mount depends on how many wells transfected)

1. To a sterile tube, add serum-free medium (100 μl per well), Then add FuGene 6 Reagent (3 (μl per well). Tap gently to mix. Pay attention to the order. Add FuGene 6 Reagent directly to medium, do not allow undiluted Fugene 6 Reagent to come in contact with plastic surfaces other than the pipette tip.
2. Add DNA solution (1 μg per well) to the prediluted FuGene 6 Reagent from step 2
3. Gentyl tap the tube to mix the contents. Do not vortex. Incubate for 15 min at room temperature (no more than 45 min).

(iii) Cells preparation.

1. Trypsinize Cos-1 cells, centrifuge cell suspension, suspend cells pellet in DMEM medium +10% FCS..
2. Dilate the cells suspension to 750,000 cell/ml (75cell/square),

(iv) Transfection

1. Add 1.7 ml DMEM medium+10%FCS to each well of 6 well plate.
2. Transfer the correct volume of the cell suspension (200 μl/well) to the transfection cocktail. Mix them gently
3. Add 0.3 ml of the mixture to each well. **Make sure that the same amount cells are added to each well.** Swirl the wells to ensure even dispersal.
4. Incubate the cells for 24 hours at 37˚C, 5% $CO_2$ until enzyme activity assay.

**(B) Enzyme Activity Assay**

(i) Reagent and material
DMEM medium +1% BSA
PBS
[$^3$H-26,27]-25(OH)$D_3$
DPPD 100mM
(ii) Procedure

1. Wash cells once with PBS. **Be careful, don't disturb the attached cells**
2. Add 0.55 ml medium (DMFM+1%BSA) each well.
3. Add 0.025 ml medium containing test compounds
4. Incubate the cells for 10 minutes
5. Add 0.025ml medium containing [$^3$H-26,27]-25(OH)$D_3$ (50,000 CPM) and DPPD (0.6 μl stock)
6. Incubate the cells for 2 hour.
7. Add 1.5 ml Methanol to stop reaction
8. Add internal standard.
9. Transfer the medium to labeled tube.
10. Add 0.75 ml dichloromethane, vortex and keep in room temperature for 15 minutes.

11. Add 0.75 ml dichloromethane and 0.75 ml saturated KCl
12. Vortex and centrifuge
13. Remove upper phase and dry the lower phase in Speed-Vac
14. Add 110 μl mobile phase, vortex and centrifuge for 5 min.
15. Transfer 105 μl to the insert in HPLC vial.
16. HPLC analysis conditions:

    Solvent: Hexane/isopropanol/methanol (91/7/2)
    Column SIL 3μm column
    Flow rate: 2 ml/min
    Detector: UV detector and radioactive detector.

**(C) Results**

See Figure 1B for Compound I(a) and see Table 1.

**(D) References**

Shink T, Shimada H, Wakino S, Anazawa H, Hayashi M, Saruta T, Deluca H, Suda T. Cloning and expression of rat 25-hydroxyvitamin D3-1-alpha -hydroxylase cDNA. (1997) Pro. Natl. Acad Sci 94:12920-12925 Muralidharan K R, Rowland-goldsmith M, Lee S A, Park G, Norman A W, Henry H L, Okamura W H. Inhibitors of 25-hydroxyvitamin D3-1alpha-hydroxylase: Thiavitamin D analogues and biological evaluation. (1997) J Steroid Biochem. Molec. Biol. 62(1):73-78.

**Example 10 CYP27A1 Enzyme Assay**

**(A) Procedure:**

As described in:

Dilworth F J, Black S M, Guo Y D, Miller W L, Jones G. Construction of a P450c27 fusion enzyme: a useful tool for analysis of vitamin D3 25-hydroxylase (1996) Biochem J 320:267-271
Sawada N, Sakaki T, Ohta M, Inouye K. Metabolism of vitamin D (3)by human CYP27A1 (2000) Biochem Biophys Res Commun 273(3):977-84

**(B) Results:**

See Figure 1C for compounds I(a) and Table 1.

**Example 11 VDR Binding Assay**

**(A) Reagent and material**

1. VDR 9,3 pmol/μl (human, recombinant, Biomol).
2. [$^3$H]-1,25$(OH)_2D_3$ in ethanol
3. 1,25$(OH)_2D_3$ in ethanol
4. $TEK_{300}$

    Tris-HCl 50 mM
    EDTA 1.5 mM
    KCl 300 mM
    Adjust pH to 7.4 (25°C)

5. $TEDK_{300}$

$TEK_{300}$
DTT (dithiothreitol) 10 mM (MW 154.24)
6. Tris buffer
22.50 g Tris-HCl
500 ml $H_2O$
13.25 g Tris-base
500 ml $H_2O$
Kept in 4˚C
7. Dextran-T70 (Mol 70,000) Pharmacia
8. Charcoal (carbon decolorizing neutral, norit) Fishery
9. Gelatin (G-2625 Sigma)

**(B) Reagent Preparation**

1. Charcoal dextran solution

(1) Tris buffer
Mix equal amount of Tris-HCl and Tris-base.
(2) Norit decolorizing neutral charcoal 2.0 g
Tris buffer 150 mL
Stirring
(3) Dextran T - 70 0.2 g
Tris buffer 50 ml.
(4) Slowly drip the suspended dextran into charcoal solution with stirring.
Keep in refrigerate overnight.
Thirty minute before use, store on ice with continuous mixing

2. $TEK_{300}$/Gelatin solution
50 mg swine gelatin
5 ml $TEDK_{300}$ solution
heating, stirring then cooling to 4˚C.
5 ml $TEDK_{300}$ solution
3. Preparation of $1,25(OH)_2D_3$ and test compounds in ethanol
$1,25(OH)_2D_3$: 125, 250, 500, 1000, 2000, 4000 pg/250. (stock 10-5 M/25μL = 100,000pg/25μL)
Test compounds: 12,500, 25,000, 50,000, 100,000, 200,000 and 400,000 pg/25 μL. (4*10-5M/25μL = 400,000 pg/25μL)

| Label | Concentration (ng/mL) | Amount (pg/50μL) |
|---|---|---|
| | 5.0 | 125 |
| Std F | 10.0 | 250 |
| Std G | 20.0 | 500 |
| Std H | 40.0 | 1000 |
| | 80.0 | 2000 |
| Std I | 160.0 | 4000 |

4. Dilution of VDR
1 μl stock VDR in 2.5 ml $TEDK_{300}$/Gelatin solution (500μl/tube), (keep on ice)

**(C) Assay:**

| label | Standards | NSB buffer | VDR | 1h RT | $^{3}H$-1,25$(OH)_2D_3$ | 1h RT - | Reagent C -charcoal | On ice 30 min | Spin at 4°C |
|---|---|---|---|---|---|---|---|---|---|
| TC (Total) | 25µL reagent D | 100 µL reagent L | 500µL reagent A | | 50µL reagent B<br>mix all tubes | | 100µL reagent C<br>mix all tubes | | 2000 rpm, 10 min<br>Add 100 µl to counting rack<br>Count 5-10 min |
| NSB (non-specific b) | | 500 µL reagent L | | | | | | | |
| Max $b_0$ binding | | | 500 µL reagent A | | | | | | |
| Standard | 25 µL of each standard | | | | | | | | |
| Test | 25 µL of each concentration of sample | | mix all tubes | | | | | | |

**(D) Calculations:**

The amount of 1,25$(OH)_2D_3$ to displace 50 percent [$^3$H]-1,25$(OH)_2D_3$ from VDR is calculated as $B_{50}$ for 1,25 $(OH)_2D_3$. The VDR binding of other compounds is calculated as $B_{50}$ relative to a value of 1 for 1,25$(OH)_2D_3$.

Dilution of 1,25$(OH)D_3$

| Concentration (pg/25ul) | Final concentration M | $10^{-5}$ M | Ethanol (ul) |
|---|---|---|---|
| 4,000 | $2*10^{-8}$ | 6 | 144 |
| 2,000 | $10^{-8}$ | 70 | 70 |
| 1,000 | $5*10^{-9}$ | 70 | 70 |
| 500 | $2.5*10^{-9}$ | 70 | 70 |
| 250 | $1.25*10^{-9}$ | 70 | 70 |
| 125 | $6.25*10^{-10}$ | 70 | 70 |

Dilution of test compounds

| Concentration (pg/50ul) | Final concentration M | $10^{-3}$ M | Ethanol |
|---|---|---|---|
| 400,000 | $2*10^{-6}$ | 6 | 144 |
| 200,000 | $10^{-6}$ | 70 | 70 |
| 10,000 | $5*10^{-7}$ | 70 | 70 |
| 5,000 | $2.5*10^{-7}$ | 70 | 70 |
| 25,000 | $1.25*10^{-7}$ | 70 | 70 |
| 12,500 | $6.25*10^{-8}$ | 70 | 70 |

**(E) Results:**

See Figure 2 and Table 1.

**(F) References:**

1. Ross T K, Prahl J M, DeLuka H. Overproduction of rat 1,25-dihydroxyvitamin D3 receptor in insect cells using the baculovirus expression system. (1991) Proc Natl Acd Sci USA 88:6555-6559

2. Wecksler W R, Norman A W. An hydroxylapatite batch assay for the quantitation of 1alpha, 25-dihydroxyvitamin D3-receptor complexes (1979) Anal Biochem 92:314-323

**Example 12** **Transcriptional Activity Assay**

**(A) Reagent and material:**

pSG5-hVDR1/3 from DRs. Mark Haussler and Kerr Whitfield, (University of Arizona, Tucson AZ); hVDR1/3 DNA inserted into the EcoRI site of pSG5vector (CT4)$^4$TKGR from DRs. Mark Haussler and Kerr Whitfield, (University of Arizona, Tucson, AZ); Four copies of the CT4 synthetic rat osteocalcin VDRE ligated and annealed into pTKGH vector which has a thymidine promoter linked to the human GH gene.
hGH ELISA kit Boehringer Mannheim
Fugene 6 transfection reagent
COS-1 cells
DMEM medium and DMEM medium+10%FCS

$1,25(OH)_2D_3$ and test compounds

**(B) Transfection:**

1. Subculture COS cells into 24-well plate (5,000 cell/well) one day before transfection.
2. Cocktail preparation (the amount depends on how may wells transfected).

   (1) To a sterile tube, add serum-free medium (100 μl per well), Then add FuGene 6 Reagent (0.6 μl per well). Tap gently to mix. Pay attention to the order. Add FuGene 6 Reagent directly to medium, do not allow undiluted Fugene 6 Reagent to come in contact with plastic surfaces other than the pipette tip.
   (2) Add DNA solution (pSG5-hVDR1/3 and $(CT4)^4$TKGH vectors) (0.1 μg each per well) to the prediluted FuGene 6 Reagent from step 2
   (3) Gently tap the tube to mix the contents. Do not vortex. Incubate for 15 min at room temperature (no more than 45 min).

3. Remove the medium and replaced by 0.4 ml fresh medium
4. Add the 100μl cocktail to each well in drop-wise manner.

**(C) Treatment of transfected cells with different concentration of $1,25(OH)_2D_3$ and test compounds:**

30 min to 1 hour after transfection, $1,25(OH)_2D_3$ (as control) and test compounds are added to the medium in 20 μl medium. The concentration range for $1,25(OH)_2D_3$ is $10^{-10}$ to $10^{-8}$ M ($10^{-10}$, $3*10^{-9}$, $10^{-9}$,$3*10^{-8}$,$10^{-8}$, M) and for test compounds is from $3*10^{-9}$M to $10^{-7}$M ($3*10^{-9}$ $10^{-9}$,$3*10^{-8}$,$10^{-8}$, $3*10^{-8}$, $10^{-7}$ M). Incubation continues for 24 hours.

**(D) Measurement of GH content in medium:**

After 24 hour incubation, 200 μL diluted aliquots of medium (dilution of 20-50 times) are used for human GH determination. Sample is assayed according to instruction of hGH ELISA kit

(E) Results:

See Figure 3 and Table 1.

**(F) References**

Hashimoto Y, Ikeda I, Ikeda M, Takahashi Y, Hosaka M, Uchida H, Kono N, Fukui H, Makino T, Honjo M. Construction of a specific and sensitive sandwich enzyme immunoassay for 20 KD human growth hormone (1998) J Immumol Methods 221:77-85
Jone G, Byford V, Makin H L J, Kremer R, Rice R H, deGraffenried L A, Knutson J C, Bishop C W. Anti-proliferative activity and target cell catabolism of the vitamin D analogue 1alpha, 24(OH)2D2 in normal and immortalized human epidermal cells (1996) Biochem Pharmacol 52:133-140

**Example 13** **<u>DBP Binding Assay (Human Plasma)</u>**

**(A) Reagents:**

1. Tris buffer: .
22.50 g Tris-HCl
500 ml $H_2O$
2. 13.25 g Tris-base
500 ml $H_2O$
Kept in 4˚C
3. Dextran-T70 (Mol 70,000) Pharmacia
4. Charcoal (carbon decolorizing neutral, norit) Fishery

5. DBP (vitamin D binding protein) (human plasma)
6. $[^3H]25(OH)D_3$
7. Gelatin (G-2625 Sigma)

**(B) Reagent preparation:**

1. Tris buffer
Mix equal volume of two Tris buffer.
2. Dextran coated charcoal solution
(1) preparation of charcoal solution

| | |
|---|---|
| Norit decolorizing neutral charcoal | 2.0 g |
| Tris buffer | 150 mL |
| Stirring | |

(2) preparation of dextran solution
Dextran T - 70 0.2 g
Tris buffer 50 ml
(3) preparation of dextran coated charcoal solution
Slowly drip the dextran solution into charcoal solution with stirring.
Keep in refrigerate overnight
Thirty minute before use, keep it on ice with continuous mixing.
This solution can be kept in 4˚C for 2 month.
3. Tris buffer/Gelatin solution
250 mg swine gelatin
50 ml Tris buffer
heating, stirring and cooling on ice.
Prepared just before use.
4. DBP solution
Human plasma is diluted to 1:5000 with Tris buffer/gelatin solution
5. Dilution of Standard $25(OH)D_3$
Stock 10,000pg/50 μl
Diluted to 0, 62.5, 125, 250, 500, 750, 1000,10,000 pg/50 μl with ethanol
6. Dilution of Standard $1,25(OH)_2D_3$
Stock 200,000 pg/50 μl ($10^{-5}$ M/50 ul)
Diluted to 6,250,12,500, 25,000, 50,000, 100,000, 200,000 pg/50 μl with ethanol
7. Dilution of test compounds
Stock 200,000pg /50 μl ($10^{-3}$ M)
Diluted to 12,500, 25,000, 50,000, 100,000, 200,000 and 400,000pg/50 μl with ethanol
8. $[^3H\text{-}26,27]\text{-}25(OH)_2D_3$ solution
The stock solution is diluted in Tris buffer, 20,000 CPM/50μl.

**(C) Assay**

| Label | 25(OH) $D_3$ | Test compounds (µl) | 3H-25(OH) $D_3$ (µl) | DBP (µl) | Super mix | Incubation (Rm T) | Charcoal dextran (µl) | On ice | Centrifuge | Counting |
|---|---|---|---|---|---|---|---|---|---|---|
| 1-3 (total) | ----- | ----- | 50 | ----- | 600<br>600 | -----<br>----- | ----- | | ----- | ----- |
| 4-8 | ----- | ----- | 50 | 500 | | ----- | ----- | | ----- | ----- |
| STD 5-35 | 50 | ----- | 50 | | - | 4 h | 200 | 1h | 2000rpm 15min, 4˚C | 200 µl Super+600 µl Supermix |
| Test 36- | ----- | 50 | 50 | | - | | | | | |

**(D) Calculation:**

The amount of 25(OH)$D_3$ to displace 50 percent [$^3$H]-25(OH)$D_3$ is calculated as $B_{50}$ for 25(OH)$D_3$ DBP binding. The DBP binding of other compounds is calculated as $B_{50}$ relative to a value of 1 for 25(OH)$D_3$.

**(E) Dilution of 25(OH)$D_3$:**

| Amount (mol/50ul) | From previous steps (μl) | Ethanol (μl) |
|---|---|---|
| $2.5*10^{-11}$ ($5*10^{-7}$ M) | $5*10^{-7}$ M | |
| $2.5*10^{-12}$ | 40 | 360 |
| $1.875*10^{-12}$ | 90 | 30 |
| $1.25*10^{-12}$ | 130 | 130 |
| $6.25*10^{-13}$ | 130 | 130 |
| $3.125*10^{-13}$ | 130 | 130 |
| $1.5625*10^{-13}$ | 130 | 130 |

**(F) Dilution of 1, 25(OH)$D_3$**

| Amount (mol in 50μl) | From previous steps (μl) | Ethanol (μl) |
|---|---|---|
| $5*10^{-10}$ ($10^{-3}$ M) | | |
| $2.5*10^{-10}$ | 130 | 130 |
| $125*10^{-10}$ | 130 | 130 |
| $6.25*10^{-11}$ | 130 | 130 |
| $3.215*10^{-11}$ | 130 | 130 |
| $1.625*10^{-11}$ | 130 | 130 |

**(G) Dilution of test compounds:**

| Amount (mol in 50μl) | From previous steps (μl) | Ethanol (μl) |
|---|---|---|
| Stock ($10^{-3}$ M) | | |
| $1.0*10^{-9}$ | 5 | 245 |
| $5.0*10^{-10}$ | 130 | 130 |
| $2.5*10^{-10}$ | 130 | 130 |
| $1.25*10^{-10}$ | 130 | 130 |
| $6.25*10^{-11}$ | 130 | 130 |
| $3.125*10^{-11}$ | 130 | 130 |

**(H) Results:**

See Figure 4.

**(I) References:**

Bouillon R, van Baelen H, Moor P D. Comparative study of the affinity of the serum vitamin D -binding protein. (1980) 1 Steroid Biochem 13:1029-44.
Jones L, Byrnes B, Palma P, Segev D, Mazur E. Displacement potency of vitamin D2 analogue in competitive protein-binding assay for 25-hydroxyvitamin D3, 24,25-dihydroxyvitamin D3 and 1,25-dihydroxyvitamin. D3 (1980) J Clin Endocrinol Metab 50:773-775

**Example 14: Calcium Excretion**

Compound I(a) was tested for its effect on calcium excretion and weight gain in rats using a protocol described in Posner et al. J. Med. Chem. 41, 3008-3014, 1998. At a concentration that was 20 fold greater than the concentration of calcitriol (1$\alpha$,25-dihydroxy vitamin $D_3$), compound I(a) did not show an increase in urinary calcium levels. Compound I(u) was also tested and found to be strongly non-calcemic.

While the present invention has been described with reference to what are presently considered to be the preferred examples, it is to be understood that the invention is not limited to the disclosed examples. To the contrary, the invention is intended to cover various modifications and equivalent arrangements included within the spirit and scope of the appended claims.

All publications, patents and patent applications are herein incorporated by reference in their entirety to the same extent as if each individual publication, patent or patent application was specifically and individually indicated to be incorporated by reference in its entirety.

**Table 1: Summary of Biological Activity for Compounds of the Invention**

| Cpd # | Structure | $IC_{50}$ (nM) | CYP27B1 $IC_{50}$ (nM) | CYP27A1 $IC_{50}$ (nM) | Cell Anti-Proliferative Activity* |
|---|---|---|---|---|---|
| I(a) | 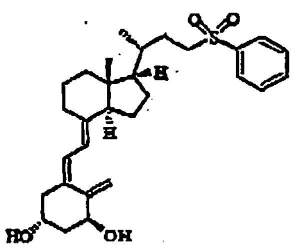 | 28 | >10,000 | >10,000 | |
| I(e) | 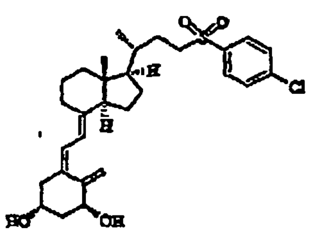 | 94 | >1000 | | |
| I(g) | 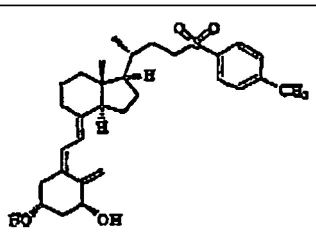 | 212 | >1000 | | |

(continued)

| Cpd # | Structure | $IC_{50}$ (nM) | CYP27B1 $IC_{50}$ (nM) | CYP27A1 $IC_{50}$ (nM) | Cell Anti-Proliferative Activity* |
|---|---|---|---|---|---|
| I(i) | 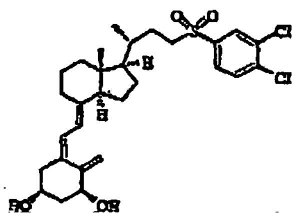 | 92 | >1000 | | |
| I(u) | 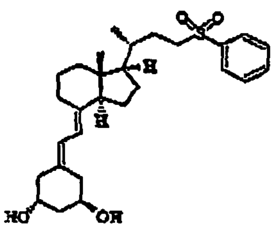 | 160 | | | |
| I(cc) | 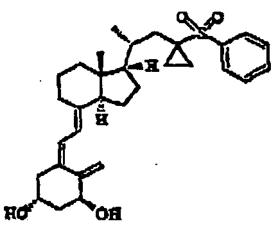 | 27 467 | | | |
| * compared to calcitrol | | | | | |

## Claims

**1.** A compound of Formula I, and pharmaceutically acceptable salts, hydrates, solvates and prodrugs thereof, wherein the prodrug is an ester prodrug selected from the group consisting of phenyl esters, aliphatic $C_8$-$C_{24}$ esters, acyloxymethyl esters, carbamates and amino acid esters:

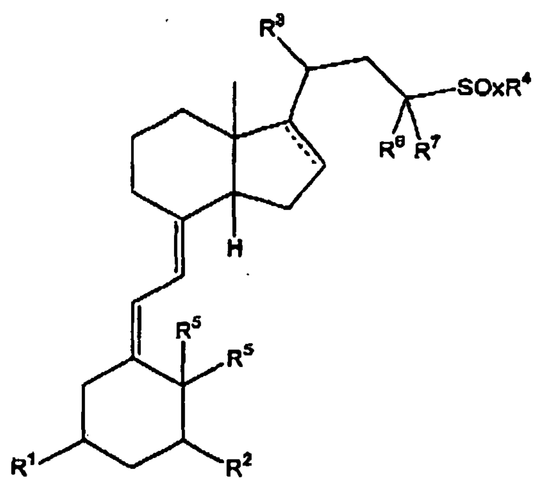

I

wherein

$R^1$ and $R^2$ are independently selected from the group consisting of OH, $OC_{1-4}$alkyl, and halo;

$R^3$ is $C_{1-4}$alkyl;

$R^4$ is selected from the group consisting of aryl and heteroaryl with both aryl and heteroaryl being unsubstituted or substituted with 1-5 groups independently selected from $C_{1-4}$alkyl, hydroxy-substituted $C_{1-6}$alkyl, $OC_{1-4}$alkyl, OH,

$CF_3$, $OCF_3$, halo, SH, $SC_{1-4}$alkyl, $NH_2$, $NHC_{1-4}$alkyl, $N(C_{1-4}alkyl)(C_{1-4}alkyl)$, CN, C(O)OH, $C(O)OC_{1-4}$alkyl, $C(O)NHC_{1-4}$alkyl, CH=N-$OC_{1-4}$alkyl, $NHC(O)C_{1-4}$alkyl, $OC(O)C_{1-4}$alkyl, $SOC_{1-4}$alkyl, $SO_2C_{1-4}$alkyl, $SO_2NHC_{1-}$alkyl and $SO_2NH_2$:
$R^5$ are either both H or together form $=CH_2$;
$R^6$ and $R^7$ are independently H, $C_{1-4}$alkyl or are taken together to form a $C_{3-6}$cyloalkyl ring;
x is 0-2; and
---- represents a single or a double bond.

**2.** A compound according to claim 1, wherein $R^1$ and $R^2$ are independently selected from the group consisting OH, $OCH_3$, and fluoro.

**3.** A compound according to claim 2, wherein $R^1$ and $R^2$ are both OH.

**4.** A compound according to claim 1, 2 or 3 wherein $R^3$ is $CH_3$.

**5.** A compound according to any one of claims 1 to 4, wherein $R^4$ is selected from the group consisting of unsubstituted and substituted phenyl, pyridyl, thienyl, furanyl and pyrrolo.

**6.** A compound according to claim 5, wherein $R^4$ is selected from unsubstituted or substituted phenyl.

**7.** A compound according to any one of claims 1 to 4, wherein both aryl and heteroaryl are either unsubstituted or substituted with 1-3 groups independently selected from $C_{1-4}$alkyl, hydroxy-substituted $C_{1-6}$alkyl, $OC_{1-4}$alkyl, OH, $CF_3$, $OCF_3$, halo, SH, $SC_{1-4}$alkyl, $NH_2$, $NHC_{1-4}$alkyl, $N(C_{1-4}alkyl)(C_{1-a}alkyl)$, CN, C(O)OH, $C(O)OC_{1-4}$alkyl, CH=N-$OC_{1-4}$alkyl, $C(O)NHC_{1-4}$alkyl, $NHC(O)C_{1-4}$alkyl, $OC(O)C_{1-4}$alkyl, $SOC_{1-4}$alkyl, $SO_2C_{1-4}$alkyl, $SO_2NHC_{1-4}$alkyl and $SO_2NH_2$.

**8.** A compound according to claim 7, wherein both aryl and heteroaryl are either unsubstituted or substituted with 1-2 groups independently selected from methyl, 3-hydroxy-3-pentyl, methoxy, OH, $CF_3$, $OCF_3$, halo, $NH_2$, $NMe_2$ and CH=N-OMe.

**9.** A compound according to claim 8, wherein both aryl and heteroaryl are either unsubstituted or substituted with 1-2 groups independently selected from methyl, 3-hydroxy-3-pentyl, Cl, F and CH=N-OMe.

**10.** A compound according to claim 6, wherein $R^4$ is selected from the group consisting of phenyl, 4-chlorophenyl, 3,4-dichloropheny, 4-fluorophenyl, 4-methylphenyl, 3,4-difluorophenyl, 4-(3-hydroxy-3-pentyl)phenyl, 4-(CH=N-OMe)phenyl, 4-methoxyphenyl, 4-trifluormethylpheny and 4-nitrophenyl.

**11.** A compound according to claim 10, wherein $R^4$ is selected from the group consisting of phenyl 4-chlorophenyl, 3,4-dichlorophenyl, 4-(3-hydroxy-3-pentyl)phenyl, 4-fluorophenyl and 4-methylphenyL

**12.** A compound according to any one of claims 1 to 11, wherein $R^6$ and $R^7$ are independently H, methyl or are taken together to form a $C_{3-4}$cyloalkyl ring.

**13.** A compound according to claim 12, wherein $R^6$ and $R^7$ an both H or are taken together to form a $C_{3-4}$cycloalkyl ring.

**14.** A compound according to any one of claims 1 to 13, wherein x is 2.

**15.** A compound according to any one of claims 1 to 14, wherein ---- is a single bond.

**16.** A compound according to claim 1, of Formula I', and pharmaceutically acceptable salts, hydrates, solvates and prodrugs thereof, wherein the prodrug is an ester selected from the group consisting of phenyl esters, aliphatic $C_8$-$C_{24}$ esters, acyloxymethyl esters carbamates and amino acid esters:

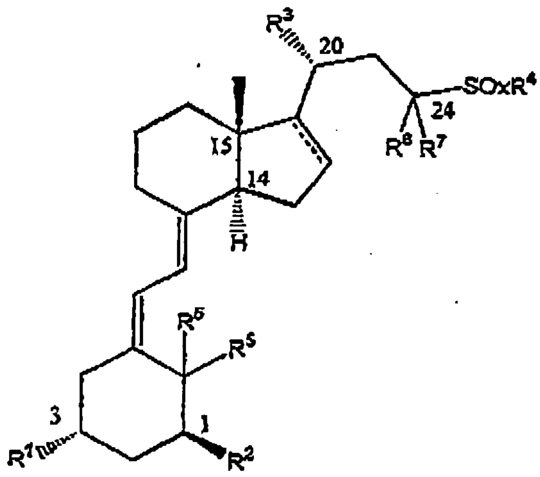

I'

wherein

$R^1$ and $R^2$ are independently selected from the group consisting of OH, $OC_{1-4}$alkyl, and halo;

$R^3$ is $C_{1-4}$alkyl;

$R^4$ is selected from the group consisting of aryl and heteroaryl with both aryl and heteroaryl being unsubstituted or substituted with 1-5 groups independently selected from $C_{1-4}$alkyl, hydroxy-substituted $C_{1-4}$alkyl, $OC_{1-4}$alkyl; OH, $CF_3$, $OCF_3$, halo, SH, $SC_{1-4}$alkyl, $NH_2$, $NHC_{1-4}$alkyl, $N(C_{1-4}alkyl)(C_{1-4}alkyl)$, CN, C(O)OH, C(O)$OC_{1-4}$alkyl, C(O) $NHC_{1-4}$alkyl, NHC(O)$C_{1-4}$alkyl, OC(O)$C_{1-4}$alkyl, $SOC_{1-4}$alkyl, $SO_2C_{1-4}$alkyl, $SO_2NHC_{1-4}$alkyl and $SO_2NH_2$;

$R^5$ are either both H or together form $=CH_2$;

$R^6$ and $R^7$ are independently H, $C_{1-4}$alkyl or are taken together to form a $C_{3-6}$cyloakyl ring;

x is 0-2; and

---- represents a single or a double bond.

**17.** A compound according to claim 1 selected from:

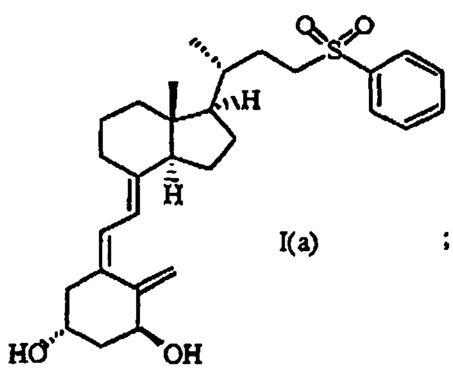

I(a) ;

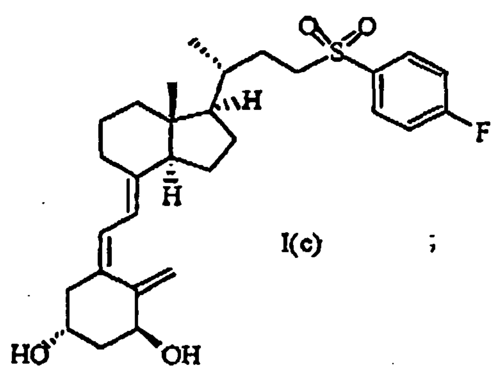

I(c) ;

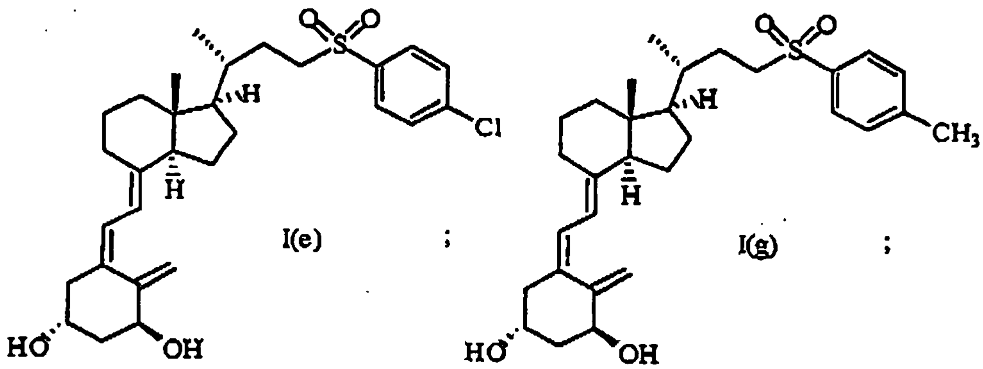

I(e) ;

I(g) ;

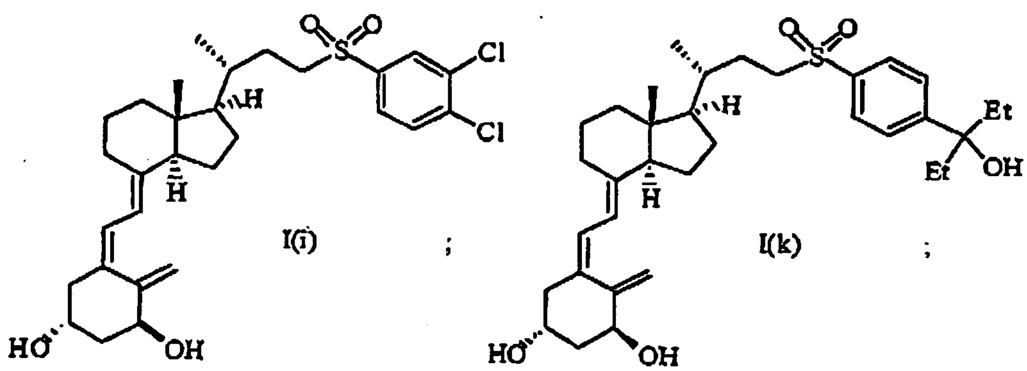

I(i) ;

I(k) ;

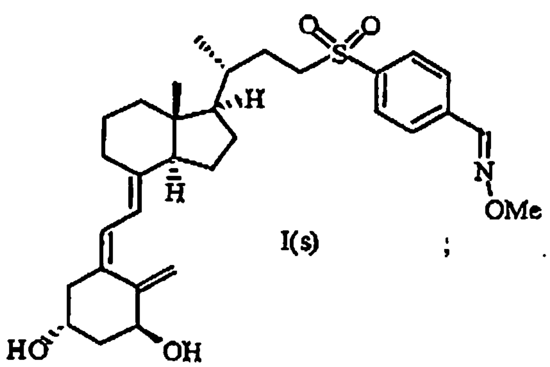

I(s) ;

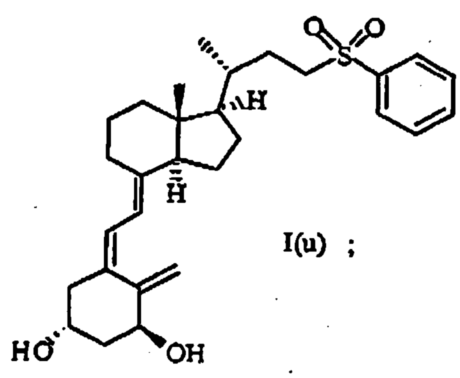

I(u) ;

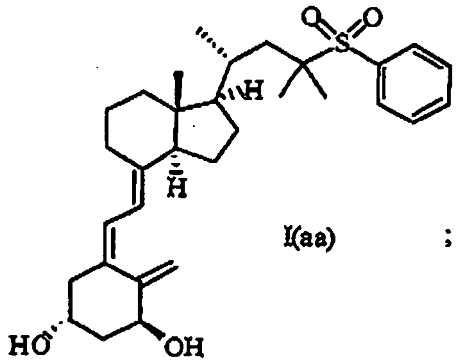

I(aa) ;

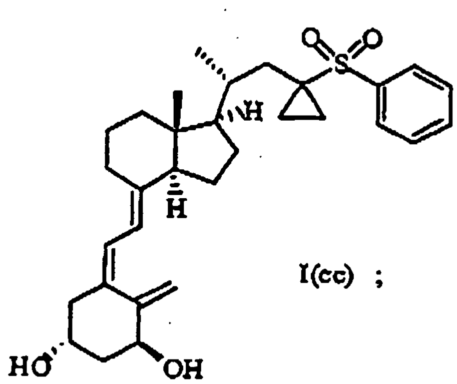

I(cc) ;

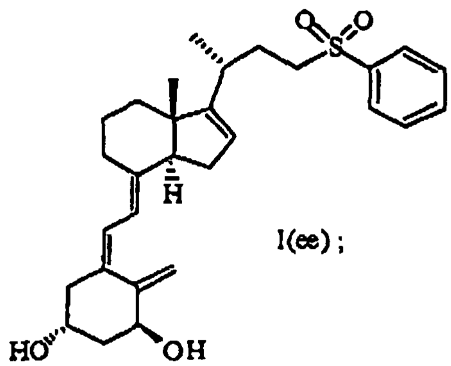

I(ee) ;

and pharmaceutically acceptable salts, hydrates, solvates and prodrugs thereof wherein the prodrug is an ester prodrug selected from the group consisting of phenyl esters, aliphatic $C_8$-$C_{24}$ esters, acyloxymethyl esters, carbamates and amino acid esters.

**18.** A compound according to any one of claims 1 to 17 for use as a medicament.

**19.** A pharmaceutical composition comprising a compound according to any of claims 1-17 and a pharmaceutically acceptable carrier.

**20.** A use of a compound according to any of claims 1-17 to prepare a medicament for the treatment of a disease selected from the group consisting of cancer, dermatological disorders, thyroid disorders, wound healing and bone disorders.

**21.** Use according to claim 19, wherein the disease is selected from the group consisting of cancer, psoriasis, thyroid disorders and osteoporosis.

**22.** A use of a compound according to any of claims 1-17 to prepare a medicament to inhibit cell proliferation.

**23.** Use according to claim 21, wherein the cell is a cancer cell.

**24.** Use according to claim 22, wherein the cancer is selected from breast cancer, lung cancer and prostate cancer.

**25.** A compound according to any one of claims 1 to 17 for the treatment of a disease selected from the group consisting of cancer, dermatological disorders, thyroid disorders, wound healing and bone disorders.

**26.** A compound according to claim 25 wherein the disease is selected from the group consisting of cancer, psoriasis, thyroid disorders and osteoporosis.

**27.** A compound according to any one of claims 1 to 17 for inhibition of cell proliferation.

**28.** A compound according to claim 27 wherein the cell is a cancer cell

**29.** A compound according to claim 28 wherein the cancer is selected from breast cancer, lung cancer and prostate cancer.

**Patentansprüche**

**1.** Verbindung der Formel 1 und pharmazeutisch verträgliche Salze, Hydrate, Solvate und Wirkstoffpräkursoren davon, wobei der Wirkstoffpräkursor ein Ester-Wirkstoffpräkursor ist, ausgewählt aus der Gruppe, bestehend aus Phenylestern, aliphatischen $C_8$-$C_{24}$-Estern, Acyloxymethylestern, Carbamaten und Aminosäureestern:

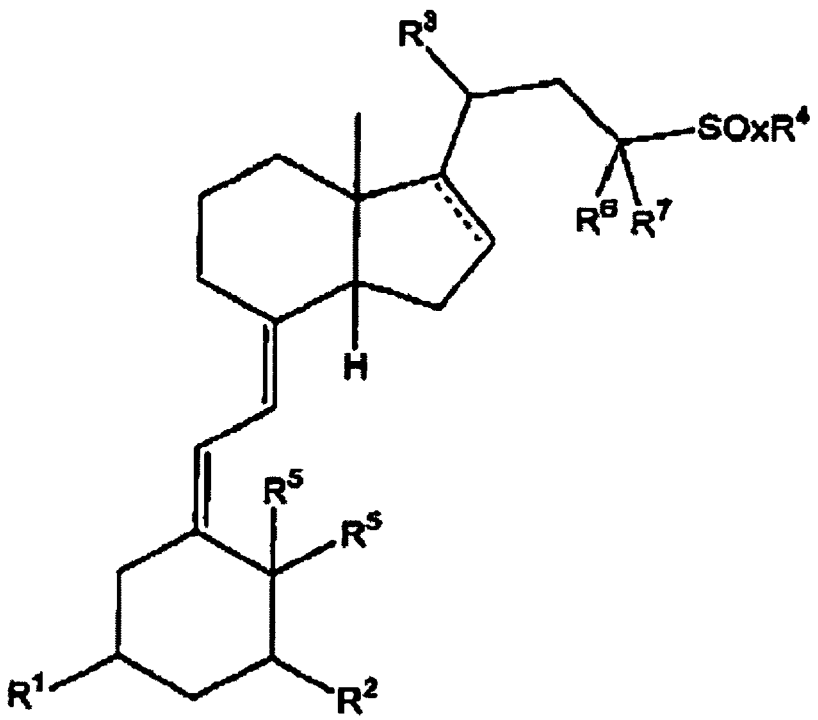

I

wobei
$R^1$ und $R^2$ unabhängig voneinander aus der Gruppe ausgewählt sind, bestehend aus OH, $OC_{1-4}$-Alkyl und Halogen,
$R^3$ $C_{1-4}$-Alkyl ist,
$R^4$ aus der Gruppe ausgewählt ist, bestehend aus Aryl und Heteroaryl, wobei sowohl Aryl als auch Heteroaryl unsubstituiert oder mit 1-5 Gruppen substituiert sind, die unabhängig voneinander unter $C_{1-4}$-Alkyl, Hydroxy-substituiertem $C_{1-6}$-Alkyl, $OC_{1-4}$-Alkyl, OH, $CF_3$, $OCF_3$, Halogen, SH, $SC_{1-4}$-Alkyl, $NH_2$, $NHC_{1-4}$-Alkyl, $N(C_{1-4}$-Alkyl)($C_{1-4}$-Alkyl), CN, C(O)OH, C(O)$OC_{1-4}$-Alkyl, C(O)$NHC_{1-4}$-Alkyl, CH=N-$OC_{1-4}$-Alkyl, NHC(O)$C_{1-4}$-Alkyl, OC(O)$C_{1-4}$-Alkyl, $SOC_{1-4}$-Alkyl, $SO_2C_{1-4}$-Alkyl, $SO_2NHC_{1-4}$-Alkyl und $SO_2NH_2$ ausgewählt sind,
$R^5$ entweder beide H sind oder zusammen =$CH_2$ bilden,
$R^6$ und $R^7$ unabhängig voneinander H oder $C_{1-4}$-Alkyl sind oder zusammengenommen werden, um einen $C_{3-6}$-Cycloalkylring zu bilden,
x 0-2 ist und
---- eine Einfach- oder Doppelbindung repräsentiert.

**2.** Verbindung nach Anspruch 1, wobei $R^1$ und $R^2$ unabhängig voneinander aus der Gruppe ausgewählt sind, bestehend aus OH, $OCH_3$ und Fluor.

3. Verbindung nach Anspruch 2, wobei $R^1$ und $R^2$ beide OH sind.

4. Verbindung nach Anspruch 1, 2 oder 3, wobei $R^3$ $CH_3$ ist.

5. Verbindung nach einem der Ansprüche 1 bis 4, wobei $R^4$ aus der Gruppe ausgewählt ist, bestehend aus unsubstituiertem und substituiertem Phenyl, Pyridyl, Thienyl, Furanyl und Pyrrolo.

6. Verbindung nach Anspruch 5, wobei $R^4$ unter unsubstituiertem oder substituiertem Phenyl ausgewählt ist.

7. Verbindung nach einem der Ansprüche 1 bis 4, wobei sowohl Aryl als auch Heteroaryl entweder unsubstituiert oder mit 1-3 Gruppen substituiert sind, die unabhängig voneinander unter $C_{1-4}$-Alkyl, Hydroxy-substituiertem $C_{1-6}$-Alkyl, $OC_{1-4}$-Alkyl, OH, $CF_3$, $OCF_3$, Halogen, SH, $SC_{1-4}$-Alkyl, $NH_2$, $NHC_{1-4}$-Alkyl, $N(C_{1-4}$-Alkyl)($C_{1-4}$-Alkyl), CN, C(O)OH, C(O)$OC_{1-4}$-Alkyl, CH=N-$OC_{1-4}$-Alkyl, C(O)$NHC_{1-4}$-Alkyl, NHC(O)$C_{1-4}$-Alkyl, OC(O)$C_{1-4}$-Alkyl, $SOC_{1-4}$-Alkyl, $SO_2C_{1-4}$-Alkyl, $SO_2NHC_{1-4}$-Alkyl und $SO_2NH_2$ ausgewählt sind.

8. Verbindung nach Anspruch 7, wobei sowohl Aryl als auch Heteroaryl entweder unsubstituiert oder mit 1-2 Gruppen substituiert sind, die unabhängig voneinander unter Methyl, 3-Hydroxy-3-pentyl, Methoxy, OH, $CF_3$, $OCF_3$, Halogen, $NH_2$, $NMe_2$ und CH=N-OMe ausgewählt sind.

9. Verbindung nach Anspruch 8, wobei sowohl Aryl als auch Heteroaryl entweder unsubstituiert oder mit 1-2 Gruppen substituiert sind, die unabhängig voneinander unter Methyl, 3-Hydroxy-3-pentyl, Cl, F und CH=N-OMe ausgewählt sind.

10. Verbindung nach Anspruch 6, wobei $R^4$ aus der Gruppe ausgewählt ist, bestehend aus Phenyl, 4-Chlorphenyl, 3,4-Dichlorphenyl, 4-Fluorphenyl, 4-Methylphenyl, 3,4-Difluorphenyl, 4-(3-Hydroxy-3-pentyl)-phenyl, 4-(CH=N-OMe)-Phenyl, 4-Methoxyphenyl, 4-Trifluormethylphenyl und 4-Nitrophenyl.

11. Verbindung nach Anspruch 10, wobei $R^4$ aus der Gruppe ausgewählt ist, bestehend aus Phenyl, 4-Chlorphenyl, 3,4-Dichlorphenyl, 4-(3-Hydroxy-3-pentyl)-phenyl, 4-Fluorphenyl und 4-Methylphenyl.

12. Verbindung nach einem der Ansprüche 1 bis 11, wobei $R^6$ und $R^7$ unabhängig voneinander H oder Methyl sind oder zusammengenommen werden, um einen $C_{3-4}$-Cycloalkylring zu bilden.

13. Verbindung nach Anspruch 12, wobei $R^6$ und $R^7$ beide H sind oder zusammengenommen werden, um einen $C_{3-4}$-Cycloalkylring zu bilden.

14. Verbindung nach einem der Ansprüche 1 bis 13, wobei x 2 ist.

15. Verbindung nach einem der Ansprüche 1 bis 14, wobei ---- eine Einfachbindung ist.

16. Verbindung nach Anspruch 1 mit der Formel I' und pharmazeutisch verträgliche Salze, Hydrate, Solvate und Wirkstoffpräkursoren davon, wobei der Wirkstoffpräkursor ein Ester-Wirkstoffpräkursor ist, ausgewählt aus der Gruppe, bestehend aus Phenylestern, aliphatischen $C_8$-$C_{24}$-Estern, Acyloxymethylestern, Carbamaten und Aminosäureestern:

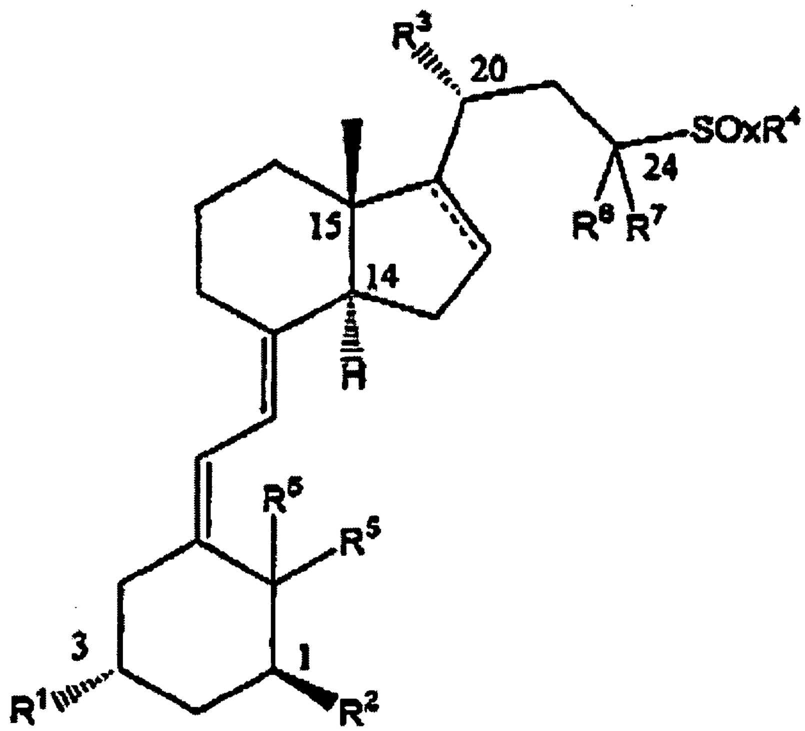

I'

wobei

$R^1$ und $R^2$ unabhängig voneinander aus der Gruppe ausgewählt sind, bestehend aus OH, $OC_{1-4}$-Alkyl und Halogen,

$R^3$ $C_{1-4}$-Alkyl ist,

$R^4$ aus der Gruppe ausgewählt ist, bestehend aus Aryl und Heteroaryl, wobei sowohl Aryl als auch Heteroaryl unsubstituiert oder mit 1-5 Gruppen substituiert sind, die unabhängig voneinander unter $C_{1-4}$-Alkyl, Hydroxy-substituiertem $C_{1-6}$-Alkyl, $OC_{1-4}$-Alkyl, OH, $CF_3$, $OCF_3$, Halogen, SH, $SC_{1-4}$-Alkyl, $NH_2$, $NHC_{1-4}$-Alkyl, $N(C_{1-4}$-Alkyl)$(C_{1-4}$-Alkyl), CN, C(O)OH, C(O)$OC_{1-4}$-Alky), C(O)$NHC_{1-4}$-Alkyl, NHC(O)$C_{1-4}$-Alkyl, OC(O)$C_{1-4}$-Alkyl, $SOC_{1-4}$-Alkyl, $SO_2C_{1-4}$-Alkyl, $SO_2NHC_{1-4}$-Alkyl und $SO_2NH_2$ ausgewählt sind,

$R^5$ entweder beide H sind oder zusammen =$CH_2$ bilden,

$R^6$ und $R^7$ unabhängig voneinander H oder $C_{1-4}$-Alkyl sind oder zusammengenommen werden, um einen $C_{3-6}$-Cycloalkylring zu bilden,

x 0-2 ist und

---- eine Einfach- oder eine Doppelbindung repräsentiert.

**17.** Verbindung nach Anspruch 1, ausgewählt unter:

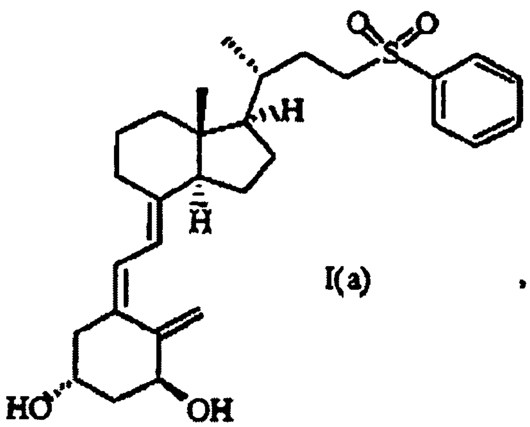

I(a) ,

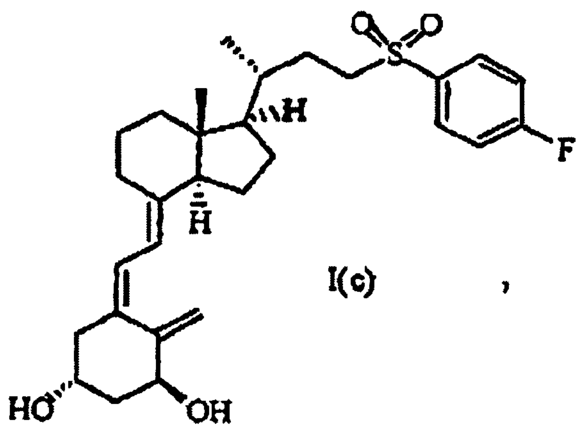

I(c) ,

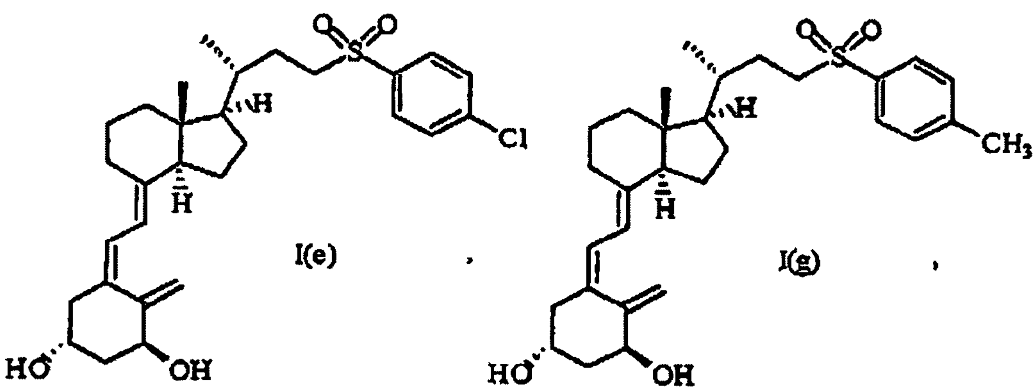

I(e) , I(g) ,

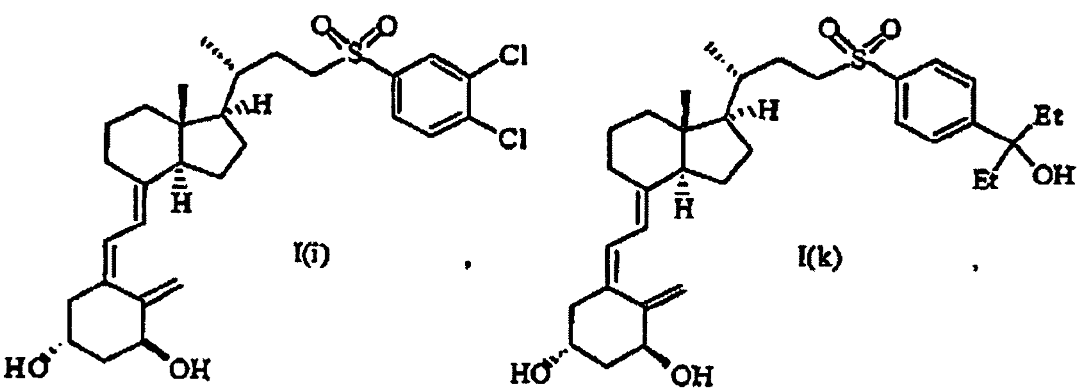

I(i) , I(k) ,

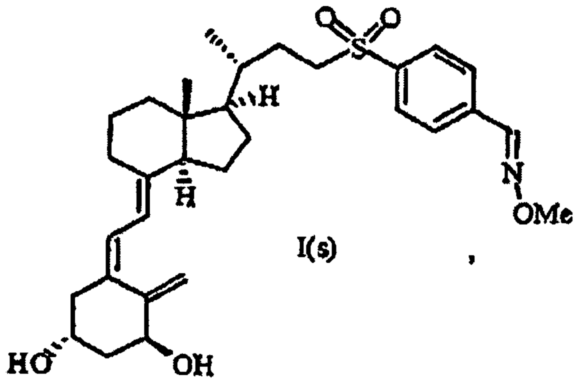

I(s) ,

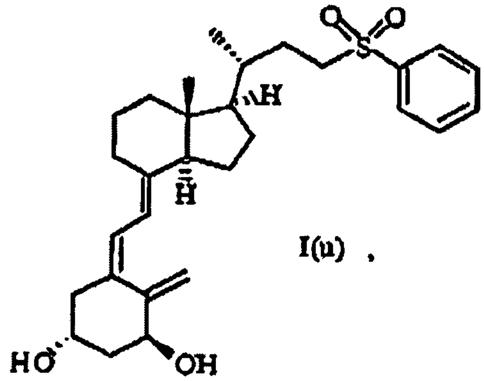

I(u) ,

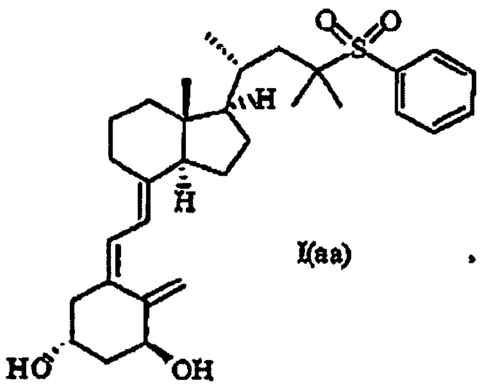

I(aa) ,

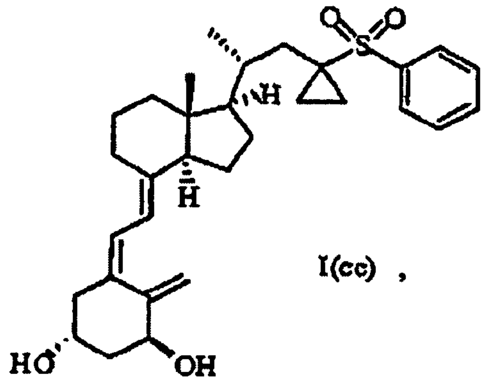

I(cc) ,

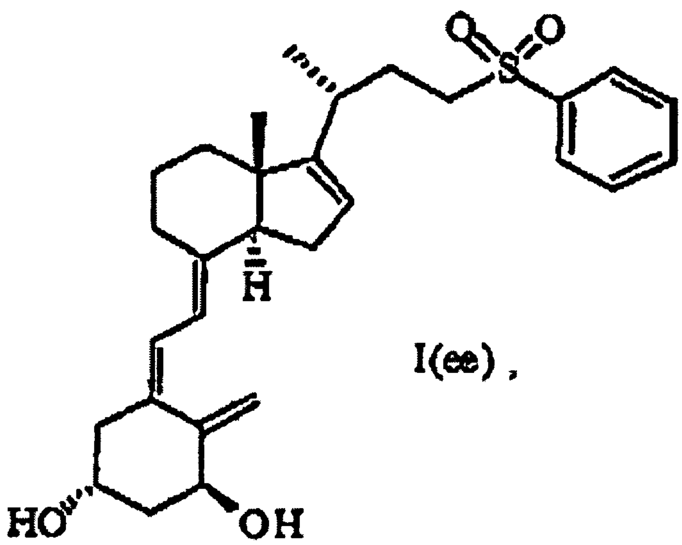

I(ee) ,

und pharmazeutisch verträgliche Salze, Hydrate, Solvate und Wirkstoffpräkursoren davon, wobei der Wirkstoffpräkursor ein Ester-Wirkstoffpräkursor ist, ausgewählt aus der Gruppe, bestehend aus Phenylestern, aliphatischen $C_8$-$C_{24}$-Estern, Acyloxymethylestem, Carbamaten und Aminosäureestern.

**18.** Verbindung nach einem der Ansprüche 1 bis 17 zur Verwendung als ein Medikament.

**19.** Pharmazeutische Zusammensetzung, welche eine Verbindung nach einem der Ansprüche 1 bis 17 und einen pharmazeutisch verträglichen Träger umfaßt.

**20.** Verwendung einer Verbindung nach einem der Ansprüche 1 bis 17 zur Herstellung eines Medikaments für die Behandlung einer Erkrankung, die aus der Gruppe ausgewählt ist, bestehend aus Krebs, dermatologischen Störungen, Schilddrüsenstörungen, Wundheilungs- und Knochenstörungen.

**21.** Verwendung nach Anspruch 19, wobei die Erkrankung aus der Gruppe ausgewählt ist, bestehend aus Krebs, Psoriasis, Schilddrüsenstörungen und Osteoporose.

**22.** Verwendung einer Verbindung nach einem der Ansprüche 1 bis 17 zur Herstellung eines Medikaments zur Inhibition der Zellproliferation.

**23.** Verwendung nach Anspruch 21, wobei die Zelle eine Krebszelle ist.

**24.** Verwendung nach Anspruch 22, wobei der Krebs unter Brustkrebs, Lungenkrebs und Prostatakrebs ausgewählt ist.

**25.** Verbindung nach einem der Ansprüche 1 bis 17 zur Behandlung einer Erkrankung, die aus der Gruppe ausgewählt ist, bestehend aus Krebs, dermatologischen Störungen, Schilddrüsenstörungen, Wundheilungs- und Knochenstörungen.

**26.** Verbindung nach Anspruch 25, wobei die Erkrankung aus der Gruppe ausgewählt ist, bestehend aus Krebs, Psoriasis, Schilddrüsenstörungen und Osteoporose.

**27.** Verbindung nach einem der Ansprüche 1 bis 17 zur Inhibition der Zellproliferation.

**28.** Verbindung nach Anspruch 27, wobei die Zelle eine Krebszelle ist.

**29.** Verbindung nach Anspruch 28, wobei der Krebs unter Brustkrebs, Lungenkrebs und Prostatakrebs ausgewählt ist.

## Revendications

**1.** Composé de formule I, et ses sels, hydrates, produits de solvatation et précurseurs médicamenteux pharmaceutiquement acceptables, dans lesquels le précurseur médicamenteux est un précurseur médicamenteux du type ester choisi dans le groupe consistant en des esters phényliques, des esters aliphatiques en $C_8$ à $C_{24}$, des esters acyloxyméthyliques, des carbamates et des esters d'aminoacides :

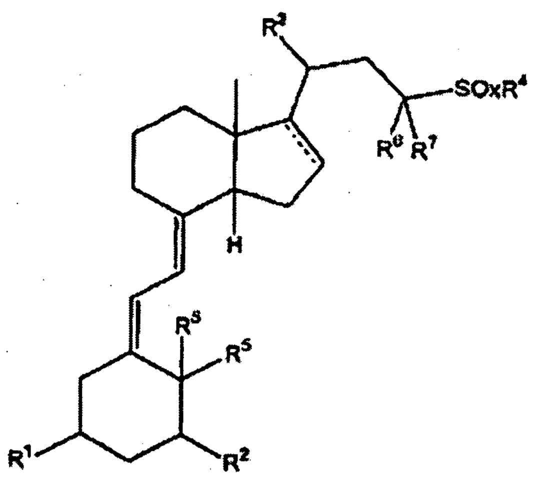

I

formule dans laquelle

$R^1$ et $R^2$ sont choisis indépendamment dans le groupe consistant en des groupes OH, O-alkyle en $C_1$ à $C_4$ et halogéno ;
$R^3$ représente un groupe alkyle en $C_1$ à $C_4$ ;
$R^4$ est choisi dans le groupe consistant en des groupes aryle et hétéroaryle, les groupes aryle et hétéroaryle étant non substitués ou substitués avec 1 à 5 groupes choisis indépendamment entre des groupes alkyle en $C_1$ à $C_4$, alkyle en $C_1$ à $C_6$ à substituant hydroxy, O-alkyle en $C_1$ à $C_4$, OH, $CF_3$, $OCF_3$, halogéno, SH, S-alkyle en $C_1$ à $C_4$, $NH_2$, NH-alkyle en $C_1$ à $C_4$, N (alkyle en $C_1$ à $C_4$) (alkyle en $C_1$ à $C_4$), CN, C(O)OH, C(O)O(alkyle en $C_1$ à $C_4$), C (O)NH(alkyle en $C_1$ à $C_4$), CH=N-O(alkyle en $C_1$ à $C_4$), NHC(O) (alkyle en $C_1$ à $C_4$), OC(O) (alkyle en $C_1$ à $C_4$), SO (alkyle en $C_1$ à $C_4$), $SO_2$ (alkyle en $C_1$ à $C_4$), $SO_2NH$ (alkyle en $C_1$ à $C_4$) et $SO_2NH_2$ ;
les groupes $R^5$ soit représentent l'un et l'autre H, soit forment conjointement un groupe $=CH_2$ ;
$R^6$ et $R^7$ représentent indépendamment H ou un groupe alkyle en $C_1$ à $C_4$ ou bien sont pris conjointement pour former un noyau cycloalkyle en $C_3$ à $C_6$ ;
x a une valeur de 0 à 2 ; et
---- représente une liaison simple ou une double liaison.

2. Composé suivant la revendication 1, dans lequel $R^1$ et $R^2$ sont choisis indépendamment dans le groupe consistant en des groupes OH, $OCH_3$ et fluoro.

3. Composé suivant la revendication 2, dans lequel $R^1$ et $R^2$ représentent l'un et l'autre un groupe OH.

4. Composé suivant la revendication 1, 2 ou 3, dans lequel $R^3$ représente un groupe $CH_3$.

5. Composé suivant l'une quelconque des revendications 1 à 4, dans lequel $R^4$ est choisi dans le groupe consistant en des groupes phényle, pyridyle, thiényle, furannyle et pyrrolo, non substitués et substitués.

6. Composé suivant la revendication 5, dans lequel $R^4$ est choisi entre un groupe phényle non substitué et un groupe phényle substitué.

7. Composé suivant l'une quelconque des revendications 1 à 4, dans lequel les groupes aryle et hétéroaryle sont non substitués ou substitués avec 1 à 3 groupes choisis indépendamment entre des groupes alkyle en $C_1$ à $C_4$, alkyle en $C_1$ à $C_6$ à substituant hydroxy, O-alkyle en $C_1$ à $C_4$, OH, $CF_3$, $OCF_3$, halogéno, SH, S-alkyle en $C_1$ à $C_4$, $NH_2$, NH-alkyle en $C_1$ à $C_4$, N (alkyle en $C_1$ à $C_4$) (alkyle en $C_1$ à $C_4$), CN, C(O)OH, C(O)O(alkyle en $C_1$ à $C_4$), CH=N-O (alkyle en $C_1$ à $C_4$), C(O)NH(alkyle en $C_1$ à $C_4$), NHC(O) (alkyle en $C_1$ à $C_4$), OC(O)-(alkyle en $C_1$ à $C_4$), SO(alkyle en $C_1$ à $C_4$), $SO_2$(alkyl en $C_1$ à $C_4$), $SO_2NH$ (alkyle en $C_1$ à $C_4$) et $SO_2NH_2$.

8. Composé suivant la revendication 7, dans lequel les groupes aryle et hétéroaryle sont non substitués ou substitués avec 1 ou 2 groupes choisis indépendamment entre des groupes méthyle, 3-hydroxy-3-pentyle, méthoxy, OH, $CF_3$, $OCF_3$, halogéno, $NH_2$, $NMe_2$ et CH=N-OMe.

9. Composé suivant la revendication 8, dans lequel les groupes aryle et hétéroaryle sont non substitués ou substitués avec 1 ou 2 groupes choisis indépendamment entre des groupes méthyle, 3-hydroxy-3-pentyle, C1, F et CH=N-OMe.

10. Composé suivant la revendication 6, dans lequel $R^4$ est choisi dans le groupe consistant en des groupes phényle, 4-chlorophényle, 3,4-dichlorophényle, 4-fluorophényle, 4-méthylphényle, 3,4-difluorophényle, 4-(3-hydroxy-3-pentyl)-phényle, 4-(CH=N-OMe)phényle, 4-méthoxyphényle, 4-trifluorométhylphényle et 4-nitrophényle.

11. Composé suivant la revendication 10, dans lequel $R^4$ est choisi dans le groupe consistant en des groupes phényle, 4-chlorophényle, 3,4-dichlorophényle, 4-(3-hydroxy-3-pentyl)phényle, 4-fluorophényle et 4-méthylphényle.

12. Composé suivant l'une quelconque des revendications 1 à 11, dans lequel $R^6$ et $R^7$ représentent indépendamment H ou un groupe méthyle ou bien sont pris conjointement pour former un noyau cycloalkyle en $C_3$ ou $C_4$.

13. Composé suivant la revendication 12, dans lequel $R^6$ et $R^7$ représentent l'un et l'autre H ou bien sont pris conjointement pour former un noyau cycloalkyle en $C_3$ ou $C_4$.

14. Composé suivant l'une quelconque des revendications 1 à 13, dans lequel x est égal à 2.

15. Composé suivant l'une quelconque des revendications 1 à 14, dans lequel ---- représente une liaison simple.

**16.** Composé suivant la revendication 1, de formule I', et ses sels, hydrates, produits de solvatation et précurseurs médicamenteux pharmaceutiquement acceptables, dans lesquels le précurseur médicamenteux est un précurseur médicamenteux du type ester choisi dans le groupe consistant en des esters phényliques, des esters aliphatiques en $C_8$ à $C_{24}$, des esters acyloxyméthyliques, des carbamates et des esters d'aminoacides :

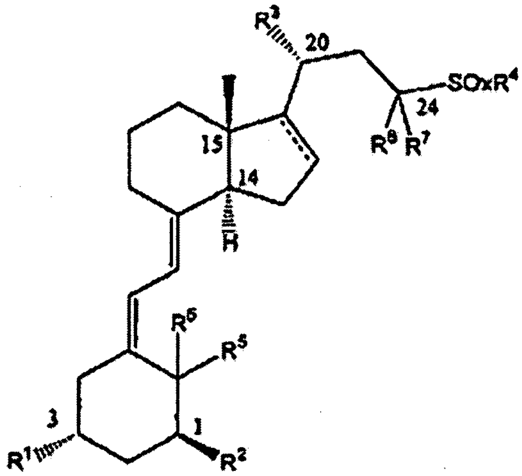

I'

formule dans laquelle

$R^1$ et $R^2$ sont choisis indépendamment dans le groupe consistant en des groupes OH, O-alkyle en $C_1$ à $C_4$ et halogéno ;

$R^3$ représente un groupe alkyle en $C_1$ à $C_4$ ;

$R^4$ est choisi dans le groupe consistant en des groupes aryle et hétéroaryle, les groupes aryle et hétéroaryle étant non substitués ou substitués avec 1 à 5 groupes choisis indépendamment entre des groupes alkyle en $C_1$ à $C_4$, alkyle en $C_1$ à $C_6$ à substituant hydroxy, O-alkyle en $C_1$ à $C_4$, OH, $CF_3$, $OCF_3$, halogéno, SH, S-alkyle en $C_1$ à $C_4$, $NH_2$, NH-alkyle en $C_1$ à $C_4$, N (alkyle en $C_1$ à $C_4$) (alkyle en $C_1$ à $C_4$), CN, C(O)OH, C(O)O(alkyle en $C_1$ à $C_4$), C (O)NH(alkyle en $C_1$ à $C_4$), NHC(O) (alkyle en $C_1$ à $C_4$), OC(O) (alkyle en $C_1$ à $C_4$), SO(alkyle en $C_1$ à $C_4$), $SO_2$ (alkyle en $C_1$ à $C_4$), $SO_2NH$ (alkyle en $C_1$ à $C_4$) et $SO_2NH_2$ ;

les groupes $R^5$ soit représentent l'un et l'autre H, soit forment conjointement un groupe $=CH_2$ ;

$R^6$ et $R^7$ représentent indépendamment H ou un groupe alkyle en $C_1$ à $C_4$ ou bien sont pris conjointement pour former un noyau cycloalkyle en $C_3$ à $C_6$ ;

x a une valeur de 0 à 2 ; et

---- représente une liaison simple ou une double liaison.

**17.** Composé suivant la revendication 1, choisi entre :

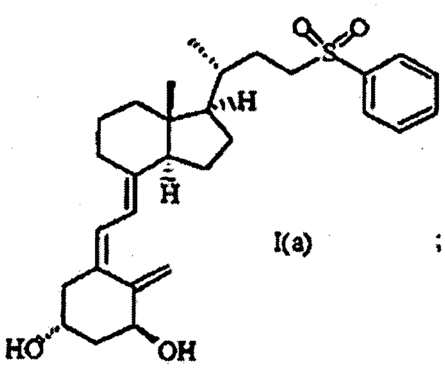

I(a) ;

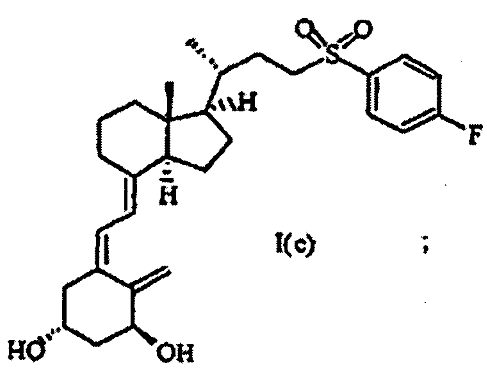

I(c) ;

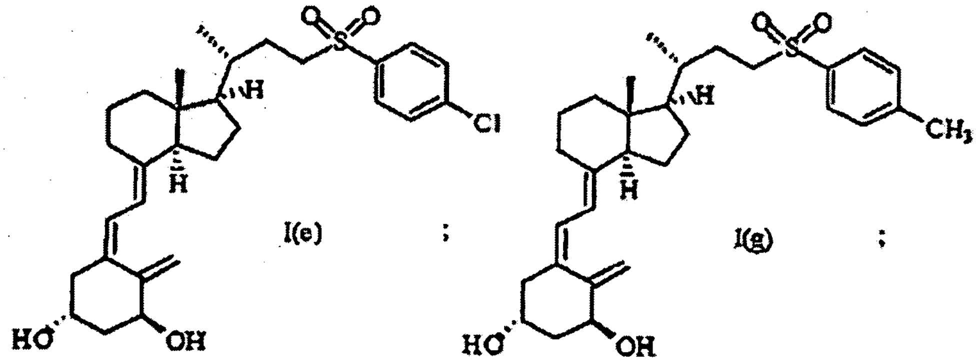

I(e) ; I(g) ;

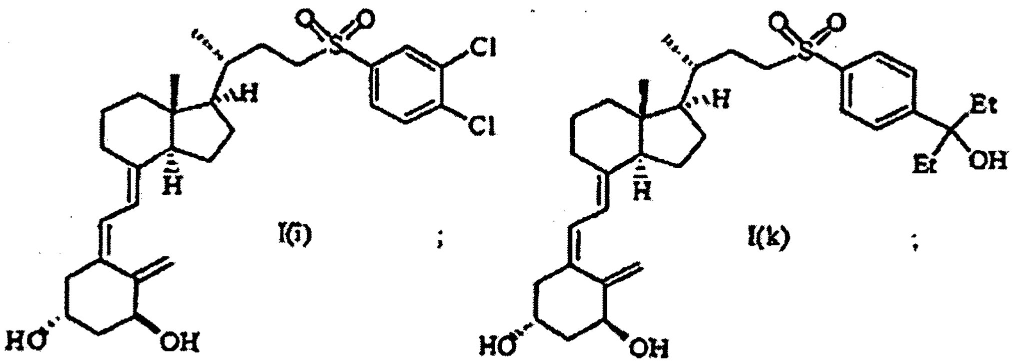

I(i) ; I(k) ;

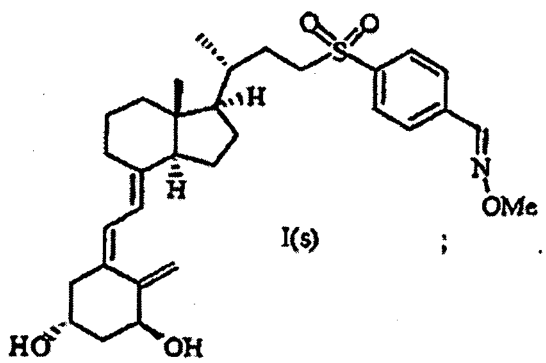

I(s) ;

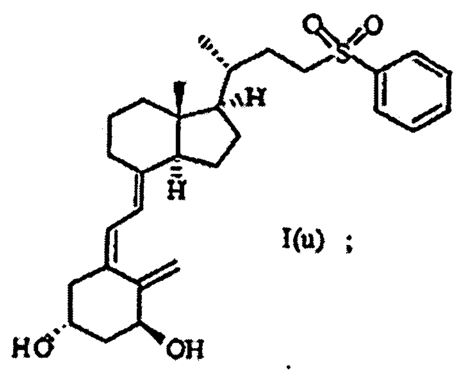

I(u) ;

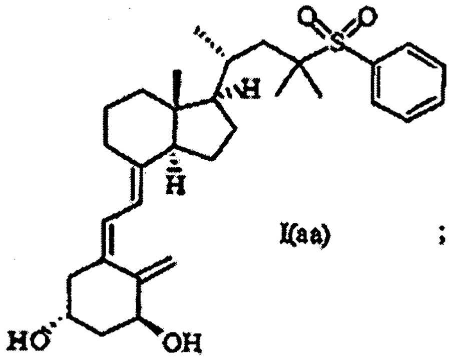

I(aa) ;

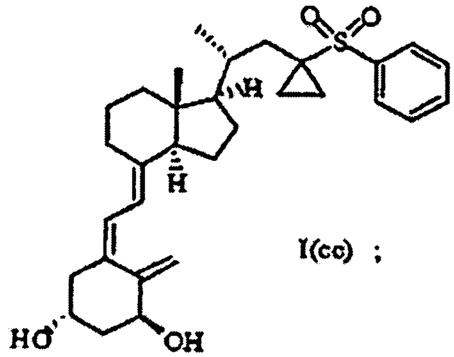

I(cc) ;

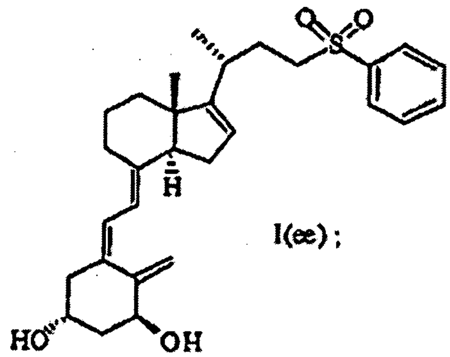

I(ee) ;

et ses sels, hydrates, produits de solvatation et précurseurs médicamenteux pharmaceutiquement acceptables, dans lesquels le précurseur médicamenteux est un précurseur médicamenteux du type ester choisi dans le groupe consistant en des esters phényliques, des esters aliphatiques en $C_8$ à $C_{24}$, des esters acyloxyméthyliques, des carbamates et des esters d'aminoacides.

**18.** Composé suivant l'une quelconque des revendications 1 à 17, destiné à être utilisé comme médicament.

**19.** Composition pharmaceutique comprenant un composé suivant l'une quelconque des revendications 1 à 17 et un support pharmaceutiquement acceptable.

**20.** Utilisation d'un composé suivant l'une quelconque des revendications 1 à 17 pour la préparation d'un médicament destiné au traitement d'une maladie choisie dans le groupe consistant en le cancer, des troubles dermatologiques, des troubles de la thyroïde, la cicatrisation de plaies et des troubles osseux.

**21.** Utilisation suivant la revendication 19, dans laquelle la maladie est choisie dans le groupe consistant en le cancer, le psoriasis, des affections de la thyroïde et l'ostéoporose.

**22.** Utilisation d'un composé suivant l'une quelconque des revendications 1 à 17 pour la préparation d'un médicament destiné à inhiber la prolifération cellulaire.

**23.** Utilisation suivant la revendication 21, dans laquelle la cellule est une cellule cancéreuse.

**24.** Utilisation suivant la revendication 22, dans laquelle le cancer est choisi entre le cancer du sein, le cancer du poumon et le cancer de la prostate.

**25.** Composé suivant l'une quelconque des revendications 1 à 17, destiné au traitement d'une maladie choisie dans le groupe consistant en le cancer, des troubles dermatologiques, des affections de la thyroïde, la cicatrisation de plaies et des troubles osseux.

**26.** Composé suivant la revendication 25, dans lequel la maladie est choisie dans le groupe consistant en le cancer, le psoriasis, des affections de la thyroïde et l'ostéoporose.

**27.** Composé suivant l'une quelconque des revendications 1 à 17, destiné à inhiber la prolifération cellulaire.

**28.** Composé suivant la revendication 27, dans lequel la cellule est une cellule cancéreuse.

**29.** Composé suivant la revendication 28, dans lequel le cancer est choisi entre le cancer du sein, le cancer du poumon et le cancer de la prostate.

A

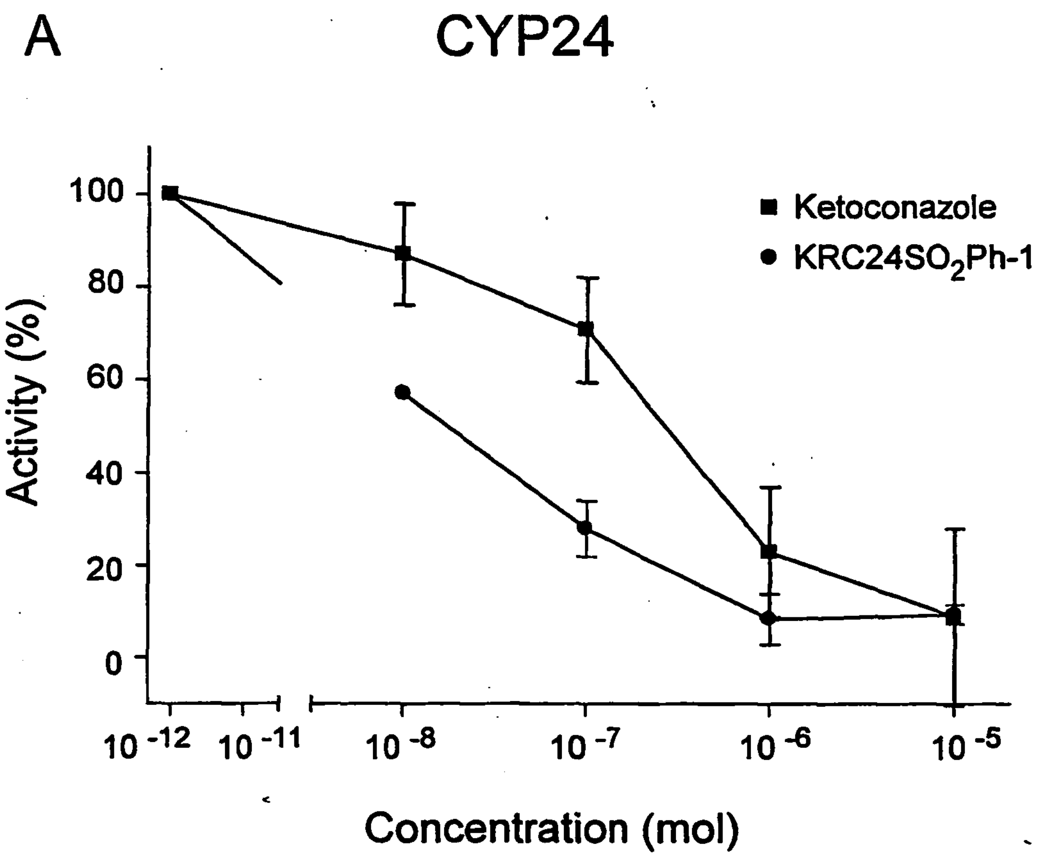
CYP24
Activity (%)
100
80
60
40
20
0
Ketoconazole
KRC24SO2Ph-1
10 -12
10 -11
10 -8
10 -7
10 -6
10 -5
Concentration (mol)

FIGURE 1

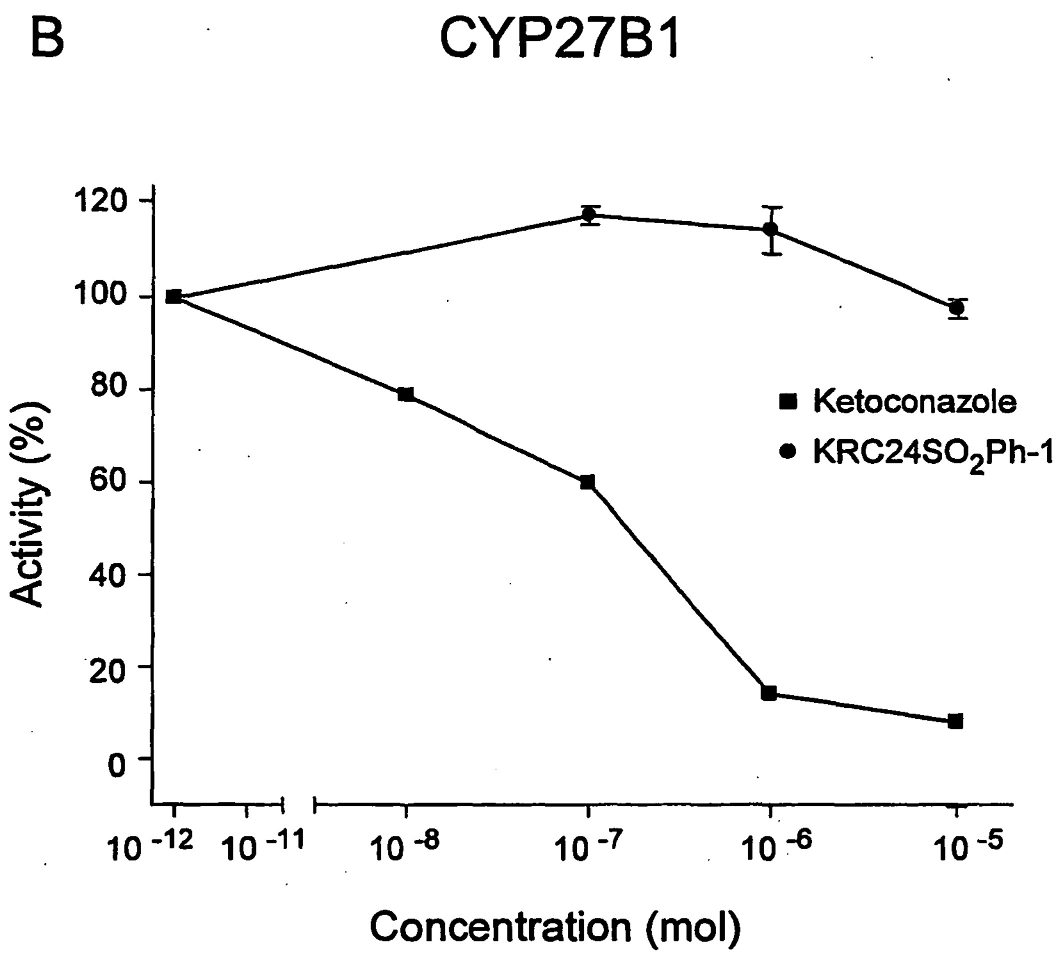


FIGURE 1 (CONT.)

C

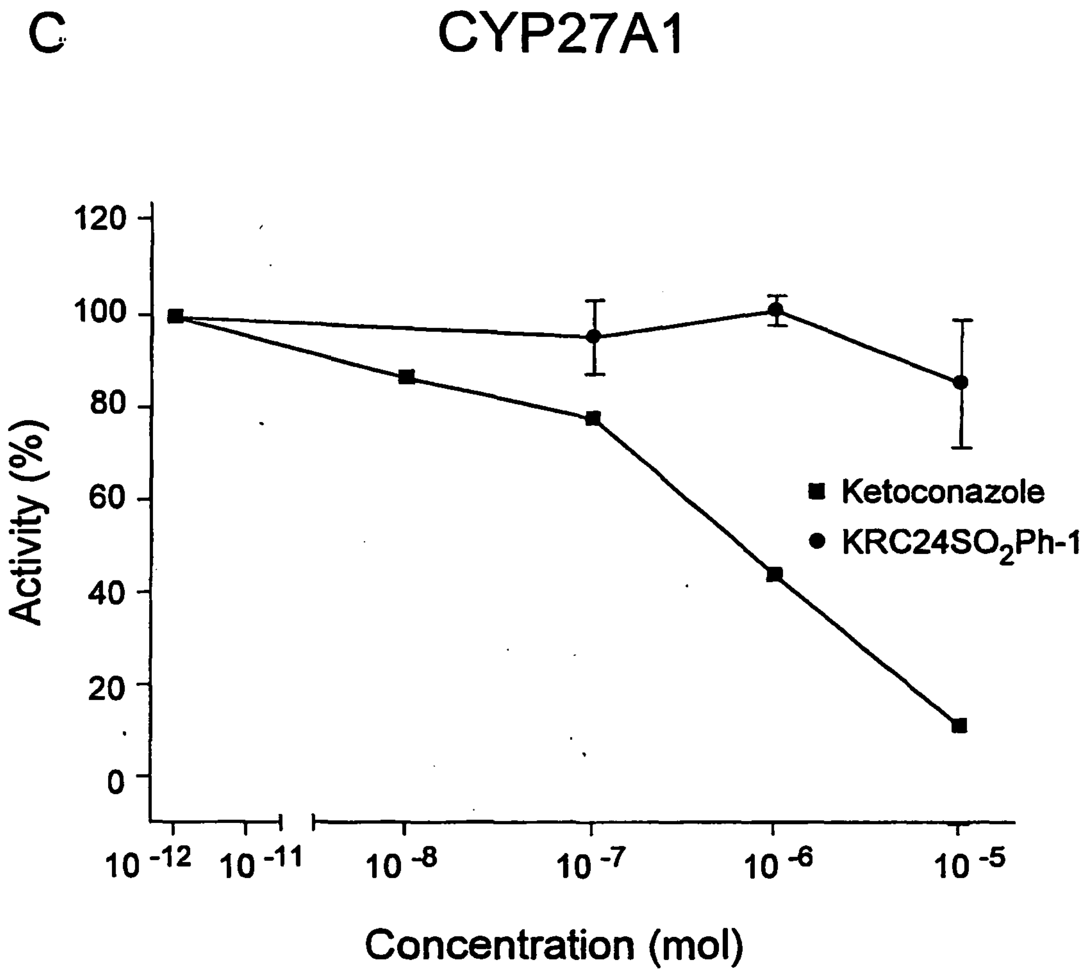


FIGURE 1 (CONT.)

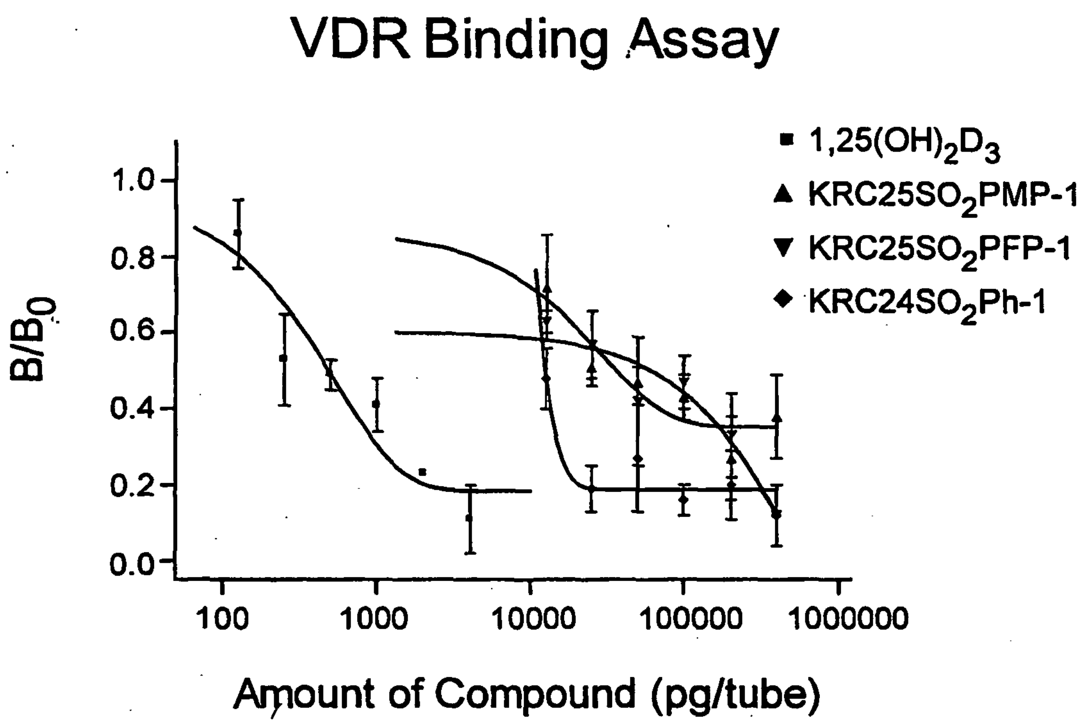


| Compound | $B_{50}$ (pg) |
|---|---|
| $1,25(OH)_2D_3$ | 370 |
| $KRC25SO_2PMP$-1 | 20090 |
| $KRC25SO_2PFP$-1 | 30160 |
| $KRC24SO_2Ph$-1 | 12500 |

FIGURE 2

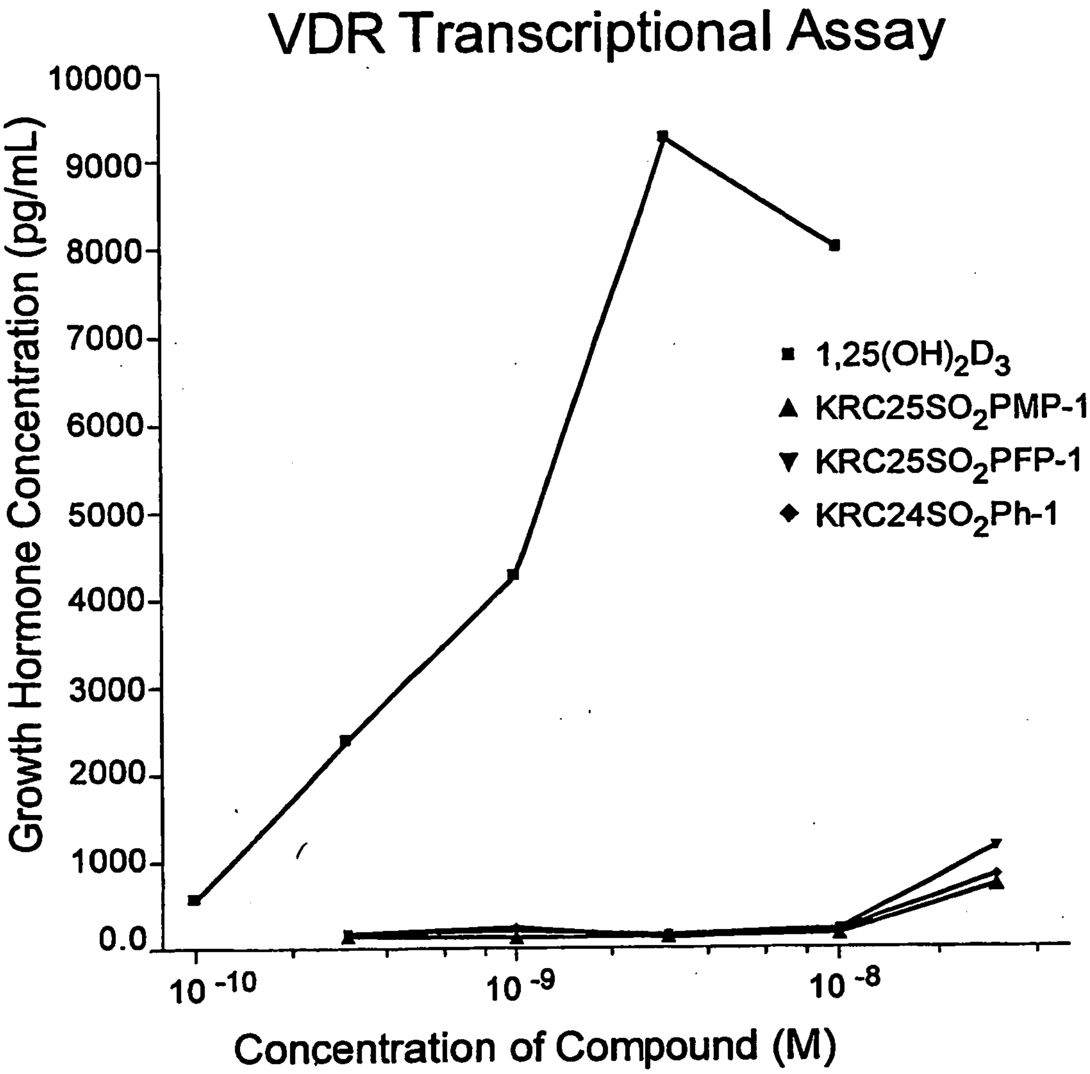


| Compound | $B_{50}$ (M) |
|---|---|
| $1,25(OH)_2D_3$ | $1.14 \times 10^{-9}$ |
| $KRC25SO_2PMP$-1 | $> 3 \times 10^{-8}$ |
| $KRC25SO_2PFP$-1 | $> 3 \times 10^{-8}$ |
| $KRC24SO_2Ph$-1 | $> 3 \times 10^{-8}$ |

FIGURE 3

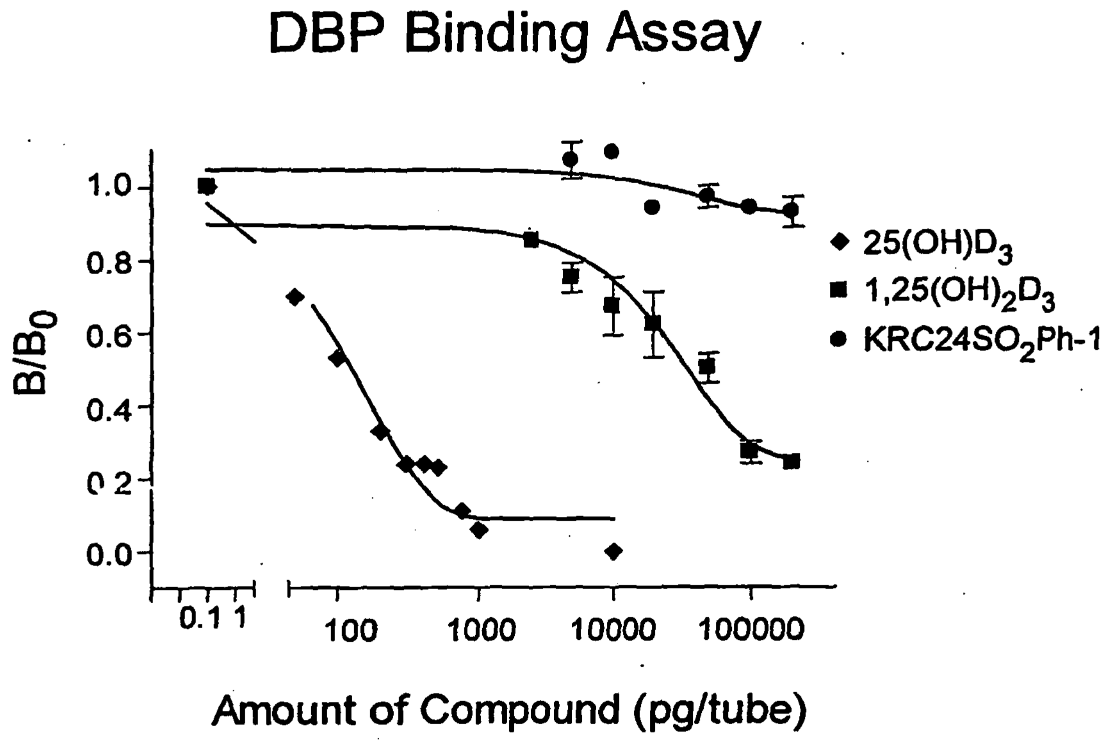
DBP Binding Assay
1.0
0.8
0.6
0.4
0 2
0.0
B/B0
0.1 1
100
1000
10000
100000
Amount of Compound (pg/tube)
25(OH)D3
1,25(OH)2D3
KRC24SO2Ph-1

FIGURE 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9410139 A
- WO 0059513 A
- US 6380408 B, Posner G.H.
- US 5830885 A

### Non-patent literature cited in the description


- **BECKMAN, M. ; DELUCA, H.** *Methods in Enzymol.,* 1997, vol. 282, 200-223
- **JONES, G. ; STRUGNELL, S. ; DELUCA, H.** *Physiol. Rev.,* 1998, vol. 78, 1193-1231
- **ARMBRECHT, H.J. ; OKUDA, K. ; WONGSURAWAT, N. ; NEMANI, R. ; CHEN, M. ; BOLTZ, M.** *J. Steroid Biochem. Molec. Biol.,* 1992, vol. 43, 1073-1081
- **BELL, N.H.** *J. Bone Miner. Res,* 1998, vol. 13, 350-35211
- Protective Groups in Organic Chemistry. Plenum Press, 1973
- **GREENE, T.W. ; WUTS, P.G.M.** Protective Groups in Organic Synthesis. John Wiley & Sons, 1991
- **KOMBLUM et al.** *J. Am Chem. Soc.,* 1959, vol. 81, 4113-4116
- **POSNER, G. H. et al.** *J. Med Chem .,* 1992, vol. 42, 3425-3435
- **POSNER, G. H. et al.** *J Med. Chem .,* 1992, vol. 42, 3425-3435
- **JAEKYOO LEE.** *Ph.D. Thesis,* 1997
- **POSNER, G. H. et al.** *J. Med Chem.,* 1992, vol. 35, 3280-3287
- **HILPERT, H. ; WIRZ, B.** *Tetrahedron,* 2001, vol. 57, 681-694
- **POSNER, G. H. et al.** *J. Org. Chem.,* 1997, vol. 62, 3299-3314
- **TRACHTENBERG, J. et al.** *J. Urol.,* 1984, vol. J32, 61-63
- **C. S. KASYAPA.** Regulation of IL-15 - Simulated TNF-alpha Production by Rolipram. *Journal of Immunology,* 1999, vol. 163, 8236
- **T. ADACHI.** A novel Lyn-Binding Peptide Inhibitor Blocks Eosinophil Differentiation, Survival, and Airway eosinophilic inflammation. *Journal of Immunology,* 1999, vol. 163, 939
- **R. ÜCHERT.** Inhibition of Keratinocyte apoptosis by IL-15: a new parameter in the pathegenosis of psoriasis. *Journal of Immunology,* 2000, vol. 165, 224
- **A. H. ENK.** T-cell receptor mimic peptides and their potential application in T-cell mediated disease. *International Archives of allergy and Immunology,* 2000, vol. 123, 275
- Remington's Pharmaceutical Sciences. Mack Publishing Company, 1985
- Remington's Pharmaceutical Sciences. The National Formulary, 1990
- **SUFFERT, J.** *J. Org. Chem.,* 1989, vol. 54, 509-510
- *Helv. Chim. Acta.,* 1999, vol. 82, 372-388
- **CABIDDU, M.G. et al.** *J. Organometallic Chem.,* 1997, vol. 531, 125-140
- **POSNER, G. H. et al.** *J. Med. Chem.,* 1992, vol. 35, 3280-3287
- **RAY S ; RAY R ; HOLICK M.** *Metabolism of H-1alpha, 25-dihydroxyvitamin D3 in the cultured human keratinocytes,* 1995, vol. 59, 117-122
- **DILWORTH F J ; SCOTT I ; GREEN A ; STRUGNELL S ; GUO Y D ; ROBERTS E A ; KREMER R ; CALVERLEY, M J ; MAKIN H L J ; JONES G.** Different mechanisms of hydroxylation site selection by liver and kidney cytochrome P450 species (CYP27 and CYP24) involved in Vitamin D metabolism. *J Biochem,* 1995, vol. 270 (28), 16766-16774
- **SHINK T ; SHIMADA H ; WAKINO S ; ANAZAWA H ; HAYASHI M ; SARUTA T ; DELUCA H ; SUDA T.** Cloning and expression of rat 25-hydroxyvitamin D3-1-alpha -hydroxylase cDNA. *Pro. Natl. Acad Sci,* 1997, vol. 94, 12920-12925
- **MURALIDHARAN K R ; ROWLAND-GOLDSMITH M ; LEE S A ; PARK G ; NORMAN A W ; HENRY H L ; OKAMURA W H.** Inhibitors of 25-hydroxyvitamin D3-1alpha-hydroxylase: Thiavitamin D analogues and biological evaluation. *J Steroid Biochem. Molec. Biol.,* 1997, vol. 62 (1), 73-78
- **DILWORTH F J ; BLACK S M ; GUO Y D ; MILLER W L ; JONES G.** Construction of a P450c27 fusion enzyme: a useful tool for analysis of vitamin D3 25-hydroxylase. *Biochem J,* 1996, vol. 320, 267-271

- **SAWADA N ; SAKAKI T ; OHTA M ; INOUYE K.** Metabolism of vitamin D (3)by human CYP27A1. *Biochem Biophys Res Commun,* 2000, vol. 273 (3), 977-84
- **ROSS T K ; PRAHL J M ; DELUKA H.** Overproduction of rat 1,25-dihydroxyvitamin D3 receptor in insect cells using the baculovirus expression system. *Proc Natl Acd Sci USA,* 1991, vol. 88, 6555-6559
- **WECKSLER W R ; NORMAN A W.** An hydroxylapatite batch assay for the quantitation of 1alpha, 25-dihydroxyvitamin D3-receptor complexes. *Anal Biochem,* 1979, vol. 92, 314-323
- **HASHIMOTO Y ; IKEDA I ; IKEDA M ; TAKAHASHI Y ; HOSAKA M ; UCHIDA H ; KONO N ; FUKUI H ; MAKINO T ; HONJO M.** Construction of a specific and sensitive sandwich enzyme immunoassay for 20 KD human growth hormone. *J Immumol Methods,* 1998, vol. 221, 77-85
- **JONE G ; BYFORD V ; MAKIN H L J ; KREMER R ; RICE R H ; DEGRAFFENRIED L A ; KNUTSON J C ; BISHOP C W.** Anti-proliferative activity and target cell catabolism of the vitamin D analogue 1alpha, 24(OH)2D2 in normal and immortalized human epidermal cells. *Biochem Pharmacol,* 1996, vol. 52, 133-140
- **BOUILLON R ; VAN BAELEN H ; MOOR P D.** Comparative study of the affinity of the serum vitamin D -binding protein. *1 Steroid Biochem,* 1980, vol. 13, 1029-44
- **JONES L ; BYRNES B ; PALMA P ; SEGEV D ; MAZUR E.** Displacement potency of vitamin D2 analogue in competitive protein-binding assay for 25-hydroxyvitamin D3, 24,25-dihydroxyvitamin D3 and 1,25-dihydroxyvitamin. D. *J Clin Endocrinol Metab,* 1980, vol. 50, 773-775
- **POSNER et al.** *J. Med. Chem.,* 1998, vol. 41, 3008-3014